# EUROPÄISCHE PATENTSCHRIFT

(11) **EP 2 872 475 B1**
(45) Veröffentlichungstag und Bekanntmachung des Hinweises auf die Patenterteilung: **08.06.2016**
(21) Anmeldenummer: 13736903.9
(22) Anmeldetag: 12.07.2013
(51) Int. Cl.: C07C 51/09, C07C 57/04, C08G 63/90

(54) **VERFAHREN ZUR HERSTELLUNG VON ACRYLSÄURE AUS ETHYLENOXID UND KOHLENMONOXID**
METHOD FOR PRODUCING ACRYLIC ACID FROM ETHYLENE OXIDE AND CARBON MONOXIDE
PROCÉDÉ DE PRODUCTION D'ACIDE ACRYLIQUE À PARTIR D'OXYDE D'ÉTHYLÈNE ET DE MONOXYDE DE CARBONE

(30) Priorität: 16.07.2012 DE 102012212437; 16.07.2012 US 201261671852 P
(43) Veröffentlichungstag der Anmeldung: 20.05.2015
(73) Patentinhaber: BASF SE, 67056 Ludwigshafen am Rhein (DE)
(72) Erfinder: PAZICKY, Marek, 69121 Heidelberg (DE); RAITH, Christian, 68167 Mannheim (DE); PACIELLO, Rocco, 67098 Bad Dürkheim (DE); BRAND, Raphael, Heinrich, 64380 Roßdorf (DE); HARTMANN, Marco, 76744 Wörth (DE); MüLLER-ENGEL, Klaus, Joachim, 76297 Stutensee (DE); ZUROWSKI, Peter, 76829 Landau (DE); FISCHER, Wolfgang, 67360 Lingenfeld (DE)
(74) Vertreter: BASF IP Association
(86) Internationale Anmeldenummer: PCT/EP2013/064767
(87) Internationale Veröffentlichungsnummer: WO 2014/012855

(56) Entgegenhaltungen:
- WO-A1-2011/100608
- WO-A2-2011/163309
- DE-A1- 10 137 046

## Beschreibung

Vorliegende Erfindung betrifft ein Verfahren zur Herstellung von Acrylsäure aus Ethylenoxid und Kohlenmonoxid, das wenigstens folgende Verfahrensschritte umfasst:
- eine carbonylierende Umsetzung von in einem nicht protischen Lösungsmittel gelöstem Ethylenoxid mit Kohlenmonoxid bei erhöhtem Druck und erhöhter Temperatur in Anwesenheit eines Katalysatorsystems, das wenigstens eine Kobaltquelle umfasst, in einer Reaktionszone A unter Erhalt eines Poly-3-hydroxypropionat enthaltenden Produktgemischs A,
- eine Abtrennung von Poly-3-hydroxypropionat vom Produktgemisch A in einer Trennzone A, und
- eine Thermolyse von in der Trennzone A abgetrenntem Poly-3-hydroxypropionat in einer Thermolysezone A unter Bildung von Acrylsäure.

Acrylsäure ist ein bedeutendes Monomeres, das als solches, in Form seiner Alkylester und/oder in Gestalt seiner Alkalimetallsalze zur Herstellung von Polymerisaten Verwendung findet. In Abhängigkeit von den zum Aufbau des jeweiligen Polymerisats im Einzelnen verwendeten Acrylmonomeren kann dieses z.B. als Klebstoff oder als Superabsorber für Wasser bzw. wässrige Lösungen eingesetzt werden.

Es ist allgemein bekannt, dass die großtechnische Herstellung von Acrylsäure derzeit im Wesentlichen durch zweistufige heterogen katalysierte partielle Gasphasenoxidation von Propylen (eine alternative Bezeichnung = Propen) über das Zwischenprodukt Acrolein erfolgt (vgl. z.B. DE 10131297 A1 und WO 2004/031107 A1).

Als Ausgangspropylen wird dabei üblicherweise kein chemisch reines, sondern ein noch Verunreinigungen aufweisendes rohes Propylen verwendet, das aber eine vergleichsweise hohe Reinheit aufweist (z.B. "polymer grade" oder "chemical grade" Propylen; vgl. DE 10131297 A1).

Die Herstellung von solchem, vergleichsweise reinem Roh-Propylen ist relativ aufwändig und kostspielig. Die entsprechenden Produktionsverfahren gehen normalerweise von rohen paraffinischen Kohlenwasserstoffen aus und bedürfen in der Regel mehrerer Reinigungsstufen ihrer Produktgemische, in denen das Propylen von von Propylen verschiedenen Olefinen sowie von anderen von Propylen verschiedenen Nebenprodukten, die auch nicht umgesetzten rohen paraffinischen Kohlenwasserstoff und in diesem bereits enthaltene Nebenkomponenten umfassen, abgetrennt werden muss.

Die vorgenannten Abtrennungen sind in der Regel investitionsintensiv und in Folge der physikalischen Ähnlichkeit von olefinischen/paraffinischen Kohlenwasserstoffen mit vergleichbarer Kettenlänge auch besonders energieaufwändig. Sie werden daher üblicherweise nur im Verbund mit Raffineriecrackern und Steamcrackern angewendet und sind nur deshalb wirtschaftlich zu betreiben, weil die überwiegende Menge des so gewonnenen Roh-Propylens (als Propylen-Hauptbedarfsstrom) für nachfolgende Polymerisationen (z.B. zur Herstellung von Polypropylen) einerseits in großen Mengen benötigt wird (Stichwort: "economy of scale") und dabei andererseits eine hohe Wertschöpfung erfährt.

Der von diesen Roh-Propylenen in die heterogen katalysierte Partialoxidation zur Herstellung von Acrylsäure fließende Anteil ist von eher untergeordneter Bedeutung und bildet im Grunde nur einen mitproduzierten Nebenbedarfsstrom, der vom Hauptbedarfsstrom mitgetragen auch für die relevante Partialoxidation noch einen akzeptablen Rohstoffpreis aufweist.

Das im Rahmen des Crackens gesättigter Kohlenwasserstoffe gebildete Hauptprodukt ist jedoch Ethylen (eine alternative Bezeichnung = Ethen). Ethylen bildet die meistproduzierte organische Grundchemikalie und wird u.a. für die Herstellung von primären Folgeprodukten wie Polyethylen, Ethylenoxid (vgl. z.B. DE 2159346 A1), Styrol oder α-Olefinen verwendet.

In Europa und Asien wird Ethylen überwiegend auf Naphta- oder Gasöl-Basis produziert, in den Vereinigten Staaten, Kanada und dem Nahen Osten auch aus Ethan, Propan und Flüssiggas.

Eine Absenkung der mit der großtechnischen Herstellung von Acrylsäure einhergehenden Rohstoffkosten wäre daher dann möglich, wenn anstelle von der Rohstoffbasis "Propylen" zur Herstellung von Acrylsäure von der Rohstoffbasis "Ethylen" ausgegangen werden könnte.

Aus der Dissertation "Multi-Site Catalysis - Novel Strategies to Biodegradable Polyesters from Epoxides/CO und Macrocylic Complexes as Enzyme Models" von Markus Allmendinger, Universität Ulm (2003) ist bekannt, dass durch carbonylierende Umsetzung von in einem nicht protischen Lösungsmittel gelöstem Ethylenoxid mit Kohlenmonoxid (Kohlenmonoxid bildet ebenfalls einen kostengünstigen Rohstoff, der aus zahlreichen Kohlenstoff haltigen Rohmaterialien wie z.B. Erdgas, Biogas, Leichtbenzin, Schwerölen und Biomasse hergestellt werden kann) bei erhöhtem Druck, erhöhter Temperatur und in Anwesenheit eines wenigstens eine Kobaltquelle umfassenden Katalysatorsystems unmittelbar (d.h., ohne dass das Propiolacton (Oxetan-2-on) als intramolekularer cyclischer Ester der β-Hydroxypropionsäure (= 3-Hydroxypropionsäure) als Zwischenprodukt gebildet wird) ein Poly-3-hydroxypropionat enthaltendes Produktgemisch erhalten wird.

In J.Am.Chem.Soc. 2002, 124, Seiten 5646-5647, in der DE 10137046 A1, in der WO 03/011941 A2 und in J.Org.Chem. 2001, 66, Seiten 5424-5426 wird dieser Sachverhalt bestätigt.

Unter dem Begriff Poly-3-hydroxypropionat werden dabei Polyester der Struktur 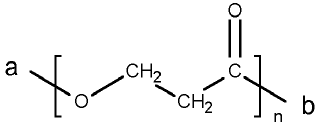 verstanden. n ist eine ganze Zahl ≥ 2, und kann z.B. bis zu 150, oder bis zu 200, oder bis zu 250 und mehr betragen.

a, b bilden den Polyester terminierende Endgruppen, deren Beschaffenheit von den Herstellbedingungen (z.B. vom verwendeten Katalysatorsystem) abhängig ist. Beispielsweise kann
a = 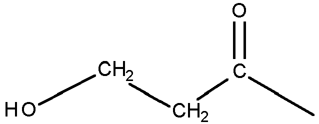 und
b= 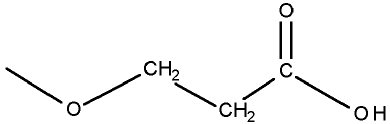 sein.

Alternativ kann
a = 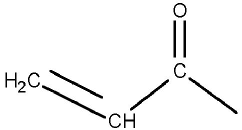 und
b= 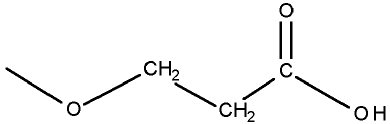 sein.

Aus der bereits erwähnten Dissertation von M. Allmendinger und aus der EP 577 206 A2 ist bekannt, dass u.a. bei längerem sich selbst überlassen des bei der Carbonylierung des Ethylenoxids im Beisein des Co-haltigen Katalysatorsystems gebildeten Produktgemischs wenigstens eine Teilmenge des in selbigem enthaltenen Poly-3-hydroxypropionats aus dem Produktgemisch ausfällt, und durch Anwendung einer mechanischen Trennoperation (z.B. Filtrieren) aus dem Produktgemisch abgetrennt werden kann. Gemäß Beispiel 7 der EP 577 206 A2 wird der durch Filtrieren vom Produktgemisch abgetrennte Niederschlag an Poly-3-hydroxypropionat anschließend noch mit Methanol gewaschen.

Aus der oben angeführten Dissertation von M. Allmendinger (beispielsweise in deren Experiment Teil X, Seite A179) und aus J.Am.Chem.Soc. 2002, 124, Seite 5646-5647 ist auch bekannt, dass die Fällung von Poly-3-hydroxypropionat aus dem dieses enthaltenden Produktgemisch alternativ durch Zusatz von Methanol zum Produktgemisch bewirkt werden kann.

Charakteristisch für die genannten Verfahren zur Abtrennung von Poly-3-hydroxypropionat vom bei der Carbonylierung des Ethylenoxids in Anwesenheit eines wenigstens eine Kobaltquelle umfassenden Katalysatorsystems anfallenden Produktgemisch ist, dass das isolierte Poly-3-hydroxypropionat noch einen nicht zu vernachlässigenden Gehalt an Kobalt aufweist.

Aus der EP 577 206 A2 ist bekannt, dass Poly-3-hydroxypropionat unter Einwirkung erhöhter Temperaturen (d.h., durch Thermolyse) in Acrylsäure (das Dehydrat der 3-Hydroxypropion-säure) gespalten wird. Die EP 577 206 A2 enthält jedoch kein Beispiel einer solchen Thermolyse.

Aus der WO 2011/100608 A1 ist ebenfalls bekannt, dass durch Thermolyse von Poly-3-hydro-xypropionat Acrylsäure erzeugt werden kann. Die Herstellung der Poly-(3-hydroxypropionsäure) erfolgt dabei biotechnologisch in gentechnisch modifizierten biologischen Organismen (z.B. aus Zuckern als nachwachsenden Rohstoffen), und damit in Abwesenheit von Co-haltigen Katalysatoren. Die thermische Spaltung von Poly-3-hydroxypropionat zu Acrylsäure ist ferner aus der US 2,568,636 A, aus der US 2,361,036 A und aus der US 3,002,017 A bekannt. Die dort für die Rückspaltung eingesetzten Polyester der β-Hydroxypropionsäure werden dabei ausgehend von β-Propiolacton (in Abwesenheit von Co-haltigen Katalysatoren) durch Ringöffnungspolymerisation gewonnen. Entsprechende Ringöffnungspolymerisationen offenbaren auch die EP 688 806 B1 und die WO 2011/163309 A2. Darüber hinaus kann zu Acrylsäure rückspaltbares Poly-3-hydroxypropionat in Abwesenheit von Co-haltigen Katalysatoren durch dehydratisierende Polykondensation von 3-Hydroxypropionsäure erhalten werden (vgl. z.B. Chinesische Zeitschrift für synthetische Chemie, Vol. 15 (2007) No. 4, Seite 452-453).

Nachteilig an diesen bekannten Verfahren zur Herstellung von Poly-3-hydroxypropionat als Grundlage für eine thermolytische Erzeugung von Acrylsäure, deren Durchführung des Beiseins eines Co-haltigen Katalysators nicht bedarf, ist, dass ihre Wirtschaftlichkeit nicht zu befriedigen vermag.

Als nachteilig an bei der Carbonylierung von Ethylen basiertem Ethylenoxid in Anwesenheit eines wenigstens eine Kobaltquelle umfassenden Katalysatorsystems anfallendem und gemäß den Vorgaben des Standes der Technik aus dem entstehenden Produktgemisch abgetrenntem Poly-3-hydroxypropionat hat sich dessen bereits erwähnter Gehalt an Kobalt erwiesen.

Eigene Untersuchungen der Anmelderin haben nämlich in überraschender Weise ergeben, dass im abgetrennten Poly-3-hydroxypropionat verbliebenes Kobalt dessen Thermolyse zu Acrylsäure wesentlich beeinträchtigt.

Die Aufgabe der vorliegenden Erfindung bestand daher darin, ein verbessertes Verfahren zur Herstellung von Poly-3-hydroxypropionat aus Ethylenoxid und Kohlenmonoxid im Beisein eines wenigstens eine Kobaltquelle umfassenden Katalysatorsystems zur Verfügung zu stellen, das insbesondere eine verbesserte Abtrennung des Poly-3-hydroxypropionats vom im Produktgemisch enthaltenen Kobalt umfasst.

Demgemäß wird ein Verfahren zur Herstellung von Acrylsäure aus Ethylenoxid und Kohlenmonoxid, das wenigstens folgende Verfahrensschritte umfasst:
- eine carbonylierende Umsetzung von in einem nicht protischen Lösungsmittel gelöstem Ethylenoxid mit Kohlenmonoxid bei erhöhtem Druck und erhöhter Temperatur in Anwesenheit eines Katalysatorsystems, das wenigstens eine Kobaltquelle umfasst, in einer Reaktionszone A unter Erhalt eines Poly-3-hydroxypropionat enthaltenden Produktgemischs A,
- eine Abtrennung von Poly-3-hydroxypropionat vom Produktgemisch A in einer Trennzone A, und
- eine Thermolyse von in der Trennzone A abgetrenntem Poly-3-hydroxypropionat in einer Thermolysezone A unter Bildung von Acrylsäure,
zur Verfügung gestellt, welches zusätzlich dadurch gekennzeichnet ist, dass die Abtrennung von Poly-3-hydroxypropionat vom Produktgemisch A in der Trennzone A wenigstens eine der nachfolgenden Verfahrensmaßnahmen umfasst:
- einen Zusatz von Wasser und/oder einer wässrigen Lösung als wässrige Fällflüssigkeit zu einer oder mehreren Teilmengen des Produktgemischs A und/oder zur Gesamtmenge des Produktgemischs A, um in einer Teilmenge des Produktgemischs A oder in der Gesamtmenge des Produktgemischs A gelöst enthaltenes Poly-3-hydroxypropionat auszufällen (und zu decobaltieren),
- ein Waschen von vom Produktgemisch A abgetrenntem Poly-3-hydroxypropionat mit Wasser und/oder mit einer wässrigen Lösung als wässrige Waschflüssigkeit (zum Zweck der Decobaltierung des abgetrennten Poly-3-hydroxypropionats).

Unter einem nicht protischen Lösungsmittel werden dabei definitionsgemäß (nicht protische) organische Substanzen = (chemische) Verbindungen (sowie Mischungen aus zwei oder mehr als zwei dieser Verbindungen (Substanzen)) verstanden, die bei einem Druck von 1,0133·10⁵ Pa (= Normaldruck) bei wenigstens einer der im Bereich von 0 °C bis 50 °C, vorzugsweise bei wenigstens einer der im Bereich von 5 °C bis 40 °C und besonders bevorzugt bei wenigstens einer der im Bereich von 10 °C bis 30 °C liegenden Temperaturen flüssig sind, kein von Kohlenstoff verschiedenes Atom (keine von Kohlenstoff verschiedene Atomsorte) enthalten, an das (an die) ein Wasserstoffatom kovalent gebunden ist, sowie weder ethylenisch noch alkinisch (jeweils ein- oder mehrfach) ungesättigt sind.

Der Siedepunkt von Ethylenoxid liegt bei Normaldruck bei einer Temperatur von 10,45 °C. Wird das Ethylenoxid beim erfindungsgemäßen Verfahren, bezogen auf die verfügbare molare Menge an CO und die Stöchiometrie der carbonylierenden Umsetzung (der Carbonylierung), im (molaren) Überschuss eingesetzt, kann das Ethylenoxid beim erfindungsgemäßen Verfahren auch selbst das erfindungsgemäß erforderliche nicht protische (= unprotische) Lösungsmittel bilden (bzw. Bestandteil eines solchen unprotischen Lösungsmittels sein, von einem solchen nicht protischen Lösungsmittel umfasst sein).

Als weitere für das erfindungsgemäße Verfahren geeignete unprotische (= nicht protische) Lösungsmittel seien beispielhaft genannt gesättigte (cyclische und nicht cyclische) und aromatische Kohlenwasserstoffe wie n-Hexan, n-Heptan, Petrolether, Cyclohexan, Benzol und Toluol, halogenierte gesättigte und aromatische Kohlenwasserstoffe wie Dichlormethan, Ester von organischen Säuren (insbesondere organischen Carbonsäuren) wie n-Butylpropionat, Phenylacetat, Glycerintriacetat und Ethylacetat, Anhydride von organischen Carbonsäuren wie das Essigsäureanhydrid, Ketone wie Aceton, Ethylmethylketon, Methylisobutylketon und Benzophenon, Nitrile wie Acetonitril, Propionitril, n-Butyronitril und Benzonitril, Dialkylamide wie Dimethylformamid und Dimethylacetamid, Kohlensäureester wie Dimethylcarbonat, Diethylcarbonat, Ethylmethylcarbonat, Ethylencarbonat und Propylencarbonat, Sulfoxide wie Dimethylsulfoxid, Sulfone wie Sulfolan, N-Alkylpyrrolidone wie N-Methylpyrrolidon sowie cyclische und nicht cyclische Ether wie Diethylether, Anisol, Tetrahydrofuran, 1,4-Dioxan, Diphenylether, Alkylenglykol-dialkylether (z.B. Ethylenglykoldialkylether wie der Ethylenglykoldimethylether) und Polyalkylenglykoldialkylether wie der Diethylenglykoldiethylether, der Diethylenglykoldimethylether (= Diglyme), der Triethylenglykoldimethylether (= Triglyme) und der Tetraethylenglykoldimethylether (= Tetraglyme).

Protische Lösungsmittel (= (protische) Verbindungen = (protische) Substanzen und deren Gemische, die wenigstens ein von Kohlenstoff verschiedenes Atom enthalten, an das ein Wasserstoffatom kovalent gebunden ist), können an der dem erfindungsgemäßen Verfahren unterliegenden Umsetzung teilnehmen und zu unerwünschter Nebenproduktbildung führen, weshalb sie zur Verwendung für das erfindungsgemäße Verfahren weniger geeignet sind. (Methanol, Ethanol, n-Butanol, tert. Butanol, Cyclohexanol und sonstige Alkohole sind z.B. keine unprotischen (= keine nicht protischen) Substanzen sondern protische Lösungsmittel). Ebenso sind Lösungsmittel, bei denen es sich um Substanzen handelt, die ethylenisch und/oder alkinisch ungesättigt sind, keine unprotischen Lösungsmittel im erfindungsgemäßen Sinn.

Erfindungsgemäß bevorzugte nicht protische (= unprotische) Lösungsmittel sind solche, die Substanzen umfassen, die wenigstens ein kovalent gebundenes Sauerstoffatom aufweisen. Dies gilt insbesondere dann, wenn es sich um wenigstens ein Ethersauerstoffatom (ein Sauerstoffatom, das eine Etherbrücke bildet) handelt.

Ferner ist es erfindungsgemäß vorteilhaft, wenn das unprotische Lösungsmittel eine (oder mehrere) Substanz ist (bzw. eine oder mehrere solche Substanzen umfasst), die als von Kohlenstoff und Wasserstoff verschiedene Atomsorte höchstens Sauerstoff und/oder Schwefel enthält.

Weist die als unprotisches Lösungsmittel beim erfindungsgemäßen Verfahren mitverwendete Substanz z.B. ein oder mehrere tertiäre Stickstoffatome auf, so können diese dem beim erfindungsgemäßen Verfahren angestrebten Polyesteraufbau in begrenztem Umfang entgegenwirken.

Bei den beim erfindungsgemäßen Verfahren erzeugten Poly-3-hydroxypropionaten handelt es sich um Polyester, die eine mittlere Polarität aufweisen.

Vorzugsweise umfassen (bzw. sind) erfindungsgemäß geeignete nicht protische Lösungsmittel daher solche Substanzen (Verbindungen), deren relative statische Permittivität (= deren Dielektrizitätskonstante = deren Dielektrizitätszahl = deren Permittivitätszahl) ε bei einer Temperatur von 293,15 K und einem Druck von 1,0133·10⁵ Pa (= Normaldruck) als flüssige Reinsubstanz im Bereich von 2 bis 35, vorzugsweise 3 bis 20, besonders bevorzugt 4 bis 15 und ganz besonders bevorzugt 5 bis 10 liegt (die relative statische Permittivität von Vakuum = 1).

Ist die relevante unprotische Substanz bei 293,15 K und Normaldruck nicht flüssig, sondern fest, so bezieht sich vorstehende Angabe auf die Temperatur ihres Schmelzpunkts bei Normaldruck. Ist die relevante unprotische Substanz (unprotische (chemische) Verbindung) bei 293,15 K und Normaldruck nicht flüssig sondern gasförmig, so bezieht sich vorstehende Angabe auf eine Temperatur von 293,15 K und den zugehörigen Sättigungsdampfdruck (den (Eigen-) Dampfdruck, bei dem die Substanz bei 293,15 K kondensiert).

Eine geeignete Quelle mit Angaben zu relativen statischen Permittivitäten von erfindungsgemäß relevanten unprotischen Substanzen ist z.B. das HANDBOOK of CHEMISTRY and PHYSICS, 92th Edition (2010-2011), CRC PRESS. Gemäß den dortigen Angaben beträgt das relevante ε z.B. für Tetrahydrofuran = 7,56, für Ethylenoxid = 12,43, für 1,4-Dioxan = 2,22, für Ethylenglykoldimethylether (1,2-Dimethoxyethan) = 7,41, für Diethylenglykoldimethylether (Diglyme) = 7,38 und für Triethylenglykoldimethylether (Triglyme) = 7,62.

Erfindungsgemäß ganz besonders bevorzugte unprotische Lösungsmittel sind daher solche, die erfindungsgemäß relevante unprotische organische Verbindungen umfassen, deren relevantes ε 2 bis 35, vorteilhaft 3 bis 20, besonders vorteilhaft 4 bis 15, und ganz besonders vorteilhaft 5 bis 10 beträgt, und die gleichzeitig wenigstens ein kovalent gebundenes Sauerstoff enthalten, bei dem es sich mit besonderem Vorteil um ein Ethersauerstoffatom handelt.

Ethylenglykoldimethylether, Diethylenglykoldimethylether und Triethylenglykoldimethylether sowie beliebige Gemische derselben umfassende bzw. aus diesen bestehende nicht protische Lösungsmittel sind deshalb für das erfindungsgemäß Verfahren ganz besonders geeignete unprotische Lösungsmittel, unter denen der Diethylenglykoldimethylether nochmals bevorzugt ist.

Selbstverständlich umfasst der Begriff "nicht protische Lösungsmittel" im Sinn der vorliegenden Erfindung auch noch solche Lösungsmittel, die nicht zu 100% ihres Gewichts (sondern nur zu 90% ihres Gewichts, oder nur zu 95% ihres Gewichts, oder nur zu 98% ihres Gewichts, oder nur zu 99% ihres Gewichts) aus definitionsgemäßen unprotischen organischen Verbindungen (unprotischen organischen Substanzen, unprotischen organischen Lösungsmitteln) bestehen.

D.h., es ist für die Zwecke der vorliegenden Erfindung bereits ausreichend und vom Begriff "unprotisches Lösungsmittel" umfasst, wenn das eingesetzte "nicht protische Lösungsmittel" zu wenigstens 90 Gew.-%, vorzugsweise zu wenigstens 95 Gew.-%, besonders bevorzugt zu wenigstens 98 Gew.-%, und ganz besonders bevorzugt zu wenigstens 99 Gew.-% (jeweils bezogen auf das Gewicht des Lösungsmittels) aus definitionsgemäßen unprotischen organischen Verbindungen besteht.

Ein solcher nicht definitionsgemäßer Bestandteil des nicht protischen Lösungsmittels können z.B. protische Substanzen wie Wasser oder Alkohole sein. Da sie z.B. im Wege einer "Esterbil-dung" eine Terminierung einer im erfindungsgemäßen Verfahren wachsenden Poly-3-hydroxy-propionatkette bedingen können, kann durch ein kontrolliertes Beisein von solchen protischen Substanzen das mittlere Molekulargewicht des bei der Durchführung des erfindungsgemäßen Verfahrens resultierenden Poly-3-hydroxpropionats gezielt beeinflusst werden (vgl. z.B. DE 10137046 A1). Mit zunehmender (z.B. durch Zusatz von "Wasserfängern", wie es die DE 10137046 A1 empfiehlt, erzeugter (bewirkter)) Abwesenheit solcher protischer Substanzen resultieren unter ansonsten gleichen Umsetzungsbedingungen zunehmend höhere mittlere Molekulargewichte des Poly-3-hydroxpropionats.

Die Terminierung einer im Rahmen des erfindungsgemäßen Verfahrens wachsenden Poly-3-hydroxpropionatkette durch eine protische Substanz wie z.B. Wasser ist erfindungsgemäß einerseits in der Regel insofern vorteilhaft, als bei der Terminierung (beim Kettenabbruch) vermutlich eine Bindung der wachsenden Polyesterkette zum Kobalt des Katalysatorsystems gebrochen wird.

Andererseits geht mit einer solchen Terminierung der Nachteil einher, dass bei der erfindungsgemäßen Thermolyse des Poly-3-hydroxpropionats z.B. das eine solche Terminierung verursachende Wasser gegebenenfalls wieder abgespalten und damit wieder freigesetzt wird. Erfolgt die erfindungsgemäße carbonylierende Umsetzung unter völligem Ausschluss von solchen protischen Substanzen (d.h., z.B. unter Ausschluss von Luftfeuchtigkeit und sonstigen Spuren von Wasser), kann die beschriebene Kettenterminierung auch erst im Rahmen einer erfindungsgemäßen Fällung und/oder Waschung des gebildeten Poly-3-hydroxpropionats erfolgen.

Typische relative (d.h., auf das Gewicht von atomarem Wasserstoff bezogene) gewichtsmittlere Molekulargewichte Mw von im Rahmen eines erfindungsgemäßen Verfahrens erhältlichem Poly-3-hydroxpropionat (d.h., von von einem Produktgemisch A abgetrenntem Poly-3-hydroxypro-pionat) können z.B. 1000 bis 20000 oder 2000 bis 15000, vielfach 3000 bis 12000 und häufig 4000 bis 10000 betragen. Grundsätzlich sind aber auch höhere und niedrigere relative gewichtsmittlere Molekulargewichte M_{w} möglich. Die zugehörige Polydispersität Q (das Verhältnis von gewichtsmittlerem relativem Molekulargewicht Mw zu zahlenmittlerem relativem Molekulargewicht Mₙ (Q = Mw /Mₙ)) liegt im Allgemeinen bei Werten ≤ 2,5, häufig bei Werten ≤ 2. In vielen Fällen beträgt Q 1,5 bis 1,8. Es können aber auch Polydispersitäten Q unterhalb von 1,5 oder unterhalb von 1,4 eingestellt werden (vgl. DE 10137046 A1).

Als weitere nicht definitionsgemäße Bestandteile eines erfindungsgemäßen nicht protischen Lösungsmittels kommen z.B. herstellungsbedingte Verunreinigungen desselben in Betracht.

Da Ethylenoxid selbst eine mittlere Polarität aufweist (der relevante Wert für ε = 12,43), ist dessen ausreichende Löslichkeit in den erfindungsgemäß zu verwendenden nicht protischen Lösungsmittel normalerweise unkritisch.

D.h., Art und Menge an erfindungsgemäß einzusetzendem nicht protischem Lösungsmittel werden in der Regel primär daran ausgerichtet, dass sie unter den erfindungsgemäß anzuwendenden Reaktionsbedingungen ausreichend sind, die erfindungsgemäß erforderliche Menge des Kobalt enthaltenden Katalysatorsystems im Reaktionsgemisch in Lösung zu halten, da das erfindungsgemäße Verfahren vorzugsweise homogen katalysiert durchgeführt wird.

Bezogen auf die bei der Durchführung des erfindungsgemäßen Verfahrens carbonylierend umzusetzende molare Menge an Ethylenoxid, liegt die molare Einsatzmenge an in der wenigstens einen Kobaltquelle des Katalysatorsystems enthaltenem Co bei der erfindungsgemäßen Verfahrensweise normalerweise im Bereich von 0,005 bis 20 mol-%, vorzugsweise im Bereich von 0,05 bis 10 mol-%, besonders bevorzugt im Bereich von 0,1 bis 8 mol-% und ganz besonders bevorzugt im Bereich von 0,5 bis 5 mol-%. Bei entsprechend bezogenen Einsatzmengen an in der wenigstens einen Kobaltquelle des Katalysatorsystems enthaltenem Co von ≤ 0,0001 mol-% findet im Regelfall im Wesentlichen keine erfindungsgemäße carbonylierende Umsetzung mehr statt ("Mehrzentren-Katalyse").

Als erfindungsgemäß geeignete Kobaltquelle kann für das erfindungsgemäße Verfahren im Wesentlichen eine beliebige Kobalt enthaltende chemische Verbindung eingesetzt werden, da diese unter dem im erfindungsgemäßen Verfahren anzuwendenden Kohlenmonoxiddruck in der Regel jeweils in die eigentliche katalytisch wirksame Verbindung des Kobalts umgewandelt wird.

Unter Anderem kommen als erfindungsgemäß geeignete Kobaltquellen z.B. Kobaltsalze wie Kobaltformiat, Kobaltacetat, Kobaltacetylacetonat und Kobaltsulfat in Betracht, die unter den erfindungsgemäß anzuwendenden Kohlenmonoxiddrücken leicht carbonyliert werden ("in situ"; ein Beisein von geringen Mengen an molekularem Wasserstoff kann sich diesbezüglich vorteilhaft auswirken). Aber auch fein verteiltes Kobaltmetall (z.B. in Staubform) kann beim erfindungsgemäßen Verfahren als Kobaltquelle verwendet werden.

Erfindungsgemäß bevorzugt werden als Kobaltquellen bereits vorgebildete Kobalcarbonylverbindungen (darunter werden Verbindungen verstanden, die wenigstens ein Kobaltatom und wenigstens einen Kohlenmonoxid-Liganden enthalten) verwendet, unter denen das Dikobaltoctacarbonyl (Co₂(CO)₈) ganz besonders bevorzugt ist (dieses enthält als [Co(CO)₄⁺Co(CO)₄-] das Co(CO)₄- gleichsam vorgebildet). Anwendungstechnisch zweckmäßig wird es als alleinige Kobaltquelle des Katalysatorsystems eingesetzt.

Ohne an die folgende Annahme gebunden zu sein, wird vermutet, dass eine wesentliche katalytisch aktive Spezies beim Kettenstart eines erfindungsgemäßen Verfahrens das Hydrotetracobaltcarbonyl (HCo(CO)₄) ist (vgl. z.B. DE 10137046 A1).

Die Bildung von Hydrotetracobaltcarbonyl aus Kobaltcarbonylverbindungen wie Co(CO)₈ setzt in der Regel immer dann ein, wenn im Reaktionsgemisch Verbindungen zugegen sind, die Wasserstoff gebunden aufweisen. Sogar Aminen und Paraffinen kann durch Kobaltcarbonylverbindungen wie Co₂(CO)₈ Wasserstoff entrissen werden (vgl. Organische Chemie in Einzeldarstellungen 10, Jürgen Falbe, Synthesen mit Kohlenmonoxyd, Springer Verlag (1967), S.14). Ein Beisein von geringen Mengen an molekularem Wasserstoff kann sich hier gegebenenfalls vorteilhaft auswirken.

Durch eine Mitverwendung von geringen Mengen einer (oder mehrerer) Brönsted-Säuren (die Bezugsbasis für die Eigenschaft "Brönsted-Säure" sind in dieser Schrift 25°C und Normaldruck sowie Wasser als Reaktionspartner der Brönsted-Säure; d.h., die Zugabe einer Brönsted-Säure zu Wasser (bei 25 °C und Normaldruck) ergibt eine wässrige Lösung, die bei den genannten Bedingungen einen niedrigeren pH-Wert aufweist als das reine Wasser) als co-Katalysatoren (auch "Promotoren" genannt) kann die Bildung von Hydrotetracobaltcarbonyl als gegebenenfalls geschwindigkeitsbestimmender Schritt begünstigt und die erwünschte erfindungsgemäße Polyesterbildung so beschleunigt werden (vgl. DE 10149269 A1 und DE 10137046 A1).

Als solchermaßen erfindungsgemäß geeignete Brönsted-Säuren ("co-Katalysatoren A" bzw. "Promotoren A") kommen die üblichen Mineralsäuren (anorganischen Säuren) wie Salzsäure, Schwefelsäure oder Phosphorsäure in verdünnter sowie insbesondere in konzentrierter Form, organische Carbonsäuren wie z.B. Alkanmono- und polycarbonsäuren (z.B. Ameisensäure, Essigsäure, Adipinsäure und Glutarsäure), deren halogenierte Abkömmlinge wie z.B. Trichlor-und Trifluoressigsäure sowie aromatische Carbonsäuren wie z.B. Benzoesäure und 2,4,6-Trimethylbenzoesäure, organische Sulfonsäuren wie p-Toluolsulfonsäure, hydroxyaromatische Verbindungen wie Phenol, 1-Naphthol und 2-Naphthol, aber auch Wasser (Wasser wird in dieser Schrift willkürlich den Brönsted-Säuren zugeteilt) in Frage.

Phenol und Essigsäure bilden für den vorgenannten Zweck erfindungsgemäß bevorzugte Brönsted-Säuren. Essigsäure wird dabei vorzugsweise in Form des so genannten Eisessigs zugesetzt. Hierunter soll vorwiegend wasserfreie bzw. nahezu wasserfreie Essigsäure verstanden werden (vorzugsweise beträgt deren Wasseranteil ≤ 1 Gew.-%, besonders bevorzugt ≤ 0,5 Gew.-%, vorteilhaft ≤ 0,1 Gew.-% und ganz besonders bevorzugt ≤ 0,01 Gew.-%). Salzsäure wird vorzugsweise im erfindungsgemäß mitzuverwendenden nicht protischen Lösungsmittel gelöst mitverwendet. Wie bereits festgestellt, können für den vorstehend ausgeführten erfindungsgemäßen Zweck auch beliebige Gemische der angeführten Brönsted-Säuren zur Anwendung kommen.

In der Regel wird man Brönsted-Säuren ("Promotoren A", "co-Katalysatoren A") beim erfindungsgemäßen Verfahren insgesamt in solchen molaren Gesamtmengen M_{A} als co-Katalysatoren A mitverwenden, dass das Verhältnis M_{A} : M_{Co}, gebildet mit der im verwendeten Katalysatorsystem insgesamt enthaltenen molaren Gesamtmenge M_{Co} an Co (diese Ausdrucksweise meint in dieser Schrift stets die in der Gesamtmenge aller Kobaltquellen eines Katalysatorsystems insgesamt enthaltene molare Menge an Co (Atomen + Ionen)), 5 : 1 bis 1 : 5, vorzugsweise 4 : 1 bis 1 : 4, besonders bevorzugt 3 : 1 bis 1 : 3 und ganz besonders bevorzugt 2 : 1 bis 1 : 2 beträgt.

Bemerkenswerterweise bilden in geringen Mengen mitverwendete Brönsted-Basen (die Bezugsbasis für die Eigenschaft "Brönsted-Base" sind in dieser Schrift 25°C und Normaldruck sowie Wasser als Reaktionspartner der Brönsted-Base; d.h., die Zugabe einer Brönsted-Base zu Wasser (bei 25 °C und Normaldruck) ergibt eine wässrige Lösung, die bei den genannten Bedingungen einen höheren pH-Wert aufweist als das reine Wasser) ebenfalls erfindungsgemäß geeignete co-Katalysatoren ("Promotoren B" bzw. "co-Katalysatoren B"), vgl. z.B. US 2,820,059 A; Organische Chemie in Einzeldarstellungen 10, Jürgen Falbe, Synthesen mit Kohlenmonoxyd, Springer Verlag (1967), Seite 16; DE 10137046; US 3,260,738 A; und DE 2901347 A1).

Es wird vermutet, dass sie als nucleophile Substanzen bei der erfindungsgemäßen Polyesterbildung das Kettenwachstum begünstigen (vgl. J.Am.Chem.Soc. 2002, 124, Seiten 5646-5647). Dies gilt insbesondere dann, wenn ihr pK_{B}-Wert (bezogen auf 25 °C, Normaldruck und Wasser) als Maß für die zugehörige Brönsted-Basenstärke ≥ 7, vorteilhaft ≥ 8, vorzugsweise ≥ 9 und besonders bevorzugt ≥ 10 beträgt, es sich also um schwache Brönsted-Basen handelt. In der Regel beträgt ihr pK_{B}-Wert ≤ 30, oft ≤ 25.

Das zugehörige nucleophile Zentrum der Brönsted-Base ist vorzugsweise wenigstens ein Sauerstoffatom (wie z.B. im Fall des Acetations), ein Phosphoratom (wie z.B. beim Triphenylphosphin) und/oder wenigstens ein Stickstoffatom (wie z.B. beim Anilin oder Pyridin). Aber auch Halogenidanionen wie J⁻ , Cl⁻ und F können derartige Nucleophile bilden.

Erfindungsgemäß als nucleophile co-Katalysatoren B besonders geeignete Brönsted-Basen sind aromatische und nicht aromatische zyklische Verbindungen, die im Ring neben Kohlenstoffatomen wenigstens ein (häufig auch 2, oder 3) Stickstoffatom(e) aufweisen (aromatische Stickstoff-Heterozyklen und Stickstoff-Heteroalizyklen). Beispielsweise können die Ringe 5-, 6-oder 7-gliedrig sein.

Als erfindungsgemäß geeignete Promotoren B seien beispielhaft genannt Pyrrol, N-Methyl-2-pyrrolidon, 2-Pyrrolidon, 3-Pyrrolidon, Piperidin, N-Methylpiperidin, Indol, Indolin, Imidazol, Pyrazol, N,N-Dimethylformamid, Acetanilid und N-Methyl-Imidazol.

Erfindungsgemäß bevorzugte Promotoren B sind schwach basische Stickstoff-Heterozyklen wie Pyrrol, N-Methyl-2-pyrrolidon, 2-Pyrrolidon, 3-Pyrrolidon, Piperidin, N-Methylpiperidin, Imidazol, Pyrazol und N-Methyl-Imidazol.

Selbstverständlich kann der aromatische bzw. nicht aromatische Stickstoff-Heterozyklus auch mit aliphatischen oder aromatischen Ringsystemen (z.B. 5-, 6-, oder 7-gliedrigen) anelliert sein, die ihrerseits ebenfalls ein- oder mehrere Stickstoff(hetero)atome aufweisen können. Beispielhaft genannt seien Indol, Indolin, Chinolin, Isochinolin, Chinoxalin, 1,10-Phenantrolin sowie 2,2'-, 2,3'-, 3,3'-, 2,4'-, 3,4'- und 4,4'-Bipyridin.

In der Regel wird man Promotoren B (Brönsted-Basen) beim erfindungsgemäßen Verfahren insgesamt in solchen molaren Gesamtmengen M_{B} als co-Katalysatoren B mitverwenden, dass das Verhältnis M_{B} : M_{Co}, gebildet mit der im eingesetzten Katalysatorsystem insgesamt enthaltenen molaren Gesamtmenge M_{Co} an Co, 5 : 1 bis 1 : 5, vorzugsweise 4 : 1 bis 1 : 4, besonders bevorzugt 3 : 1 bis 1 : 3 und ganz besonders bevorzugt 2 : 1 bis 1 : 2 beträgt. Zu große Überschüsse an Promotoren B gegenüber der vorhandenen molaren Menge an Co sollten vermieden werden, da die co-katalytische Wirkung mit zunehmendem Überschuss in eine Hemmung übergehen kann.

Erfindungsgemäß vorteilhaft umfasst ein erfindungsgemäß zu verwendendes Katalysatorsystem neben wenigstens einer Kobaltquelle zusätzlich noch wenigstens einen co-Katalysator A und wenigstens einen co-Katalysator B (z.B. die Kombination aus Phenol als co-Katalysator A und aus Pyridin als co-Katalysator B).

Das Verhältnis M_{A} : M_{B}, gebildet aus der molaren Gesamtmenge M_{A} an im Reaktionsgemisch enthaltenen co-Katalysatoren A sowie aus der molaren Gesamtmenge M_{B} an im Reaktionsgemisch enthaltenen co-Katalysatoren B beträgt dabei erfindungsgemäß vorteilhaft 1 : 4 bis 4 : 1, besonders vorteilhaft 1 : 2 bis 2 : 1 und häufig 1 : 1.

Erfindungsgemäß ganz besonders vorteilhaft ist die Mitverwendung von solchen Verbindungen als co-Katalysatoren C (wenigstens einem) im erfindungsgemäß einzusetzenden, wenigstens eine Kobaltquelle umfassenden Katalysatorsystem, die sowohl wenigstens eine nucleophile brönstedbasische (im Sinn von Brönsted basische) Funktionalität wie ein co-Katalysator B, als auch wenigstens eine brönstedsaure (im Sinne von Brönsted saure) Funktionalität wie ein co-Katalysator A aufweisen (d.h., enthielten diese Verbindungen nur die wenigstens eine brönstedsaure (nur die wenigstens eine brönstedbasische) Funktionalität und keine brönstedbasische (brönstedsaure) Funktionalität, würden sie eine Brönstedsäure, d.h., einen co-Kata-lysator A (eine Brönstedbase, d.h., einen co-Katalysator B) bilden.

Zu diesen co-Katalysatoren C ("Promotoren C") gehören insbesondere aromatische Stickstoff-Heterozyklen (diese können z.B. 5-, 6- oder 7-gliedrige Ringe sein; sie weisen wenigstens ein Stickstoffatom im aromatischen Ring (Zyklus) auf), die im Molekül zusätzlich zum brönstedbasischen Stickstoff wenigstens eine brönstedsaure (freie) Hydroxylgruppe (-OH) und/oder wenigstens eine brönstedsaure (freie) Carboxylgruppe (-COOH) kovalent gebunden aufweisen. Selbstverständlich kann der aromatische Stickstoff-Heterozyklus dabei wieder mit anderen aromatischen und/oder aliphatischen (z.B. 5-, 6- oder 7-gliedrigen) Ringsystemen anelliert sein. Die wenigstens eine Hydroxylgruppe und/oder Carboxylgruppe kann sich dabei sowohl am aromatischen Grund-Stickstoff-Heterozyklus (bevorzugt) als auch (und/oder) am anellierten aliphatischen und/oder aromatischen Ringsystem befinden. Selbstverständlich kann auch der anellierte Teil ein oder mehr als ein Stickstoffatom als Heteroatom aufweisen. Neben der wenigstens einen Hydroxylgruppe und/oder Carboxylgruppe können zusätzlich auch noch z.B. aliphatische, aromatische und/oder Halogensubstituenten vorhanden sein.

Als erfindungsgemäß besonders bevorzugte co-Katalysatoren C seien beispielhaft genannt 2-Hydroxypyridin, 3-Hydroxypyridin, 4-Hydroxypyridin, 3,4-Dihydroxypyridin, 3-Hydroxychinolin, 4-Hydroxy-2-Methylpyridin, 3-Hydroxy-4-Methylpyridin, 2,6-Dihydroxypyridin, 2-Hydroxychinolin, 1-Hydroxyisochinolin, 3-Hydroxychinolin, 2,3-Dihydroxychinoxalin, 8-Hydroxychinolin, 2-Pyridylmethanol, 3-Pyridylmethanol und 2-(2-Pyridyl)ethanol. Selbstverständlich kann, wie bereits gesagt, anstelle und/oder zusätzlich zur Hydroxylgruppe eine Carboxylgruppe vorhanden sein, wie es im Fall der Nicotinsäure gegeben ist. Ganz besonders bevorzugt wird als co-Katalysator C beim erfindungsgemäßen Verfahren 3-Hydroxypyridin eingesetzt (und dies insbesondere in Kombination mit Diglyme als unprotischem Lösungsmittel und Dicobaltoctacarbonyl als Kobaltquelle des Katalysatorsystems).

In der Regel wird man Promotoren C beim erfindungsgemäßen Verfahren in solchen molaren Gesamtmengen M_{C} als co-Katalysatoren C mitverwenden, dass das Verhältnis M_{C} : M_{Co}, gebildet mit der im eingesetzten Katalysatorsystem insgesamt enthaltenen molaren Gesamtmenge M_{Co} an Co, 5 : 1 bis 1 : 5, vorzugsweise 4 : 1 bis 1 : 4, besonders bevorzugt 3 : 1 bis 1 : 3 und ganz besonders bevorzugt 2 : 1 bis 1 : 2 bzw. 2 : 1 bis 1 : 1 beträgt. Mit Vorteil umfasst ein wenigstens einen Promotor C enthaltendes Katalysatorsystem weder einen Promotor A noch einen Promotor B (selbstverständlich können solche aber zusätzliche Bestandteile des Katalysatorsystems sein).

In der folgenden Tabelle 1 werden beispielhaft für das erfindungsgemäße Verfahren geeignete Katalysatorsysteme, einschließlich einer geeigneten carbonylierend umzusetzenden Menge an Ehtylenoxid (EO) sowie einer für das resultierende Reaktionsgemisch angemessenen Menge des nicht protischen Lösungsmittels Diglyme (LM), bezüglich ihrer Einsatzmenge(nverhältnisse) quantifiziert. Die verwendete Kobaltquelle Q ist dabei stets Co₂(CO)₈.

**Tabelle 1**

| **LM** | **EO** | **Q** | **co-Katalysatoren** |
|---|---|---|---|
| 37,6 g | 0,2 mol | 1 mmol | 4 mmol 3-Hydroxypyridin |
| 94 g | 0,6 mol | 2 mmol | 4 mmol 3-Hydroxypyridin |
| 75,2 g | 0,4 mol | 2 mmol | 7,9 mmol 3-Hydroxypyridin |
| 9,4 g | 0,1 mol | 0,32 mmol | 1,6 mmol 3-Hydroxypyridin |
| 9,4 g | 0,11 mol | 0,32 mmol | 0,64 mmol 3-Hydroxypyridin |
| 75,2 g | 0,4 mol | 2 mmol | 4 mmol 3-Hydroxypyridin |
| 75,2 g | 0,4 mol | 2 mmol | 8 mmol 3-Hydroxypyridin |
| 75,2 g | 0,4 mol | 2 mmol | 0,5 mmol 3-Hydroxypyridin |
| 75,2 g | 0,4 mol | 2 mmol | 8 mmol 3-Hydroxypyridin |
| | | | 8 mmol Essigsäure |
| 75,2 g | 0,4 mol | 2 mmol | 4 mmol Pyridin |
| | | | 8 mmol Essigsäure |
| 75,2 g | 0,4 mol | 2 mmol | 4 mmol Pyridin |
| | | | 8 mmol Phenol |
| 75,2 g | 0,4 mol | 2 mmol | 8 mmol 2,6-Difluoropyridin |
| | | | 8 mmol Phenol |
| 75,2 g | 0,4 mol | 2 mmol | 4 mmol Anilin |
| | | | 8 mmol Essigsäure |
| 75,2 g | 0,4 mol | 2 mmol | 4 mmol 2-Methylpyridin |
| | | | 8 mmol Essigsäure |
| 75,2 g | 0,4 mol | 2 mmol | 2 mmol 2,2'-Bipyridin |
| | | | 8 mmol Essigsäure |
| 75,2 g | 0,4 mol | 2 mmol | 4 mmol 2,2'-Bipyridin |
| | | | 8 mmol Essigsäure |
| 75,2 g | 0,4 mol | 2 mmol | 4 mmol 2,2'-Bipyridin |
| | | | 12 mmol Essigsäure |
| 75,2 g | 0,4 mol | 2 mmol | 4 mmol 2,2'-Bipyridin |
| | | | 8 mmol Phenol |
| 75,2 g | 0,4 mol | 2 mmol | 2 mmol 1,10-Phenanthrolin |
| | | | 8 mmol Essigsäure |
| 75,2 g | 0,4 mol | 2 mmol | 2 mmol 1,10-Phenanthrolin |
| | | | 8 mmol Phenol |
| 75,2 g | 0,4 mol | 2 mmol | 4 mmol Imidazol |
| | | | 8 mmol Methanol |
| 75,2 g | 0,4 mol | 2 mmol | 8 mmol Pyrazol |
| 75,2 g | 0,4 mol | 2 mmol | 4 mmol CsF |
| | | | 4 mmol Essigsäure |
| 75,2 g | 0,4 mol | 2 mmol | 4 mmol CsF |
| | | | 8 mmol Essigsäure |
| 75,2 g | 0,4 mol | 2 mmol | 4 mmol Cs-Acetat |
| | | | 8 mmol Essigsäure |
| 75,2 g | 0,4 mol | 2 mmol | 8 mmol Cs-Acetat |
| | | | 16 mmol Essigsäure |
| 75,2 g | 0,4 mol | 2 mmol | 4 mmol K-Acetat |
| | | | 8 mmol Essigsäure |
| 75,2 g | 0,4 mol | 2 mmol | 4 mmol K-Acetat |
| | | | 4 mmol Essigsäure |
| 75,2 g | 0,4 mol | 2 mmol | 4 mmol Na-Acetat |
| | | | 4 mmol Essigsäure |
| 9,4 g | 0,125 mol | 0,41 mmol + 0,825 mmol Et₄N Co(CO)₄ | 0,825 mmol Essigsäure |
| 75,2 g | 0,4 mol | 2 mmol | 4 mmol Li-Acetat |
| | | | 4 mmol Essigsäure |
| 75,2 g | 0,4 mol | 2 mmol | 4 mmol Cs-Acetat |
| | | | 8 mmol Trifluoressigsäure |
| 75,2 g | 0,4 mol | 2 mmol | 4 mmol Cs-Aceat |
| | | | 8 mmol p-Toluolsulfonsäure |
| 75,2 g | 0,4 mol | 2 mmol | 4 mmol Cs-Acetat |
| | | | 8 mmol 2,4,6-Trimethylbenzoesäure |

Kohlenmonoxid wird in allen vorstehenden Fällen vorzugsweise im Überschuss (relativ zur Re-aktionsstöchiometrie) eingesetzt.

Selbstverständlich können für ein erfindungsgemäßes Verfahren auch Salze des Anions Co(CO)₄⁻ und/oder dessen Brönstedsäure HCo(CO) als Kobaltquellen mitverwendet werden. Beispiele für derartige Salze sind das Tetramethylammoniumtetracarbonylcobaltat(-1) = Et₄NCo(CO)₄ und das Bis(triphenylphosphoranyliden)ammoniumtetracarbonylcobaltat(-1). Weitere Beispiele offenbart z.B. die DE 10149269 A1.

Die bei der erfindungsgemäßen carbonylierenden Umsetzung anzuwendende Reaktionstemperatur sowie der bei der erfindungsgemäßen carbonylierenden Umsetzung anzuwendende Arbeitsdruck sind nicht kritisch und können innerhalb weiter Grenzen variieren. Es ist ein vorteilhaftes Merkmal des erfindungsgemäßen Verfahrens, das die carbonylierende Umsetzung bei vergleichsweise milden Reaktionsbedingungen ausgeführt werden kann. Geeignete Reaktionstemperaturen liegen im Bereich von 25 bis 150 °C, vorzugsweise im Bereich von 35 bzw. 50 bis 120 °C, besonders bevorzugt im Bereich von 60 bis 100 °C und ganz besonders bevorzugt im Bereich von 70 bis 90 °C. Bei vergleichsweise geringen Temperaturen verläuft die carbonylierende Umsetzung zwar mit einer etwas verminderten Reaktionsgeschwindigkeit, jedoch mit einer vergleichsweise erhöhten Zielproduktselektivität, die nahe bei 100 mol-% liegt.

Durch überatmosphärische Arbeitsdrücke wird die erfindungsgemäße carbonylierende Umsetzung begünstigt. Anwendungstechnisch zweckmäßig übersteigt der Arbeitsdruck (das ist der Absolutdruck in der Gasatmosphäre des (normalerweise in einem Überdruckreaktor befindlichen) Reaktionsraums) bei der erfindungsgemäßen carbonylierenden Umsetzung 2,5 10⁷ Pa (abs.) normalerweise nicht, da höhere Arbeitsdrücke normalerweise übermäßige Anlagekosten bedingen. Arbeitsdrücke von 2 · 10⁵ Pa bis 2 · 10⁷ Pa sind erfindungsgemäß vorteilhaft. Erfindungsgemäß bevorzugt wird für die erfindungsgemäße carbonylierende Umsetzung ein Arbeitsdruck im Bereich von 5 · 10⁵ Pa bis 1,5 · 10⁷ Pa, besonders bevorzugt im Bereich von 1 · 10⁶ Pa bis 1 · 10⁷ Pa und ganz besonders bevorzugt im Bereich von 2 · 10⁶ Pa bis 9 · 10⁶ Pa bzw. im Bereich von 4 · 10⁶ Pa bis 8 · 10⁶ Pa angewendet. In dem Vorgenannten entsprechender Weise wird die erfindungsgemäße carbonylierende Umsetzung üblicherweise in einem Überdruckgefäß (in einem Autoklaven, in einem Überdruckreaktor) als Reaktionszone A durchgeführt.

Oxidierende Gase wie O₂, CO₂ und Wasserdampf wirken bezüglich der erfindungsgemäßen carbonylierenden Umsetzung normalerweise als Katalysatorgifte und werden daher weitgehend bzw. vorzugsweise vollständig aus dem zu verwendenden Kohlenmonoxid (bzw. generell aus den zu verwendenden Reaktionsgemischbestandteilen) ausgeschlossen. Ihre individuellen Volumenanteile am Gesamtvolumen des verwendeten Kohlenmonoxids sollten ≤ 1 Vol.-%, besser ≤ 0,1 Vol.-%, vorzugsweise ≤ 0,01 Vol.-%, besonders bevorzugt ≤ 0,001 Vol.-% betragen und ganz besonders bevorzugt verschwindend sein. D.h., vorzugsweise wird die erfindungsgemäße Polyesterbildung unter inerten Bedingungen, d.h., in Abwesenheit von Feuchtigkeit und Luft durchgeführt. Da Ethylenoxid ein hochentzündliches Gas bildet, ist ein Beisein von molekularem Sauerstoff auch unter diesem Gesichtspunkt bei der erfindungsgemäßen Carbonylierung prohibitiv. Wasserdampf vermag zudem den Ethylenoxidring in unerwünschter Weise zu öffnen, weshalb ein Beisein von Wasserdampf (abgesehen von den bereits erwähnten geringen Mengen) im Überdruckgefäß auch unter diesem Blickwinkel unerwünscht ist.

Das für die erfindungsgemäße carbonylierende Umsetzung zu verwendende Kohlenmonoxid kann dem Überdruckreaktor somit sowohl im Gemisch mit Inertgasen (z.B. N₂, Edelgase wie Ar) als auch im Wesentlichen als Reinsubstanz zugeführt werden. Letzteres ist erfindungsgemäß bevorzugt, weshalb die vorstehend für die erfindungsgemäße carbonylieren-de Umsetzung ausgeführten Arbeitsdrücke auch günstige CO-Partialdrücke (in der Gasatmosphäre des Reaktionsraums vorliegende) für die erfindungsgemäße Carbonylierung bilden.

Wie bereits erwähnt, wird die erfindungsgemäße carbonylierende Umsetzung normalerweise in einem gasdicht verschließbaren Druckbehälter für Reaktionen im Überdruckbereich durchgeführt (in einem Autoklaven = die Reaktionszone A). Grundsätzlich kann die erfindungsgemäße Polyesterbildung in einem Überdruckreaktor sowohl absatzweise als kontinuierlich durchgeführt werden. Wird sie absatzweise durchgeführt, kann der Arbeitsdruck (und mit diesem der CO-Partialdruck) konstant gehalten werden, oder dem Umsatz der Carbonylierung folgend abfallen. Ersteres ist in einfacher Weise dadurch möglich, dass verbrauchtes CO stetig in den Reaktionsraum des Druckreaktors nachgepresst wird.

Für eine erfindungsgemäße Carbonylierung eignet sich beispielsweise Kohlenmonoxid 2.3 der GCH Gerling Holz & Handels GmbH, das folgende Spezifikationen (die Angaben beziehen sich auf die Gasphase) aufweist:
≥ 99,3 Vol.-% CO,
≤ 4000 Vol.ppm N₂,
≤ 3500 Vol.ppm O₂,
≤ 3500 Vol.ppm Ar,
≤ 1000 Vol.ppm H₂,
≤ 500 Vol.ppm CO₂,
≤ 500 Vol.ppm Kohlenwasserstoffe (insgesamt), und
≤ 20 Vol.ppm H₂O.

Alternativ kann auch Kohlenmonoxid 3.0 der GCH Gerling Holz & Handels GmbH, das die Spezifikationen (die Angaben beziehen sich auf die Gasphase)
≥ 99,9 Vol.-% CO,
≤ 700 Vol.ppm N₂,
≤ 50 Vol.ppm O₂,
≤ 50 Vol.ppm Ar,
≤ 200 Vol.ppm H₂,
≤ 50 Vol.ppm CO₂,
≤ 25 Vol.ppm Kohlenwasserstoffe (insgesamt), und
≤ 20 Vol.ppm H₂O
aufweist, eingesetzt werden.

Ferner kann Ethylenoxid 3.0 (Stickstoff reduziert) der GCH Gerling Holz & Handels GmbH, das die Spezifikationen
≥ 99,9 Gew.-% Ethylenoxid,
≤ 60 Gew.ppm H2O,
≤ 20 Gew.ppm Säure (als Essigsäure),
≤ 50 Gew.ppm Aldehyde (als Acetaldehyd), und
≤ 0,1 Gew.% Inertgas in der Flüssigphase gelöst
aufweist, als Rohstoff für das erfindungsgemäße Verfahren eingesetzt werden (die Angaben beziehen sich auf die Flüssigphase). Dabei kann ein Restaldehydgehalt des Ethylenoxids durch in an sich bekannter Weise vorzunehmende Behandlung desselben mit Aldehydfängern (wie z.B. Aminoguanidinhydrogencarbonat), vorab seiner erfindungsgemäßen Verwendung zum völligen Verschwinden gebracht werden.

Auch kann Ethylenoxid 3.0 mit ca. 4-6 bar Stickstoff sowie Ethylenoxid 3.0 mit ca. 10 bar Stickstoff (derselben Lieferfirma) als Rohstoff für das erfindungsgemäße Verfahren verwendet werden (dabei handelt es sich um Ethylenoxid 3.0, das technisch bedingt mit Stickstoffanteilen überlagert ist).

Normalerweise wird man im Fall einer absatzweisen erfindungsgemäßen Carbonylierung des Ethylenoxids so verfahren, dass man den Reaktionsraum des Autoklaven anwendungstechnisch zweckmäßig zunächst mit Inertgas (z.B. Ar) spült. Anschließend wird man unter der Inertgasatmosphäre und bei vergleichsweise niedriger Temperatur das Katalysatorsystem, das unprotische Lösungsmittel und das Ethylenoxid in den Reaktionsraum des Autoklaven geben und selbigen verschließen.

Vorzugsweise wird der Reaktionsraum gerührt betrieben. Danach wird durch ein entsprechendes Druckventil eine für die Zwecke der Carbonylierung geeignete Menge an Kohlenmonoxid in den Reaktionsraum des Autoklaven gepresst.

Dann wird die Temperatur im Reaktionsraum durch äußeres Beheizen auf die Reaktionstemperatur erhöht, und das Reaktionsgemisch im Autoklaven z.B. unter Beibehalt der Reaktionstemperatur gerührt. Wird im Verlauf der Umsetzung kein Kohlenmonoxid in den Reaktionsraum nachgepresst, wird die carbonylierende Umsetzung in der Regel dann abgebrochen, wenn der Innendruck im Reaktionsraum auf einen sich mit der Zeit nicht mehr verändernden Wert abgefallen ist. Durch entsprechendes Abkühlen wird die Temperatur im Inneren des Reaktionsraumes abgesenkt, der erhöhte Innendruck nachfolgend auf Atmosphärendruck entspannt und der Autoklav geöffnet, so dass der Zugang zum im Reaktionsraum desselben befindlichen Produktgemisch A gegeben ist.

Wie bereits erwähnt, wird das Kohlenmonoxid insbesondere bei einer absatzweisen Ausführung des erfindungsgemäßen Verfahrens normalerweise in überstöchiometrischen Mengen eingesetzt. Grundsätzlich können aber auch die der Stöchiometrie entsprechende Menge oder auch eine unterstöchiometrische Menge an CO beim erfindungsgemäßen Verfahren eingesetzt werden.

Bezogen auf die molare Gesamtmenge an in den Autoklaven geführtes Ethylenoxid sind so bei vergleichsweise geringen Reaktionsdauern (in der Regel ≥0,1 bis ≤ 10 h, häufig ≥ 0,25 bis ≤ 5 h) üblicherweise Umsätze von ≥ 90 mol-%, vorteilhaft ≥ 95 mol-% oder ≥ 98 mol-%, vorzugsweise ≥ 99 mol-%, und besonders bevorzugt ≥ 99,9 mol-% zu erreichen.
Normalerweise enthält das bei der erfindungsgemäßen Carbonylierung von Ethylenoxid resultierende Produktgemisch A das gewünschte Poly-3-hydroxypropionat in gelöstem Zustand (Teilmengen des gebildeten Poly-3-hydroxypropionats können gegebenenfalls auch bereits ausgefallen sein).

Durch sich selbst überlassen und/oder Abkühlen des Produktgemischs A können wenigstens Teilmengen an Poly-3-hydroxypropionat in selbigem zur Ausfällung gebracht und durch Anwendung einer oder mehrerer mechanischer Trennoperationen (z.B. Filtrieren und/oder Zentrifugieren in einem Filter bzw. in einer Zentrifuge als Element der Trennzone A) vom Produktgemisch A abgetrennt werden (die dabei jeweils verbleibende Flüssigkeitsphase (z.B. das Filtrat bzw. das Serum) bildet z.B. eine Teilmenge des Produktgemischs A im Sinne der vorliegenden Anmeldung).

Alternativ oder ergänzend kann zur Abtrennung von Poly-3-hydroxypropionat vom Produktgemisch A oder von einer Teilmenge desselben zu diesen in der Trennzone A eine Fällflüssigkeit hinzugegeben werden, die, gegebenenfalls begleitet von einer zusätzlichen Absenkung der Temperatur, eine weitergehende Ausfällung an Poly-3-hydroxypropionat als die alleinige Maßnahme der Temperaturabsenkung erzwingt. Anschließend kann das so ausgefällte Poly-3-hy-droxypropionat wieder durch die Anwendung einer oder mehrerer mechanischer Trennoperationen in einem Element der Trennzone A vom jeweiligen Gemisch abgetrennt werden. Mit der dabei verbleibenden Flüssigphase (die eine weitere Teilmenge des Produktgemischs A umfasst) kann (z.B. zum Zweck der Ausbeutesteigerung an abgetrenntem Poly-3-hydroxypro-pionat) in entsprechender Weise weiterverfahren werden (die Erstzugabemenge an Fällflüssigkeit kann grundsätzlich aber auch bereits so ausreichend gewählt sein, dass die angestrebte Zielmenge an Poly-3-hydroxypropionat bereits im ersten Fällungsschritt ausfällt).

Wird zu Selbigen weitere Fällflüssigkeit hinzugefügt, entstehen dabei Gemische, die ihrerseits eine Teilmenge des Produktgemischs A mit zu dieser hinzugefügter Fällflüssigkeit bilden.

Als solche Fällflüssigkeiten kommen im Normalfall insbesondere Flüssigkeiten in Betracht, deren Polarität geringer oder höher (vorzugsweise "wesentlich geringer" bzw. "wesentlich höher") als die in der Regel mittlere Polarität des für die erfindungsgemäße carbonylierende Umsetzung verwendeten unprotischen Lösungsmittels ist.

Handelt es sich bei Letzterem z.B. um Diglyme (= Bis(2-methoxyethyl)ether), können als solche Fällflüssigkeiten z.B. Methanol, Cyclohexan, n-Heptan, und/oder der tert.-Butylmethylether verwendet werden.

Nachteilig an unter Verwendung vorgenannter Fällflüssigkeiten (vom Produktgemisch A) wie beschrieben abgetrenntem Poly-3-hydroxypropionat ist jedoch, dass dieses noch spürbare, auf das zur Carbonylierung verwendete Katalysatorsystem zurückzuführende, Mengen an Kobalt enthält.

Selbige sind insofern nachteilig, als sie eine nachfolgende Thermolyse des Poly-3-hydroxypropionats beeinträchtigen. Wird Methanol als Fällflüssigkeit mitverwendet, erfolgt darüber hinaus Esterbildung mit endständigen Carboxylgruppen des Poly-3-hydroxypropionats. Dies bedingt im Rahmen einer späteren Thermolyse desselben die Bildung von Methylacrylat als ein unerwünschtes Nebenprodukt.

Erfindungsgemäße Abhilfe ist nun in einfacher Weise z.B. dadurch möglich, dass man als Fällflüssigkeit Wasser und/oder eine wässrige Lösung wenigstens mitverwendet.

D.h., erfindungsgemäß vorteilhaft kann man zur Gesamtmenge des Produktgemischs A oder zu einer oder mehrerer Teilmengen des Produktgemischs A Wasser und/oder eine wässrige Lösung geben, um in der Teilmenge an Produktgemisch A oder in der Gesamtmenge an Produktgemisch A gelöst enthaltenes Poly-3-hydroxypropionat auszufällen. Selbstverständlich kann zusätzlich die Temperatur des resultierenden wässrigen Gemischs abgesenkt werden. Auch kann das Wasser bzw. die wässrige Lösung bei der jeweiligen Zugabe bereits eine vergleichsweise geringe Temperatur aufweisen. Diese kann z.B. ≥ 0 °C und ≤ 25 °C betragen.

Grundsätzlich ist es erfindungsgemäß vorteilhaft, einen Zusatz von Wasser und/oder einer wässrigen Lösung als wässrige Fällflüssigkeit zu einer oder mehreren Teilmengen des Produktgemischs A und/oder zur Gesamtmenge des Produktgemischs A, um in einer Teilmenge des Produktgemischs A oder in der Gesamtmenge des Produktgemischs A enthaltenes Poly-3-hy-droxypropionat auszufällen, im Beisein wenigstens eines Oxidationsmittels (für Co in Oxidationsstufen < +2) vorzunehmen.

Beispielsweise ist es erfindungsgemäß bevorzugt, den vorgenannten Zusatz in Gegenwart von Luft und/oder in Gegenwart eines von Luft verschiedenen molekularen Sauerstoff enthaltenden Gases vorzunehmen. Alternativ oder ergänzend kann einer Teilmenge des Produktgemischs A, der wässrigen Fällflüssigkeit selbst, der Gesamtmenge des Produktgemischs A und/oder dem entstehenden Gemisch aus einer Teilmenge bzw. der Gesamtmenge des Produktgemischs A und der wässrigen Fällflüssigkeit vor, während und/oder nach dem Zusatz der wässrigen Fällflüssigkeit ein oder mehr als ein Oxidationsmittel wie z.B. Ozon, Wasserstoffperoxid, molekularer Sauerstoff, ein Perchlorat und/oder eine oxidierend wirkende Säure wie z.B. Salpetersäure und/oder Perchlorsäure zugesetzt werden.

Bevor man das ausfallende Poly-3-hydroxypropionat durch wenigstens eine mechanische Trennoperation (z.B. durch Filtrieren und/oder Zentrifugieren) von der jeweiligen wässrigen Mischung abtrennt, wird man das jeweilige wässrige Gemisch anwendungstechnisch zweckmäßig intensiv durchmischen. Wie bereits gesagt, ist es erfindungsgemäß vorteilhaft, das wässrige Gemisch vorab der mechanischen Abtrennung des Poly-3-hydroxypropionats mit molekularem Sauerstoff oder mit einem molekularen Sauerstoff enthaltenden Gas (z.B. Luft oder Mischungen aus molekularem Sauerstoff und molekularem Stickstoff, CO, CO₂, Edelgas und/oder Wasserdampf) zu behandeln. In einfacher Weise kann dies dadurch erfolgen, dass das intensiv durchmischte wässrige Gemisch von molekularem Sauerstoff oder von dem molekularen Sauerstoff enthaltenden Gas durchströmt wird. Auch kann das Wasser oder eine wässrige Lösung vorab ihrer Verwendung als erfindungsgemäß zu verwendende Fällflüssigkeit mit molekularem Sauerstoff gesättigt werden. In entsprechender Weise kann auch die Gesamtmenge des Produktgemischs A oder die entsprechende Teilmenge des Produktgemischs A vorab des Zusatzes einer erfindungsgemäßen wässrigen Fällflüssigkeit zu dieser Gesamtmenge bzw. Teilmenge mit molekularem Sauerstoff angereichert bzw. gesättigt werden.

Selbstverständlich kann die Behandlung des wässrigen Gemischs mit molekularem Sauerstoff oder mit einem molekularen Sauerstoff enthaltenden Gas (z.B. Luft oder Mischungen aus molekularem Sauerstoff und molekularem Stickstoff) auch bei erhöhter Temperatur durchgeführt werden. Diese kann z.B. 10 bis 95 °C, oder 20 bis 95 °C, oder 30 bis 95 °C, vorteilhaft 40 bis 90 °C und besonders vorteilhaft 50 bis 80 °C bzw. 50 bis 60 °C betragen. In der Regel sind Behandlungsdauern von wenigen Minuten ausreichend. Anschließend kann das wässrige Gemisch auf Temperaturen ≤ 25 °C, vorzugsweise ≤ 20 °C und besonders bevorzugt ≤ 15 °C oder ≤ 10 °C abgekühlt werden, um die Fällung des Poly-3-hydroxypropionats zu fördern. Abschlie-ßend kann das ausgefallene Poly-3-hy-droxypropionat von dem wässrigen Gemisch durch wenigstens eine mechanische Trennoperation abgetrennt werden. Selbstverständlich kann die Abtrennung von ausgefallenem Poly-3-hydroxypropionat auch noch am warmen wässrigen Gemisch vorgenommen werden.

Auf eine solche Weise vom Produktgemisch A abgetrenntes Poly-3-hydroxypropionat weist einen signifikant verminderten bis verschwindenden Co-Gehalt auf (dies gilt selbstredend in entsprechender Weise für den jeweiligen Gesamtgehalt an den verschiedenen möglichen individuellen Oxidationsstufen des Co, d.h., für den Gesamtgehalt an Co⁺², bzw. an Co⁺¹, bzw. an Co⁰, bzw. an Co⁻¹). Es kann z.B. mit Methanol nachgewaschen, nachfolgend unter Einwirkung von Wärme getrocknet und schließlich der angestrebten Thermolyse zu Acrylsäure unterzogen werden.

Das Vorgenannte trifft insbesondere dann zu, wenn die als Fällflüssigkeit verwendete wässrige Lösung eine solche ist, deren pH-Wert bei einer Temperatur von 25 °C und bei Normaldruck ≤ 7,5, vorteilhaft ≤ 7 beträgt. Vorzugsweise beträgt der vorgenannte pH-Wert der wässrigen Fällflüssigkeit ≤ 6, und besonders bevorzugt ≤ 5, und ganz besonders bevorzugt ≤ 4. In der Regel wird der vorgenannte pH-Wert der wässrigen Fällflüssigkeit den Wert 0 nicht unterschreiten und vielfach ≥ 1 oder ≥ 2 betragen. Erfindungsgemäß vorteilhaft treffen vorstehende pH-Werte (ebenfalls bezogen auf 25 °C und Normaldruck) auch auf die bei Zugabe der wässrigen Fällflüssigkeit zum Produktgemisch A oder zu einer Teilmenge des Produktgemischs A resultierenden wässrigen Gemische zu, die wahlweise erfindungsgemäß vorteilhaft mit einem molekularen Sauerstoff enthaltenden Gas behandelt werden und von denen das ausgefallene Poly-3-hydroxypropionat durch die Anwendung wenigstens einer mechanischen Trennoperation abgetrennt wird. Vorzugsweise beträgt der pH-Wert (25 °C, Normaldruck) dieser wässrigen Gemische 2 bis 4, z.B. 3.

Die angegebenen pH-Werte beziehen sich in dieser Schrift, soweit nicht ausdrücklich etwas anderes gesagt wird, auf eine Bestimmung mit einer Checker® pH-Elektrode HI 98103 der Firma HANNA Instruments Deutschland GmbH, D-77694 Kehl. Selbige wird dabei anwendungstechnisch zweckmäßig vor einer jeweiligen Messung mit Hilfe von zwei wässrigen Pufferlösungen kalibriert, deren pH-Werte unter den entsprechenden Bedingungen z.B. 7,01 und 4,01 betragen (Technical Buffer, Modell TEP 7 (Bestellnummer 108702) und TEP 4 (Bestellnummer 108700) der Firma WTW (Wissenschaftlich Technische Werkstätten GmbH) in D-82362 Weinheim). Für oberhalb von 7 gelegene pH-Werte kann die Kalibrierung auch mit Hilfe von anderen wässrigen Pufferlösungen durchgeführt werden.

Als Stellmittel zur Einstellung des relevanten pH-Werts kommen anorganische und/oder organische Säuren in Betracht (im Sinne von Brönsted). Beispielhaft genannt seien Schwefelsäure, Kohlensäure, Salzsäure und/oder Phosphorsäure als mögliche anorganische Säuren. Erfindungsgemäß bevorzugt werden organische Carbonsäuren, insbesondere Alkancarbonsäuren, als pH-Stellmittel verwendet. Unter diesen seien beispielhaft aufgeführt Acrylsäure, Oxalsäure, Ameisensäure, Essigsäure, Propionsäure, Fumarsäure und/oder Maleinsäure. Selbstverständlich können zur Einstellung des relevanten pH-Wertes auch organische Sulfonsäuren wie z.B. Methansulfonsäure verwendet bzw. mitverwendet werden.

Als erfindungsgemäß geeignete wässrige Fällflüssigkeiten kommen somit z.B. wässrige Lösungen in Betracht, die eine oder mehr als eine der vorgenannten anorganischen und/oder organischen Säuren gelöst aufweisen. Solche wässrigen Fällflüssigkeiten sind z.B. wässrige Schwefelsäure, wässrige Kohlensäure, wässrige Salzsäure, wässrige Phosphorsäure, wässrige Acrylsäure, wässrige Oxalsäure, wässrige Ameisensäure, wässrige Essigsäure, wässrige Propionsäure, wässrige Fumarsäure, wässrige Maleinsäure und/oder wässrige Methansulfonsäure. Selbstverständlich kann die Hinzufügung von Wasser und einer oder mehrerer Säuren zum Zweck einer Fällung von Poly-3-hydroxypropionat auch zeitlich und/oder räumlich voneinander getrennt erfolgen, so dass sich die effektiv hinzugefügte saure wässrige Fällflüssigkeit z.B. erst im das Poly-3-hydroxypropionat aufweisenden wässrigen Gemisch ausbildet.

Anwendungstechnisch zweckmäßig enthält eine der vorgenannten erfindungsgemäß verwendbaren wässrigen Fällflüssigkeiten, auf das Gewicht der wässrigen Flüssigkeit bezogen, wenigstens 10 Gew.-%, besser wenigstens 20 Gew.-% oder wenigstens 30 Gew.-%, vorteilhaft wenigstens 40 Gew.-% oder wenigstens 50 Gew.-%, besonders vorteilhaft wenigstens 60 Gew.-% oder wenigstens 70 Gew.-%, wahlweise wenigstens 80 Gew.-% oder wenigstens 90 Gew.-%, vielfach wenigstens 95 Gew.-%, oder wenigstens 97 Gew.-%, oder wenigstens 99 Gew.-% Wasser.

Grundsätzlich kann der Zusatz einer Fällflüssigkeit zur Gesamtmenge eines Produktgemischs A und/oder zu einer Teilmenge eines Produktgemischs A portionsweise und bei erhöhter Temperatur so erfolgen, dass erst durch jeweiliges nachfolgendes langsames Abkühlen die (weitergehende) Kristallisation bzw. Fällung bewirkt wird. Auf diese Weise kann die Gesamtzahl der "Fällkeime (Kristallisationskeime)" gering gehalten werden, woraus letztlich in der Regel eine grobkörnigere Qualität des gefällten Poly-3-hydroxypropionats resultiert, dessen Abtrennung nachfolgend vergleichsweise einfacher möglich ist.

Selbstverständlich kann die Ausfällung von Poly-3-hydroxypropionat aus dem Produktgemisch A oder aus einer Teilmenge des Produktgemischs A im Rahmen der erfindungsgemäßen Verfahrensweise aber auch durch eine Absenkung von deren Temperatur und/oder durch den Zusatz einer nicht wässrigen Fällflüssigkeit zu denselben bewirkt werden (z.B. durch einen Zusatz von Methanol, Cyclohexan, n-Heptan, und/oder tert.-Butylmethylether als Fällflüssigkeit).

In diesem Fall wird das ausgefällte Poly-3-hydroxypropionat nachfolgend durch wenigstens eine mechanische Trennoperation (z.B. Filtrieren und/oder Zentrifugieren) abgetrennt, und der Kobaltgehalt des so abgetrennten Poly-3-hydroxypropionats erfindungsgemäß durch ein späteres Waschen desselben mit Wasser und/oder einer wässrigen Lösung abgesenkt bzw. zum Verschwinden gebracht.

Als wässrige Lösungen kommen dabei zum Zweck eines solchen Waschens insbesondere alle diejenigen in Betracht, die auch bereits als mögliche wässrige Fällflüssigkeiten aufgeführt und empfohlen wurden. Die bei einer Verwendung als wässrige Fällflüssigkeit gemachten Bevorzugungen gelten hinsichtlich einer Verwendung als wässrige Waschlösungen (als wässrige Waschflüssigkeiten) in entsprechender Weise.

D.h., erfindungsgemäß als Waschflüssigkeit bevorzugte wässrige Lösungen sind solche, deren pH-Wert bei einer Temperatur von 25 °C und bei Normaldruck ≤ 7,5, mit Vorteil ≤ 7 beträgt. Vorzugsweise beträgt der vorgenannte pH-Wert der wässrigen Waschflüssigkeit (der wässrigen Waschlösung) ≤ 6, besonders bevorzugt ≤ 5, und ganz besonders bevorzugt ≤ 4. In der Regel wird der vorgenannte pH-Wert einer wässrigen Waschflüssigkeit (einer wässrigen Waschlösung) den Wert 0 nicht unterschreiten und vielfach ≥ 1 oder ≥ 2 betragen.

Als Stellmittel zur Einstellung des pH-Wertes einer wässrigen Waschlösung kommen die bereits im Zusammenhang mit den entsprechenden wässrigen Fällflüssigkeiten angeführten anorganischen und/oder organischen Säuren (im Sinne Brönsteds) in Betracht.

Beispielhaft genannt seien Schwefelsäure, Kohlensäure, Salzsäure und/oder Phosphorsäure als mögliche anorganische Säuren. Erfindungsgemäß bevorzugt werden organische Carbonsäuren, insbesondere Alkancarbonsäuren, als solche pH-Stellmittel verwendet. Unter diesen seien die Acrylsäure, Oxalsäure, Ameisensäure, Essigsäure, Propionsäure, Fumarsäure und/oder Maleinsäure beispielhaft aufgeführt. Selbstverständlich kann zur Einstellung des relevanten pH-Wertes auch eine organische Sulfonsäure wie z.B. Methansulfonsäure verwendet oder mitverwendet werden.

Als erfindungsgemäß geeignete wässrige Waschflüssigkeiten kommen somit z.B. wässrige Lösungen in Betracht, die eine oder mehr als eine der vorgenannten anorganischen und/oder organischen Säuren gelöst aufweisen. Solche wässrigen Waschflüssigkeiten sind z.B. wässrige Schwefelsäure, wässrige Kohlensäure, wässrige Salzsäure, wässrige Phosphorsäure, wässrige Acrylsäure, wässrige Oxalsäure, wässrige Ameisensäure, wässrige Essigsäure, wässrige Propionsäure, wässrige Fumarsäure, wässrige Maleinsäure und/oder wässrige Methansulfonsäure.

Anwendungstechnisch zweckmäßig enthält eine der vorgenannten erfindungsgemäß zu verwendenden wässrigen Waschflüssigkeiten, auf das Gewicht der wässrigen Waschflüssigkeit bezogen, wenigstens 10 Gew.-%, besser wenigstens 20 Gew.-% oder wenigstens 30 Gew.-%, vorteilhaft wenigstens 40 Gew.-% oder wenigstens 50 Gew.-%, besonders vorteilhaft wenigstens 60 Gew.-% oder wenigstens 70 Gew.-%, wahlweise wenigstens 80 Gew.-% oder wenigstens 90 Gew.-%, vielfach wenigstens 95 Gew.-%, oder wenigstens 97 Gew.-%, oder wenigstens 99 Gew.-% Wasser. Wasser selbst ist nicht nur eine erfindungsgemäß geeignete Fällflüssigkeit, sondern auch eine erfindungsgemäß geeignete Waschflüssigkeit.

Auch beim Waschen von vom Produktgemisch A in der Trennzone A abgetrenntem Poly-3-hy-droxypropionat mit Wasser und/oder mit einer wässrigen Lösung als wässrige Waschflüssigkeit ist es erfindungsgemäß vorteilhaft, das Waschen im Beisein wenigstens eines Oxidationsmittels (für Co in Oxidationsstufen < +2) vorzunehmen. Beispielsweise ist es vorteilhaft, das Waschen in Gegenwart von Luft und/oder in Gegenwart von einem molekularen Sauerstoff enthaltenden Gas vorzunehmen. Alternativ oder ergänzend kann der wässrigen Waschflüssigkeit und/oder dem zu waschenden Poly-3-Hydroxypropionat vorab und/oder während der Wäsche ein oder mehr als ein Oxidationsmittel wie z.B. Ozon, Wasserstoffperoxid, molekularer Sauerstoff, ein Perchlorat und/oder eine oxidierend wirkende Säure wie z.B. Salpetersäure und/oder Perchlorsäure zugesetzt werden.

Erfindungsgemäß vorteilhaft werden die vorstehend aufgeführten wässrigen Waschflüssigkeiten vorab ihrer Verwendung zur Wäsche von von einem Produktgemisch A abgetrenntem, Co enthaltendem Poly-3-hydroxypropionat daher z.B. mit molekularem Sauerstoff gesättigt.

In einer solchen Ausführungsform kann die Wäsche des von einem Produktgemisch A abgetrennten Co enthaltenden Poly-3-hydroxypropionats so erfolgen, dass man die wässrige Waschflüssigkeit durch das Poly-3-hydroxypropionat (das z.B. auf einem Filter als Filterkuchen vorliegt, oder auf einer Siebzentrifuge als Feststoffkuchen) hindurchdrückt oder hindurchsaugt. Die Temperatur der wässrigen Waschflüssigkeit kann dabei z.B. 10 bis 95 °C, oder 20 bis 90°C, oder 30 bis 80 °C, oder 40 bis 70 °C, oder 50 bis 60 °C betragen. Vorzugsweise wird die Temperatur der Waschflüssigkeit dabei so gewählt, dass der Schmelzpunkt des Poly-3-hydroxypropionats nicht überschritten ist.

Alternativ kann das von einem Produktgemisch A abgetrennte Co enthaltende Poly-3-hydroxy-propionat zum Zweck seiner Wäsche mit einer wässrigen Waschflüssigkeit auch in derselben resuspendiert werden. Dabei erweist es sich erfindungsgemäß als vorteilhaft, die resultierende wässrige Suspension von Poly-3-hydroxypropionat zusätzlich mit einem molekularen Sauerstoff enthaltenden Gas (z.B. Luft oder Mischungen aus molekularem Sauerstoff und molekularem Stickstoff, CO, CO₂, Edelgas und/oder Wasserdampf) zu behandeln.

In einfacher Weise kann dies dadurch erfolgen, dass die wässrige Suspension intensiv durchmischt und zusätzlich von molekularem Sauerstoff oder von einem molekularen Sauerstoff enthaltenden Gas (z.B. Luft) durchströmt wird. Auch hier kann das Wasser oder die wässrige Lösung vorab seiner Verwendung als erfindungsgemäß zu verwendende Waschflüssigkeit mit molekularem Sauerstoff gesättigt werden. Die Temperatur der wässrigen Suspension des Poly-3-hydroxypropionats kann bei der beschriebenen Verfahrensweise z.B. 10 bis 95 °C, oder 20 bis 90 °C, oder 30 bis 80 °C, oder 40 bis 70 °C, oder 50 bis 60 °C betragen. Gegebenenfalls kann sich die wässrige Suspension bei einem Waschen unter einer erhöhten Temperatur in eine Lösung oder Emulsion wandeln. Durch nachfolgendes Abkühlen derselben kann in diesen Fällen die Suspension rückgebildet werden. Als Abschluss der Wäsche kann das Poly-3-hy-droxypropionat (wahlweise nach zuvor erfolgter Abkühlung der wässrigen Mischung) wieder durch wenigstens eine mechanische Trennoperation (z.B. Filtrieren und/oder Zentrifugieren) aus der wässrigen Suspension abgetrennt werden.

Selbstverständlich kann die beschriebene erfindungsgemäße wässrige Wäsche von von einem Produktgemisch A abgetrenntem Poly-3-hydroxypropionat mehrfach wiederholt werden, um das in ihm enthaltene Co (z.B. quantitativ) abzutrennen. Diese Aussage gilt nicht nur bezogen auf die in ihm insgesamt enthaltene Gesamtmenge an Co. Vielmehr trifft sie jeweils auch auf die jeweilige in ihm enthaltene Gesamtmenge an Co in einer seiner möglichen individuellen Oxidationsstufen zu. D.h., sie gilt für die in ihm enthaltene Gesamtmenge an Co²⁺, für die in ihm enthaltene Gesamtmenge an Co⁺¹, für die in ihm enthaltene Gesamtmenge an Co⁰, und für die in ihm enthaltene Gesamtmenge an Co⁻¹.

Auch kann die beschriebene erfindungsgemäße wässrige Wäsche von von einem Produktgemisch A abgetrenntem Poly-3-hydroxypropionat mit Wasser und/oder mit einer der wässrigen Lösungen erfindungsgemäß auch dann angewendet werden, wenn die Abtrennung des Poly-3-hydroxypropionats vom Produktgemisch A bereits unter Zusatz von Wasser und/oder einer wässrigen Lösung als wässrige Fällflüssigkeit zu einer oder mehrerer Teilmengen des Produktgemischs A oder zu der Gesamtmenge des Produktgemischs A vorgenommen worden ist.

Die erfindungsgemäß zu verwendenden Mengen an wässriger Fällflüssigkeit und/oder wässriger Waschflüssigkeit sowie deren wahlweise einzustellenden Gehalte an anorganischen und/oder organischen Säuren werden anwendungstechnisch zweckmäßig so gewählt, dass unter den für die jeweilige Fällung angewandten Bedingungen vorzugsweise keine Ausfällung eines Kobaltsalzes erfolgt.

Erfindungsgemäß besonders bevorzugt wird als wässrige Fällflüssigkeit und/oder als wässrige Waschflüssigkeit eine wässrige Essigsäurelösung und/oder eine wässrige Ameisensäurelösung verwendet, wobei die Anwendung einer wässrigen Essigsäurelösung aufgrund der erhöhten Löslichkeit von Kobaltacetat in wässrigen (Essigsäure)Lösungen ganz besonders bevorzugt ist.

Grundsätzlich können beim erfindungsgemäßen Verfahren auch wässrige Lösungen als wäss-rige Fällflüssigkeiten und/oder wässrige Waschflüssigkeiten eingesetzt werden, deren pH-Wert (bezogen auf 25 °C und Normaldruck) > 7,5 beträgt (z.B. die wässrige Lösung wenigstens einer Brönsted-Base). Im Besonderen sei diesbezüglich eine Fällung und/oder Wäsche mit einer wässrigen Ammoniaklösung aufgeführt. Allerdings ist diese Vorgehensweise erfindungsgemäß weniger bevorzugt. Auch können erfindungsgemäß zu verwendende wässrige Fäll- und Waschflüssigkeiten Komplexbildner (insbesondere Chelatbildner) wie z.B. Ethylendiamintetraessigsäure und/oder eines deren Alkalisalze (z.B. eines ihrer Natriumsalze) zugesetzt aufweisen, die Kationen des Kobalts zu komplexieren vermögen.

Selbstverständlich kommen als erfindungsgemäß geeignete wässrige Fällflüssigkeiten und/oder wässrige Waschflüssigkeiten auch solche wässrigen Lösungen in Betracht, die anstelle oder zusätzlich zu anorganischen und/oder organischen (Brönsted) Säuren (insbesondere den in dieser Schrift individualisiert genannten) wenigstens ein in Wasser lösliches organisches Lösungsmittel wie z.B. Alkohole (z.B. Methanol, Ethanol etc.), Ketone (z.B. Aceton, Methylethylketon), Amide (z.B. N.N-Dimethylformamid und Formamid), Sulfoxide (z.B. Dimethylsulfoxid), N-Methyl-2 pyrrolidon oder cyclische Ether (z.B. Tetrahydrofuran und 1,4-Dioxan) gelöst enthalten. In der Regel wird der Wassergehalt solcher wässriger Lösungen, bezogen auf ihr Gesamtgewicht, in entsprechender Weise wenigstens 10 Gew.-%, oder wenigstens 20 Gew.-%, oder wenigstens 30 Gew.-%, oder wenigstens 40 Gew.-%, oder wenigstens 50 Gew.-%, oder wenigstens 60 Gew.-%, oder wenigstens 70 Gew.-%, oder wenigstens 80 Gew.-%, oder wenigstens 90 Gew.-%, oder wenigstens 95 Gew.-%, oder wenigstens 97 Gew.-%, oder wenigstens 99 Gew.-% betragen.

Generell ist erfindungsgemäß eine Nachwäsche des Poly-3-hydroxypropionats mit z.B. Wasser und/oder (besonders bevorzugt) Methanol empfehlenswert.

Durch eine solche Nachwäsche können letzte unerwünschte Bestandteile aus dem Poly-3-hy-droxypropionat beseitigt werden. Eine Nachwäsche mit Methanol erleichtert darüber hinaus eine anschließende Trocknung des vergleichsweise reinen Poly-3-hydroxypropionats. Selbige kann z.B. während mehrerer Stunden (z.B. über Nacht) bei Temperaturen von 50 bis 90 °C, vorzugsweise 50 bis 60 °C, z.B. im Trockenschrank, erfolgen. Auch kann sie bei vermindertem Druck und/oder vergleichsweise geringerer Temperatur durchgeführt werden. Vorzugsweise erfolgt eine solche Trocknung bei Temperaturen, bei denen der Schmelzpunkt des Poly-3-hydroxypropionats noch nicht überschritten wird.

Selbstverständlich kann beim erfindungsgemäßen Verfahren ein solches Trocknen auch unterbleiben. In diesem Fall verflüchtigt sich eine im P3HP enthaltene Restfeuchte im Rahmen der für seine Thermolyse erforderlichen (erfolgenden) Erwärmung (und zwar normalerweise bevor die Thermolyse einsetzt).

Die Wirksamkeit der erfindungsgemäßen Fällung und/oder Wäsche mit Wasser und/oder wässrigen Lösungen ist zum Teil wahrscheinlich darauf zurück zu führen, dass im Verlauf der erfindungsgemäßen carbonylierenden Umsetzung Polyesterketten der nachfolgenden Struktur ausgebildet werden (vgl. J.Am.Chem.Soc., 2002, 124, Seite 5646-5647): 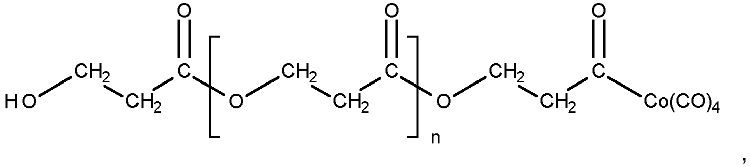 wobei n eine ganze Zahl ≥ 1 ist, und bis zu 150, oder bis zu 200, oder bis zu 250 und mehr betragen kann. Das linke Ende reflektiert den Kettenstart und das rechte Ende reflektiert das Kettenwachstum.

Wasser vermag das rechte Kettenende unter Ausbildung von 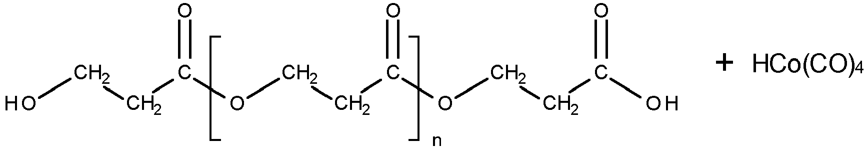 durch Hydrolyse zu spalten und die Polyesterkette auf diese Weise zu terminieren. Ein Beisein von molekularem Sauerstoff (bzw. allgemein eines Oxidationsmittels) fördert die Ausbildung von Co²⁺, dessen Löslichkeit in wässrigen Lösungen eine vergleichsweise erhöhte ist.

Darüber hinaus wirkt eine Kombination aus Wasser/Oxidationsmittel bzw. wässriger Lösung/Oxidationsmittel (z.B. O₂) vermutlich auch auf im abgetrennten Poly-3-hydroxypropionat lediglich absorbiert enthaltene Verbindungen des Kobalts in entsprechender Weise im erfindungsgemäßen Sinn ein.

Die erfindungsgemäße Abtrennung von Poly-3-hydroxypropionat von einem erfindungsgemäß hergestellten Produktgemisch A vermag somit auf vergleichsweise effiziente Weise Poly-3-hy-droxypropionat mit beliebig geringem Co-Gehalt (einschließlich Co-Gehalten jenseits der Co-Nachweisgrenze) für eine erfindungsgemäße Thermolyse des Poly-3-hydroxypropionats zur Verfügung zu stellen. Diese Aussage gilt nicht nur bezogen auf die im erfindungsgemäß zu spaltenden Poly-3-hydroxypropionat insgesamt enthaltene Gesamtmenge an Co. Vielmehr trifft sie jeweils auch auf die jeweilige in ihm (im durch erfindungsgemäße Thermolyse zu spaltenden Poly-3-hydroxypropionat) enthaltene Gesamtmenge an Co in einer seiner möglichen individuellen Oxidationsstufen zu. D.h., sie gilt für die in ihm enthaltene Gesamtmenge an Co²⁺, für die in ihm enthaltene Gesamtmenge an Co⁺¹, für die in ihm enthaltene Gesamtmenge an Co⁰, und für die in ihm enthaltene Gesamtmenge an Co⁻¹.

Beispielsweise kann der Co-Gehalt des Poly-3-hydroxypropionats (bezogen auf dessen Gesamtgewicht) bei seiner erfindungsgemäßen Thermolyse ≤ 2 Gew.-%, oder ≤ 1 Gew.-%, oder ≤ 0,5 Gew.-%, oder ≤ 0,1 Gew.-%, oder ≤ 0,05 Gew.-%, oder ≤ 0,01 Gew.-%, oder ≤ 0,001 Gew.-%, oder ≤ 10⁻⁴ Gew.-%, oder ≤ 10⁻⁵ Gew.-%, oder ≤ 10⁻⁶ Gew.-% betragen (oder jenseits der Nachweisgrenze liegen).

Vorstehende Aussage und individuell aufgeführten numerischen Gehalte gelten nicht nur bezogen auf die im erfindungsgemäß zu spaltenden Poly-3-hydroxypropionat möglicherweise insgesamt enthaltenen Gesamtmengen an Co. Vielmehr treffen sie jeweils auch auf die jeweilige in ihm (im durch erfindungsgemäße Thermolyse zu spaltenden Poly-3-hydroxypropionat) enthaltene Gesamtmenge an Co in einer seiner möglichen individuellen Oxidationsstufen zu. D.h., sie gelten auch individuell für die in ihm (im durch erfindungsgemäße Thermolyse zu spaltenden Poly-3-hydroxypropionat) möglicherweise enthaltene Gesamtmenge an Co²⁺, für die in ihm möglicherweise enthaltene Gesamtmenge an Co⁺¹, für die in ihm möglicherweise enthaltene Gesamtmenge an Co⁰, und für die in ihm möglicherweise enthaltene Gesamtmenge an Co⁻¹.

Zwar ist die Störwirkung des Co bei der Thermolyse von Poly-3-hydroxypropionat umso geringer, je geringer der Co-(Gesamt)Gehalt des Poly-3-hydroxypropionats ist (diese gilt insbesondere für enthaltenes Co in einer Oxidationsstufe < +2). In der industriellen Praxis wird man den anzuwendenden Gesamtaufwand im Rahmen einer erfindungsgemäßen Decobaltierung des Poly-3-hydroxypropionats nach wirtschaftlichen Gesichtspunkten normalerweise jedoch dahingehend beschränken, dass der für die Thermolyse verbliebene Co-(Gesamt)Gehalt des Poly-3-hydroxypropionats gesamtwirtschaftlich noch als tolerabel erscheint.

In diesem Sinne kann der Co-(Gesamt)Gehalt von erfindungsgemäß erzeugtem Poly-3-hydroxypropionat bei seiner erfindungsgemäßen Thermolyse, und bezogen auf sein Gesamtgewicht, z.B. 0 Gew.-% bis 1 Gew.-%, oder 10⁻⁶Gew.-% bis 1 Gew.-%, oder 10⁻⁵ Gew.-% bis 1 Gew.-%, oder 10⁻⁴ Gew.-% bis 1 Gew.-%, oder 0,001 Gew.-% bis 0,75 Gew.-%, oder 0,01 Gew.-% bis 0,75 Gew.-%, oder 0,05 bis 0,75 Gew.-%, oder 0,1 Gew.-% bis 0,5 Gew.-% betragen.

Vorstehende Aussage und individuell aufgeführten numerischen Gehaltsbereiche gelten nicht nur bezogen auf die im erfindungsgemäß zu spaltenden Poly-3-hydroxypropionat möglicherweise insgesamt enthaltenen Gesamtmengen an Co. Vielmehr treffen sie jeweils auch auf die jeweilige in ihm (im durch erfindungsgemäße Thermolyse zu spaltenden Poly-3-hydroxypropionat) enthaltene Gesamtmenge an Co in einer seiner möglichen individuellen Oxidationsstufen zu. D.h., sie gelten auch individuell für die in ihm (im durch erfindungsgemäße Thermolyse zu spaltenden Poly-3-hydroxypropionat) möglicherweise enthaltene Gesamtmenge an Co²⁺, für die in ihm möglicherweise enthaltene Gesamtmenge an Co⁺¹, für die in ihm möglicherweise enthaltene Gesamtmenge an Co⁰, und für die in ihm möglicherweise enthaltene Gesamtmenge an Co⁻¹.

Aus dem Stand der Technik ist bekannt, dass Poly-3-hydroxypropionat durch alleinige Einwirkung einer erhöhten Temperatur zu Acrylsäure gespalten werden kann (vgl. US 2,568,636 A, US 2,361,036 A und EP 577206 A2), wobei diese thermolytische Spaltung normalerweise in weitgehender Abwesenheit von molekularem Sauerstoff ausgeführt wird.

Aufgrund dieser Eigenschaft bildet Poly-3-hydroxypropionat eine besonders vorteilhafte Depotform (Lagerform)/Transportform der Acrylsäure, die im Unterschied zur Acrylsäure selbst im Wesentlichen keinem Alterungsprozess unterworfen ist (bei Normalbedingungen (= 25 °C und Normaldruck) flüssige Acrylsäure altert beim sich selbst überlassen z.B. in unvermeidbarer Weise infolge unerwünschter Michael-Addition der Acrylsäure an sich selbst und an die dabei entstehenden Additionsprodukte; unerwünschte radikalische Polymerisation der Acrylsäure mit sich selbst bildet einen weiteren Alterungsstrang, dem üblicherweise (z.B. aus Sicherheitsgründen) durch Zusatz von Polymerisationsinhibitoren in die Acrylsäure entgegengewirkt werden muss; bei einer radikalisch initiierten Polymerisation unter Mitverwendung einer solchen Polymerisationsinhibitor enthaltenden Acrylsäure, kann der in dieser enthaltene Polymerisationsinhibitor die radikalisch initiierte Polymerisation beeinträchtigen; außerdem sind Polymerisationsinhibitoren vergleichsweise teure Wirkverbindungen, die zudem in der Regel von Zeit zu Zeit erneuert werden müssen, da sich ihre Wirkung erschöpfen kann).

Insbesondere kann Poly-3-hydroxypropionat, das bei Normalbedingungen in der Regel im festen Aggregatszustand vorliegt, sowohl problemlos gelagert als auch transportiert werden.

Es ist auch bekannt, dass die für angemessene Spaltraten erforderlichen Temperaturen durch den Zusatz geeigneter Spaltkatalysatoren zum zu spaltenden Poly-3-hydroxypropionat (bzw. zu einem dieses enthaltenden Spaltgemisch) erheblich verringert werden können (vgl. z.B. WO 2011/100608 und US 2,961,036 A).

In typischer Weise anzuwendende Spalttemperaturen können sich daher im Bereich von 50 bis 350 °C oder im Bereich von 100 bis 300 °C bewegen. Anwendungstechnisch bevorzugt betragen typische Spalttemperaturen 150 bis 220 °C und besonders bevorzugt 160 bis 200 °C.

Je nach Schmelzpunkt und Löslichkeit des Poly-3-hydroxypropionats kann seine thermische Spaltung zu Acrylsäure aus seiner festen Substanz, oder aus seiner Schmelze, oder aus seiner Lösung in einem Lösungsmittel, oder aus seiner Suspension in einer organischen Flüssigkeit (einem Dispergiermittel), oder aus seiner Emulsion in einer organischen Flüssigkeit (einem Dispergiermittel) heraus erfolgen. Der Siedepunkt (bezogen auf Normaldruck) eines solchen Lösungsmittels/Dispergiermittels liegt dabei anwendungstechnisch vorteilhaft deutlich (z.B. wenigstens 20 °C, besser wenigstens 40 °C, noch besser wenigstens 50 °C oder wenigstens 60 °C, vorzugsweise wenigstens 80 °C und besonders bevorzugt wenigstens 100 °C) oberhalb der entsprechenden Siedetemperatur von Acrylsäure (= 141 °C). Beispielsweise kommen als solche Lösungsmittel/Dispergiermittel auch ionische Flüssigkeiten in Betracht.

Der Arbeitsdruck kann während der thermischen Spaltung des Poly-3-hydroxypropionats sowohl bei Normaldruck (= 1,0133 · 10⁵ Pa) als auch oberhalb oder unterhalb von Normaldruck liegen.

Liegt der Arbeitsdruck unterhalb vom Normaldruck (z.B. bei Drücken bis zu 10² Pa und weniger) folgt die bei der Spaltung gebildete Acrylsäure dem vorliegenden Druckgefälle und wird auf diese Weise dem flüssigen Spaltgemisch kontinuierlich entzogen.

Liegt der Arbeitsdruck bei oder oberhalb vom Normaldruck (z.B. bei Drücken bis zu 10⁷ Pa und mehr) kann die bei der Spaltung gebildete Acrylsäure anwendungstechnisch zweckmäßig mit Hilfe eines Strippgases (z.B. molekularer Stickstoff, Edelgas, Kohlendioxid, Luft, Magerluft (bevorzugt; an molekularem Sauerstoff abgereicherte Luft (in der Regel < 6 Vol.-% O₂))) kontinuierlich aus dem z.B. flüssigen Spaltgemisch (das z.B. auch die alleinige Schmelze des P3HP sein kann) herausgestrippt werden.

Die Maßnahme des Strippens kann auch im Rahmen einer Spaltung bei vermindertem Druck mitangewendet werden.

Selbstverständlich kann die bei der Spaltung gebildete Acrylsäure auch in konventioneller Weise dem entsprechenden Temperaturgefälle folgend aus dem z.B. flüssigen Spaltgemisch abdestilliert werden.

Führt man den vom z.B. flüssigen Spaltgemisch wegströmenden, die bei der Spaltung entstehende Acrylsäure enthaltenden Gasstrom im Gegenstrom zu absteigender Rücklaufflüssigkeit durch eine dem (bzw. einem)Spaltreaktor aufgesetzte Rektifikationskolonne, kann die Acrylsäure in erhöhter Reinheit vom flüssigen Spaltgemisch abgetrennt werden.

Alle derartigen Spaltungen von Poly-3-hydroxypropionat durch Einwirkung erhöhter Temperaturen werden in dieser Schrift unter dem Begriff "Thermolyse" bzw. "Pyrolyse" von Poly-3-hydro-xypropionat zusammengefasst.

Alle aus dem Stand der Technik für Poly(3-hydroxypropionsäure) bekannten Spaltverfahren können in entsprechender Weise auf erfindungsgemäß erhältliches Poly-3-hydroxypropionat angewendet werden. Typische Arbeitsdruckbereiche betragen dabei 10² bis 10⁷ Pa, oft 10³ bis 10⁶ Pa, vielfach 2 · 10³ bis 5 · 10⁵ Pa oder 5 · 10³ bis 3 · 10⁵ Pa.

Der Schmelzpunkt von erfindungsgemäß erhältlichem Poly-3-hydroxypropionat beträgt bei Normaldruck üblicherweise ≥ 50 °C und ≤ 150 °C, meist ≤ 100 °C.

Vor diesem Hintergrund ist es erfindungsgemäß zweckmäßig, die Thermolyse eines erfindungsgemäß erhältlichen Poly-3-hydroxypropionats zu Acrylsäure aus dessen Schmelze heraus durchzuführen.

Dabei wäre es wünschenswert, die Thermolyse im Beisein wenigstens eines Spaltkatalysators durchführen zu können, der sich unter den Bedingungen des Spaltverfahrens (Arbeitsdruck, Spalttemperatur) mit seiner jeweils erforderlichen katalytisch wirksamen zuzusetzenden Menge in der Schmelze des thermisch zu spaltenden Poly-3-hydroxypropionats vollständig löst ("erste Anforderung").

Eine Mitverwendung von Spaltkatalysatoren ermöglicht nicht nur eine Durchführung der Thermolyse bei geringeren Temperaturen, sondern gewährleistet unter vorgegebenen Thermolysebedingungen normalerweise insbesondere auch eine erhöhte Raum-Zeit-Ausbeute an Acrylsäure (der Spaltkatalysator verbessert bei vorgegebenen Bedingungen in der Regel sowohl die Spaltrate als auch die Selektivität der Zielproduktbildung (der Acrylsäurebildung)).

Im gegebenen Zusammenhang wäre es von weiterem Vorteil, wenn der wenigstens eine Spaltkatalysator eine Wirksubstanz (eine Wirkverbindung) ist, deren Siedepunkt bei Normaldruck zusätzlich wenigstens 180 °C, vorzugsweise wenigstens 185 °C und besonders bevorzugt wenigstens 190 °C beträgt ("zweite Anforderung").

Die zusätzliche Vorteilhaftigkeit liegt darin begründet, dass solche Spaltkatalysatoren im Rahmen der durch sie katalysierten Thermolyse des Poly-3-hydroxypropionats normalerweise nicht in notwendiger Weise mit der bei der Spaltung gebildeten Acrylsäure aus dem Spaltgemisch ausgetragen werden müssen, sondern in der Regel im Spaltgemisch verbleiben können (letzteres kann durch eine im Rücklauf betriebene, dem Spaltreaktor aufgesetzte Rektifikationskolonne unterstützt werden). Durch sukzessives Ergänzen von frischem zu spaltendem P3HP ins Spaltgemisch hinein, kann in diesem Fall von der Wirkung ein und desselben Spaltkatalysatorzusatzes mehrfach (wiederholt) Gebrauch gemacht werden.

Eine zusätzliche vorteilhafte ("zweite Anforderung*") obere Siedepunktsgrenze einer erfindungsgemäß als Spaltkatalysator für die Thermolyse geeigneten Wirkverbindung (d.h., ein Siedepunkt bei Normaldruck von ≤ 350 °C, vorzugsweise ≤ 345 °C, besser ≤ 340 °C, vorteilhaft ≤ 335 °C, besonders vorteilhaft ≤ 330 °C oder ≤ 325 °C, ganz besonders vorteilhaft ≤ 320 °C oder ≤ 315 °C, noch besser ≤ 310 °C, sowie am besten ≤ 300 °C, oder ≤ 290 °C, oder ≤ 280 °C, oder ≤ 270 °C, oder ≤ 260 °C, oder ≤ 250 °C bzw. ≤ 240 °C, oder ≤ 230 °C, oder ≤ 200 °C) eröffnet darüber hinaus die Möglichkeit, nach beendeter Spaltung (nach beendeter Thermolyse) eine erfindungsgemäß als Spaltkatalysator mitverwendete Wirkverbindung aus bei der erfindungsgemäßen Thermolyse in der Regel verbleibenden Rückständen nachträglich, gegebenenfalls bei erhöhter Temperatur und/oder vermindertem Druck, z.B. destillativ und/oder rektifikativ abzutrennen, und so als Wertprodukt, z.B. für ein erfindungsgemäßes Thermolyseverfahren, wiederverwertbar zu erhalten. Wird eine solche Abtrennung nicht vorgenommen, sollten erfindungsgemäß als Spaltkatalysator verwendbare Wirkverbindungen eine Entsorgung von Spaltrückständen durch z.B. Energie liefernde Vollverbrennung derselben ermöglichen, ohne dass die Bildung besonders kritischer, auf eine mitverwendete Wirkverbindung zurückgehender, Verbrennungsgase zu befürchten ist.

Weiterhin wäre es vorteilhaft, wenn der Schmelzpunkt der als Spaltkatalysator verwendeten wenigstens einen Wirksubstanz (wenigstens einen Wirkverbindung) bei Normaldruck zusätzlich ≤ 70 °C, vorzugsweise ≤ 60 °C, besonders bevorzugt ≤ 50 °C, ganz besonders bevorzugt ≤40°C) und am besten ≤ 25 °C beträgt ("dritte Anforderung").

Ein Spaltkatalysator, der neben der ersten und der zweiten Anforderung auch noch die dritte Anforderung erfüllt ist insofern vorteilhaft, als er normalerweise bei einer tieferen Temperatur als das zu spaltende Poly-3-hydroxypropionat selbst schmilzt und dadurch gegenüber dem zu spaltenden Poly-3-hydroxypropionat gegebenenfalls als Lösungsmittel oder als Dispergiermittel fungieren kann. Im Extremfall kann die Thermolyse des Poly-3-hydroxypropionats so aus dessen Lösung, oder aus dessen Suspension, oder aus dessen Emulsion im Spaltkatalysator heraus erfolgen.

Weiterhin ist es vorteilhaft, wenn eine als Spaltkatalysator zu verwendende Wirksubstanz zusätzlich die folgenden Anforderungen erfüllt:
- eine vergleichsweise geringe dynamische Viskosität der Schmelze des Spaltkatalysators unter den Bedingungen der Thermolyse ("vierte Anforderung");
- die Schmelze des Spaltkatalysators vermag festes Poly-3-hydroxypropionat gut zu benetzen ("fünfte Anforderung"); und
- der Flammpunkt der Wirksubstanz liegt bei einer möglichst hohen Temperatur ("sechste Anforderung").

Darüber hinaus sollte eine erfindungsgemäß als Spaltkatalysator zu verwendende Wirksubstanz die Grundanforderung einer möglichst hohen massenspezifischen katalytischen Wirkung erfüllen. D.h., eine möglichst geringe Einsatzmasse der Wirksubstanz (der Wirkverbindung) sollte ausreichend sein, um die gewünschte katalytische Wirkung zu entfalten.

Eigene Untersuchungen der Anmelderin haben zu dem Ergebnis geführt, dass die Grundanforderung und mit dieser in der Regel auch die erste Anforderung von organischen Wirkmolekülen bzw. molekularen organischen Wirkverbindungen (molekularen organischen (Wirk)Substanzen; "molekular" meint hier explizit, dass die organische Wirkverbindung kein Salz, keine ionische Verbindung, ist, und somit z.B. quartäre Ammoniumverbindungen nicht umfasst) erfüllt werden, die neben Kohlenstoff und Wasserstoff als von diesen verschiedene Heteroatome wenigstens ein Stickstoffatom sowie wahlweise wenigstens ein oder mehr als ein Sauerstoffatom mit der Maßgabe kovalent gebunden aufweisen, dass
- das Wirkmolekül (die (Wirk)Substanz) kein über Stickstoff und Sauerstoff hinausgehendes, von Kohlenstoff und Wasserstoff verschiedenes, Heteroatom aufweist, und
- wenigstens ein Stickstoffatom ein tertiäres Stickstoffatom ist.

Vorzugsweise weisen diese Wirkverbindungen kein Stickstoffatom auf, an das ein oder mehr als ein Wasserstoffatom kovalent gebunden ist. Weiterhin weisen diese Wirkverbindungen vorteilhaft kein Sauerstoffatom auf, an das ein Wasserstoffatom kovalent gebunden ist.

Unter einem tertiären Stickstoffatom soll in dieser Schrift ein Stickstoffatom verstanden werden, das nur zu Kohlenstoffatomen kovalente chemische Bindungen aufweist, wobei die Gesamtzahl dieser Kohlenstoffatome nicht mehr als drei und wenigstens zwei beträgt und keines dieser Kohlenstoffatome gleichzeitig eine kovalente chemische Doppelbindung zu einem Sauerstoffatom aufweist. Zur Menge solcher tertiärer Stickstoffatome zählen insbesondere z.B. "iminische" Stickstoffatome, wie sie z.B. das Pyridin und das 3-Hydroxypyridin aufweist.

Das Vorgenannte gilt insbesondere dann, wenn das Molgewicht M der molekularen organischen Wirksubstanz ≥ 59,1 g/mol und ≤ 600 g/mol, vorzugsweise ≥ 75 g/mol und ≤ 500 g/mol, besonders bevorzugt ≥ 100 g/mol und ≤ 400 g/mol, besser ≥ 125 g/mol und ≤ 300 g/mol sowie ganz besonders bevorzugt ≥ 150 g/mol und ≤ 250 g/mol, bzw. ≤ 200 g/mol beträgt.

Eine erhöhte massenspezifische katalytische Spaltwirkung der molekularen organischen Wirksubstanz liegt in der Regel dann vor, wenn sie mehr als ein tertiäres Stickstoffatom (z.B. wenigstens zwei oder wenigstens drei tertiäre Stickstoffatome) aufweist.

Erfindungsgemäß günstig für eine erhöhte massenspezifische katalytische Spaltwirkung ist es auch, wenn wenigstens ein tertiäres Stickstoffatom der organischen Wirksubstanz zu drei voneinander verschiedenen Kohlenstoffatomen (zu nicht mehr und nicht weniger als diesen drei und auch zu keinem anderen Atom) kovalente chemische Bindungen aufweist.

Erfindungsgemäß bevorzugt enthält die relevante molekulare organische Wirksubstanz wenigstens zwei und ganz besonders bevorzugt wenigstens drei tertiäre Stickstoffatome, von denen jedes jeweils zu drei voneinander verschiedenen Kohlenstoffatomen kovalente chemische Bindungen aufweist.

Ganz besonders bevorzugt enthält die relevante Wirksubstanz nur Stickstoffatome die tertiäre Stickstoffatome und vorzugsweise solche tertiären Stickstoffatome sind, dass jedes dieser Stickstoffatome jeweils zu drei voneinander verschiedenen Kohlenstoffatomen kovalente chemische Bindungen aufweist.

Unter den vorstehend ausgeführten, als erfindungsgemäße Spaltkatalysatoren geeigneten molekularen organischen Wirkverbindungen sind daher u.a. jene hervorzuheben, die formal dadurch vom Ammoniak abgeleitet werden können, dass man dessen drei H-Atome durch drei Alkylgruppen ersetzt.

Unter diesen tertiären aliphatischen Aminen sind jene der allgemeinen Formel I bevorzugt, 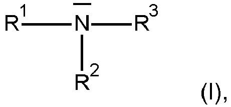 mit R¹, R² und R³ = unabhängig voneinander 1 bis 8 C-Atome, vorzugsweise 1 bis 6 C-Atome und besonders bevorzugt 1 bis 4 C-Atome aufweisende Alkylgruppen (diese Gruppen sind nur aus Wasserstoff und Kohlenstoffatomen aufgebaut und umfassen nicht die Cykloalkylgruppen) oder Cycloalkylgruppen (sind ebenfalls nur aus Wasserstoff und Kohlenstoff aufgebaut).

Unter den tertiären aliphatischen Aminen der allgemeine Formel I sind jene bevorzugt, in denen R¹ = R² = R³ (und vorzugsweise = eine Alkylgruppe) zutrifft. Bevorzugte Reste R¹, R², R³ sind die Methylgruppe, die Ethylgruppe, die iso-Propylgruppe, die n-Propylgruppe, die n-Butylgruppe, die tert.-Butylgruppe und die n-Hexylgruppe sowie die Cyclohexylgruppe.

Beispielhaft genannt seien als Wirkverbindungen der allgemeinen Formel I das Trimethylamin, Triethylamin, Tri-n-Hexylamin, Tri-n-Butylamin und N-Ethyl-N,N-diisopropylamin.

Ferner kommen solche molekularen organischen Wirkverbindungen als erfindungsgemäß vorteilhafte Spaltkatalysatoren in Betracht (im weiteren Verlauf dieser Schrift als Wirkverbindungen der allgemeinen Formel II bezeichnet), die sich von den Wirkverbindungen der allgemeinen Formel (I) formal dadurch ableiten, dass wenigstens einer der Reste R¹, R², R³ eine Alkyl- oder eine Cycloalkylgruppe ist, bei der ein oder mehr als ein Wasserstoffatom durch wenigstens eine der Gruppen -OH, -NH₂, -NHCH₃ und -N(CH₃)₂ ersetzt und/oder die (wahlweise cyclische) Kohlenstoffkette wenigstens einmal durch ein Sauerstoffatom und/oder ein Stickstoffatom unterbrochen ist. Dabei können zwei dieser Reste gemeinsam mit dem sie tragenden tertiären Stickstoffatom auch einen Zyklus (einen Ring) bilden. Vorzugsweise enthalten Wirkverbindungen (II) kein Stickstoffatom, an das ein oder mehr als ein Wasserstoffatom kovalent gebunden ist und/oder kein Sauerstoffatom an das ein Wasserstoffatom kovalent gebunden ist.

Unter den Wirkverbindungen der allgemeinen Formel (II) seien als erfindungsgemäß besonders geeignete Spaltkatalysatoren (für alle in dieser (in der vorliegenden) Schrift aufgeführten Thermolysen, und dies an allen in der vorliegenden Schrift aufgeführten, zu Acrylsäure spaltbaren, Poly-3-hydroxypropionaten) beispielhaft hervorgehoben das Pentamethyldiethylentriamin (M = 173,30 g/mol; Sdp. = 199 °C; Smp. < -20 °C; zu beziehen als Lupragen^{®} N301 von der BASF SE), das N,N,N',N'-Tetramethyl-1,6-hexandiamin (M = 172,31 g/mol; Sdp. = 212 °C; Smp. = -46 °C; zu beziehen als Lupragen^{®} N500 von der BASF SE), der Bis(2-Dimethylaminoethyl)ether (M = 160,3 g/mol; Sdp. = 189 °C; Smp. = 60 °C; zu beziehen als Lupragen® N205 von der BASF SE), der 2,2'-Dimorpholinodiethylether (M = 244,33 g/mol; Sdp. = 309 °C; Smp. = -28 °C; zu beziehen als Lupragen^{®} N106 von der BASF SE), das N,N-Diethylethanolamin (M = 117,19 g/mol; Sdp. = 161 °C; Smp. = -70 °C), das N,N,N',N'-Tetramethyl-1,3-pro-pandiamin (M = 130,23 g/mol; Sdp. = 146 °C; Smp. = -78 °C), das N,N-Dimethylcyclohexylamin (M = 127,23 g/mol; Sdp. = 159 °C; Smp. = -60 °C; zu beziehen als Lupragen^{®} N100 von der BASF SE) und das N,N-Dimethyl-1,3-diaminopropan (M = 102,18 g/mol; Sdp. = 133 °C; Smp. = -60 °C).

Weitere als erfindungsgemäße Spaltkatalysatoren geeignete relevante Wirkverbindungen (Wirkverbindungen III) sind z.B. Derivate des 1,3-Diazols (Imidazols), bei denen der Wasserstoff am Stickstoff des 1,3-Diazols in der 1-Stellung durch eine Alkylgruppe R⁴ mit 1 bis 8 C-Atomen, vorzugsweise 1 bis 6 C-Atomen und besonders bevorzugt 1 bis 4 C-Atomen ersetzt ist. Wahlweise kann zusätzlich der Wasserstoff an einem der Kohlenstoffatome im Diazolring durch eine Alkylgruppe R⁵ mit 1 bis 8 C-Atomen, vorzugsweise 1 bis 6 C-Atomen und besonders bevorzugt 1 bis 4 C-Atomen ersetzt sein.

Beispielhaft aufgeführt seien als Wirkverbindungen III das N-Methylimidazol (M = 82,12 g/mol; Sdp. = 198 °C; Smp. = -2 °C; zu beziehen als Lupragen ® NMI von der BASF SE) und das 1,2-Dimethylimidazol (M = 96,13 g/mol; Sdp. = 204 °C; Smp. = 38 °C).

Unter den vorstehend als erfindungsgemäß besonders geeignete Spaltkatalysatoren beispielhaft aufgeführten molekularen organischen Wirkverbindungen ist das Pentamethyldiethylentriamin nochmals bevorzugt (insbesondere für alle in dieser (in der vorliegenden) Schrift aufgeführten Thermolysen, und dies an allen in der vorliegenden Schrift aufgeführten, zu Acrylsäure spaltbaren, Poly-3-hydroxypropionaten), da es in besonders vorteilhafter Weise die gewünschten Eigenschaften eines erfindungsgemäß günstigen Spaltkatalysators in sich vereint.

Weitere definitionsgemäße Wirkverbindungen, die als erfindungsgemäß geeignete Spaltkatalysatoren von besonderem Interesse sind, sind die Hydroxypyridine (2-Hydroxypyridin, 3-Hydroxypyridin und 4-Hydroxypyridin).

Dies vor allem auch deshalb, weil sie, wie bereits erwähnt, auch für die erfindungsgemäße carbonylierende Umsetzung des Ethylenoxids zum Poly-3-hydroxypropionat geeignete co-Katalysatoren C bilden. In der Regel verbleibt bei der erfindungsgemäßen Abtrennung des Poly-3-hydroxypropionats von einem Produktgemisch A derartiger co-Katalysator C (z.B. 3-Hydro-xypyridin), und/oder gegebenenfalls auch co-Katalysator B, (wenigstens) teilweise im abgetrennten Poly-3-hydroxypropionat (z.B. in selbigem absorbiert), so dass im Rahmen seiner Thermolyse von einer separaten Zugabe an zusätzlichem(n) Spaltkatalysator(en) abgesehen werden kann, da z.B. die im Poly-3-hydroxypropionat bei der Abtrennung desselben verbliebene co-Katalysator C-Verbindung (z.B. das 3-Hydroxypyridin) in ausreichendem Umfang spaltkatalytisch wirkt.

Von besonderer Bedeutung ist in diesem Zusammenhang, dass z.B. bei einer Verwendung von 3-Hydroxypyridin als ein solcher co-Katalysator C wenigstens eine Teilmenge des 3-Hydroxypyridins als Kopfgruppe an die Polyesterkette des Poly-3-hydroxypropionats kovalent gebunden wird, was z.B. zu Polyesterketten der folgenden Strukturen führt: 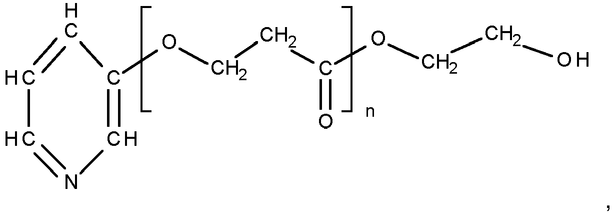 oder 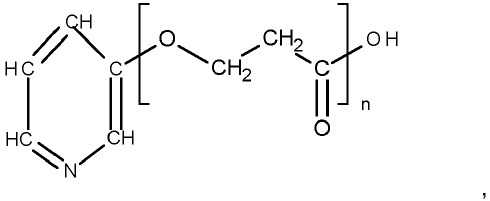 wobei n jeweils eine ganze Zahl ≥ 1 ist, und bis zu 150, oder bis zu 200, oder bis zu 250 und mehr betragen kann.

D.h., es bildet sich Poly-3-hydroxypropionat, das mit der Hydroxylgruppe des 2-Hydroxy-pyridins, oder des 3-Hydroxypyridins, oder des 4-Hydroxypyridins verethert ist.

Dieser Sachverhalt ist erfindungsgemäß deshalb so bemerkenswert, weil ein auf diese Weise an das Poly-3-hydroxypropionat kovalent gebundenes 2-Hydroxypyridin, oder 3-Hydroxypyridin, oder 4-Hydroxypyridin sowohl bei einer erfindungsgemäßen wässrigen Fällung desselben aus z.B. einem Produktgemisch A heraus, als auch bei einer erfindungsgemäßen wässrigen Wäsche desselben nach seiner Abtrennung von einem Produktgemisch A im Poly-3-hydroxypropionat verbleibt (und nicht mit ausgewaschen wird). Dies ist erfindungsgemäß deshalb von wesentlicher Bedeutung, weil als Kopfgruppe an Poly-3-hydroxypropionat chemisch gebundenes Hydroxypyridin (z.B. 2-Hydroxypyridin, oder 3-Hydroxypyridin, oder 4-Hydroxypyridin) seine Fähigkeit, als Spaltkatalysator für die Thermolyse des Poly-3-hydroxypropionats zu wirken, nicht eingebüßt hat. Dies gilt auch mit Wirkung auf Poly-3-hydroxypropionatketten, die kein entsprechendes Hydroxypyridin als Kopfgruppe chemisch gebunden aufweisen.

Im Übrigen betragen katalytisch wirksame Mengen der beschriebenen, erfindungsgemäß als Spaltkatalysatoren geeigneten molekularen organischen Wirkverbindungen, bezogen auf das Gewicht der zu spaltenden Masse an Poly-3-hydroxypropionat, in der Regel 0,01 bis 15 Gew.-% oder 0,05 bis 10 Gew.-%, oft 0,1 bis 5 Gew.-%, vorzugsweise 0,5 bis 4 Gew.-%, oder 1,5 bis 3,5 Gew.-%.

In natürlicher Weise kann die Einsatzmenge an Spaltkatalysator beim erfindungsgemäßen Verfahren auch oberhalb der vorstehend genannten Werte liegen. Dies insbesondere dann, wenn der Spaltkatalysator gleichzeitig als Lösungsmittel bzw. (oder) als Dispergiermittel für das zu spaltende Poly-3-hydroxypropionat fungiert. Vor allem in diesen Fällen können sich die in entsprechender Weise bezogenen Einsatzmengen an Spaltkatalysator leicht auf bis zu 50 Gew.-%, oder bis zu 100 Gew.-%, oder bis zu 150 Gew.-%, oder bis zu 200 Gew.-%, oder bis zu 250 Gew.-%, oder bis zu 300 Gew.-% und mehr belaufen.

Selbstverständlich können auch andere, z.B. hochsiedende organische, Lösungsmittel bzw. (oder) Dispergiermittel wie z.B. ionische Flüssigkeiten, oligomere (vor allem Di-bis Hexamere) Michael-Addukte (Additionsprodukte) der Acrylsäure an sich selbst und an die dabei entstehenden Addukte, wie sie im Rahmen der konventionellen Herstellung von Acrylsäure üblicherweise entstehen (insbesondere z.B. als Sumpfprodukte bei Rektifikationen von Acrylsäuren), Dimethylsulfoxid, N-Methyl-2-pyrrolidon, Dialkylformamid, längerkettige paraffinische Kohlenwasserstoffe, längerkettige Alkanole, Y-Butyrolacton, Ethylencarbonat, Diphenylether, Diglyme, Triglyme, Tetraglyme, Biphenyl, Trikresylphosphat, Dimethylphthalat und/oder Diethylphthalat Bestandteil des das zu spaltende Poly-3-hydroxypropionat und den in der Regel wenigstens einen Spaltkatalysator enthaltenden Spaltgemischs sein. Allerdings mindert ein Beisein von Lösungsmittel bzw. (oder) Dispergiermittel unter ansonsten gleichen Bedingungen in der Regel die Spaltrate.

Der Gewichtsanteil des Poly-3-hydroxypropionats an einem solchen auch Lösungsmittel bzw. (oder) Dispergiermittel enthaltenden Spaltgemisch kann, bezogen auf das Gesamtgewicht des Spaltgemischs, weniger als 95 Gew.-%, oder weniger als 90 Gew.-%, oder weniger als 80 Gew.-%, oder weniger als 70 Gew.-%, oder weniger als 60 Gew.-%, oder weniger als 50 Gew.-%, oder weniger als 40 Gew.-%, oder weniger als 30 Gew.-%, oder weniger als 20 Gew.-%, oder weniger als 10 Gew.-% betragen. In der Regel liegt dieser Gewichtsanteil jedoch bei ≥ 5 Gew.-%.

Unabhängig davon, ob das Poly-3-hydroxypropionat im Spaltgemisch in Form seiner Schmelze, oder in einem Lösungsmittel gelöst, oder in einem Dispergiermittel als Suspension oder Emulsion dispers verteilt vorliegt, liegt die als Spaltkatalysator zugesetzte wenigstens eine molekulare organische Wirkverbindung im Spaltgemisch (d.h., in der Schmelze, im Lösungsmittel, oder im Dispergiermittel) vorzugsweise gelöst vor.

Aus Gründen der Schmelzpunkterniedrigung kann es auch angebracht sein, mehr als einen, z.B. zwei oder drei voneinander verschiedene, Spaltkatalysatoren einzusetzen.

Um einer unerwünschten radikalischen Polymerisation von im Spaltgemisch gebildeter Acrylsäure wahlweise entgegen zu wirken, können dem Spaltgemisch zusätzlich noch entsprechende Polymerisationsinhibitoren zugesetzt werden.

Als solche Polymerisationsinhibitoren kommen grundsätzlich alle diejenigen in Betracht, die im Stand der Technik zum Zweck der Inhibierung einer radikalischen Polymerisation von in flüssiger Phase befindlicher Acrylsäure empfohlen werden. Als solche Polymerisationsinhibitoren kommen in Betracht Alkylphenole, wie o-, m- oder p-Kresol (Methylphenol), 2-tert.-Butyl-4-methylphenol, 6-tert.-Butyl-2,4-dimethylphenol, 2,6-Di-tert.-Butyl-4-methylphenol, 2-tert.-Butylphenol, 4-tert.-Butylphenol, 2,4-di-tert.-Butylphenol und 2-Methyl-4-tert.-Butylphenol, Hydroxyphenole wie Hydrochinon, Brenzcatechin, Resorcin, 2-Methylhydrochinon und 2,5-Di-tert.-Bu-tylhydrochinon, Aminophenole wie z.B. para-Aminophenol, Nitrosophenole wie z.B. para-Nitrosophenol, Alkoxyphenole wie 2-Methoxyphenol, 2-Ethoxyphenol, 4-Methoxyphenol (Hydrochinonmonomethylether) und Mono- oder Di-tert.-Butyl-4-methoxyphenol, Tocopherole wie z.B. α-Tocopherol, N-Oxyle wie 4-Hydroxy-2,2,6,6-tetramethyl-piperidin-N-oxyl, 2,2,6,6-Tetramethylpiperidin-N-oxyl, 4,4',4"-Tris(2,2,6,6-tetramethyl-piperidin-N-oxyl)-phosphit oder 3-Oxo-2,2,5,5-tetramethyl-pyrrolidin-N-oxyl, aromatische Amine oder Phenylendiamine wie z.B. N,N-Diphenylamin, N-Nitroso-di-phenylamin und N,N'-Dialkyl-para-phenylendiamin, wobei die Alkylreste gleich oder verschieden sein können und jeweils unabhängig voneinander aus 1 bis 4 Kohlenstoffatomen bestehen und geradkettig oder verzweigt sein können, Hydroxylamine wie z.B. N,N-Diethylhydroxylamin, phosphorhaltige Verbindungen wie z.B. Triphenylphosphin, Triphenylphosphit, hypophosphorige Säure oder Triethylphosphit, schwefelhaltige Verbindungen wie z.B. Diphenylsulfid oder Phenothiazin, sowie alle vorgenannten Inhibitoren gegebenenfalls in Kombination mit Metallsalzen wie beispielsweise den Chloriden, Dithiocarbonaten, Sulfaten, Salicylaten oder Acetaten von Kupfer, Mangan, Cer, Nickel und/oder Chrom.

Auch können unterschiedliche Gemische der genannten Polymerisationsinhibitoren eingesetzt werden. Bevorzugt werden als Polymerisationsinhibitoren Phenothiazin und/oder Hydrochinonmonomethylether eingesetzt. Darüber hinaus können die vorstehend benannten Polymerisationsinhibitoren durch ein molekularen Sauerstoff enthaltendes Gas (z.B. Luft oder mit Stickstoff verdünnte Luft (vorteilhaft Magerluft)) unterstützt werden. Anwendungstechnisch zweckmäßig werden dabei die Explosionsgrenzen von gasförmigen, Acrylsäure und Sauerstoff enthaltenden, Mischungen beachtet (vgl. z.B. WO 2004/007405 A1). Beispielsweise kann die vorstehende Unterstützung so erfolgen, dass die bei der Spaltung gebildete Acrylsäure mit Hilfe eines molekularen Sauerstoff enthaltenden Strippgases kontinuierlich aus dem Spaltgemisch herausgestrippt wird (ein solches Strippen kann sowohl bei Unterdruck, Normaldruck oder auch bei oberhalb von Normaldruck liegenden Arbeitsdrücken erfolgen).

Je nach verwendetem Polymerisationsinhibitor (oder Gemisch an Polymerisationsinhibitoren) wird seine (dessen) Einsatzmenge bezogen auf den Gehalt an Poly-3-hydroxypropionat im Spaltgemisch 10 bis 1000 Gew.ppm, häufig 50 bis 500 Gew.ppm und vielfach 150 bis 350 Gew.ppm betragen.

Ansonsten kann die Spaltung des erfindungsgemäß erhältlichen Poly-3-hydroxypropionats wie bei der Würdigung der bekannten Spaltverfahren des Standes der Technik bereits ausgeführt erfolgen. D.h., in typischer Weise anzuwendende Spalttemperaturen (Temperaturen, bei denen die Thermolyse durchgeführt wird) können sich im Bereich von 50 bis 350 °C, oder im Bereich von 100 bis 300 °C bewegen. Erfindungsgemäß vorteilhaft werden die angewendeten Spalttemperaturen 150 bis 220 °C und besonders bevorzugt 160 bis 200 °C betragen.

Ebenso kann der Arbeitsdruck während der erfindungsgemäßen Thermolyse des Poly-3-hydroxypropionats (in der Gasatmosphäre) sowohl bei Normaldruck (= 1,0133 · 10⁵ Pa) als auch oberhalb oder unterhalb von Normaldruck liegen. D.h., der Arbeitsdruck kann 10² bis 10⁷ Pa, oder 10³ bis 10⁶ Pa, oder 2 · 10³ bis 5 · 10⁵ Pa, oder 5 · 10³ bis 3 · 10⁵ Pa betragen.

Aus der bei der Thermolyse des erfindungsgemäß abgetrennten Poly-3-hydroxypropionats erzeugten Acrylsäure enthaltenden Gasphase kann die Acrylsäure in an sich bekannter Weise durch absorptive und/oder kondensative Maßnahmen in die flüssige Phase überführt werden. Im Regelfall kann diese flüssige Phase bereits die für Folgeverwendungen wie z.B. radikalische Polymerisationen geeignete erfindungsgemäß erhältliche Acrylsäure sein (insbesondere wenn die so gewonnene Acrylsäure vorab ihrer Folgeverwendung im Rahmen einer radikalisch initiierten Polymerisation nicht zwischengelagert wird, wird man vorgenannte Überführung in die flüssige Phase vorteilhaft ohne Mitverwendung von, eine (spätere) radikalisch initiierte Polymerisation beeinträchtigende, Polymerisationsinhibitoren vornehmen).

Unter Anwendung eines oder mehrerer thermischer Trennverfahren (derartige thermische Trennverfahren sind insbesondere die Rektifikation, die Extraktion, die Desorption, die Destillation, die Strippung, die Absorption, die Azeotroprektifikation und/oder die Kristallisation) auf die Acrylsäure enthaltende flüssige Phase kann die Acrylsäure aus der flüssigen Phase heraus aber auch bedarfsgerecht zu beliebiger Reinheit aufgereinigt werden (z.B. analog wie in den Schriften DE 10243625 A1, DE 10332758 A1, DE 102007004960 A1 und DE 102012204436 A1 und dem in diesen Schriften zitierten Stand der Technik beschrieben).

Als ein bevorzugtes thermisches Trennverfahren eignet sich das Verfahren der Kristallisation. Innerhalb der kristallisativen Trennverfahren ist für den vorgenannten Zweck das Verfahren der Suspensionskristallisation bevorzugt anzuwenden (z.B. analog wie in DE 102007043759 A1, DE 102008042008 A1 und DE 102008042010 A1 sowie dem in diesen Schriften zitierten Stand der Technik beschrieben).

Die Abtrennung des Suspensionskristallisats aus der Kristallsuspension wird anwendungstechnisch zweckmäßig in einer Waschschmelzewaschkolonne (vgl. WO 01/77056 A1, als Waschflüssigkeit wird die Schmelze von bereits entsprechend gereinigten Acrylsäurekristallen verwendet), vorzugsweise in einer hydraulischen Waschschmelzewaschkolonne vorgenommen (analog wie z.B. in WO 01/77056 A1, WO 02/09839 A1, WO 03/041832 A1, WO 2006/111565 A2, WO 2010/094637 A1 und WO 2011/045356 A1 und dem in diesen Schriften zitierten Stand der Technik beschrieben).

Im Übrigen kann die erfindungsgemäße Spaltung des Poly-3 hydroxypropionats großtechnisch sowohl absatzweise als auch kontinuierlich durchgeführt werden.

Anwendungstechnisch zweckmäßig kann eine kontinuierliche Verfahrensführung (und mit ihr eine zugehörige erfindungsgemäße Thermolysezone A) wie folgt ausgestaltet sein. Als Spaltreaktor fungiert der Sumpfraum einer trennwirksame Einbauten enthaltenden Trennkolonne (als trennwirksame Einbauten kommen z.B. Stoffaustauschböden wie Dual-Flow-Böden in Betracht; grundsätzlich kann die Trennkolonne aber auch leer sein, d.h., keine trennwirksamen Einbauen aufweisen). Das flüssige Spaltgemisch (das eine Schmelze, eine Lösung, eine Suspension oder eine Emulsion sein kann) wird im unteren Drittel der Trennkolonne zugeführt (grundsätzlich kann die Zufuhr auch unmittelbar in den Sumpfraum hinein erfolgen; eine solche Zufuhr kann im Prinzip auch "fest" erfolgen).

Unterhalb der Zufuhrstelle (vorteilhaft aus dem Sumpfraum heraus) wird mittels einer Pumpe kontinuierlich ein flüssiger Stoffstrom (der gegebenenfalls auch eine Suspension sein kann) entnommen und über einen indirekten Wärmetauscher unterhalb der Zufuhrstelle des Spaltgemischs wieder in die Trennkolonne zurückgeführt. Beim Durchströmen des indirekten Wärmeaustauschers wird die für die Thermolyse benötigte thermische Energie zugeführt. Anwendungstechnisch vorteilhaft ist der indirekte Wärmeaustauscher ein Zwangsumlaufentspannungswärmeüberträger.

Am Kopf oder via Seitenabzug kann die Acrylsäure aus der Trennkolonne herausgeführt werden. Für den Fall, dass die Trennkolonne trennwirksame Einbauten aufweist, wird im Kopfbereich der Trennkolonne Kondensatbildung bewirkt und ein Teil des gebildeten Kondensats in der Trennkolonne im Gegenstrom zur in selbiger aufsteigenden Acrylsäure (z.B. von einem Strippgas geführt und/oder bei vermindertem Kopfdruck dem Druckgefälle folgend) als Rücklaufflüssigkeit absteigend geführt. Als Auslass für schwerstsiedende Nebenkomponenten wird von der Sumpfflüssigkeit kontinuierlich eine Teilmenge ausgelassen und ihrer Entsorgung (z.B. Verbrennung) zugeführt.

Vorteilhaft an gemäß der erfindungsgemäßen Verfahrensweise hergestellter (bzw. auf eine erfindungsgemäße Herstellung zurückgehender), z.B. durch absorptive und/oder kondensative Maßnahmen aus der bei der Thermolyse des Poly-3-hydroxypropionats erzeugten Gasphase in die kondensierte (vorzugsweise flüssige) Phase überführter Acrylsäure ist, dass sie nicht den für durch heterogen katalysierte Partialoxidationen von C₃-Vorläuferverbindungen erzeugte Acrylsäure (z.B. Propylen, Propan, Acrolein, Glycerin, Propionsäure, Propanol etc.) typischen Fingerabdruck an in ihr als Verunreinigungen enthaltenen niedermolekularen Aldehyden aufweist (vgl. z.B. DE 102011076931 A1).

Diese erweisen sich bei einer Verwendung der Acrylsäure und/oder ihrer konjugierten (Brönsted-)Base zur Herstellung von Polymerisaten durch radikalisch initiierte Polymerisation, wahlweise im Gemisch mit anderen einfach und/oder mehrfach (z.B. ethylenisch) ungesättigten Verbindungen, selbst in geringsten Mengen (1 bis 10 Gew.ppm bezogen auf das Gewicht der Masse an Acrylsäure) als äußerst störend (beispielsweise können sie die radikalisch initiierte Polymerisation in unerwünschter Weise verzögern oder die Herstellung von Polymerisat mit besonders hohem Molekulargewicht infolge ihrer "regelnden Wirkung" behindern bzw. beeinträchtigen).

Demgemäß sind solche Verfahren der erfindungsgemäßen Herstellung von Acrylsäure besonders vorteilhaft, an die sich ein Verfahren der radikalischen Polymerisation anschließt, in welchem die hergestellte Acrylsäure als solche und/oder in Form ihrer konjugierten Base (gemeint ist hier die konjugierte Brönsted-Base, das Acrylatanion), wahlweise im Gemisch mit anderen einfach und/oder mehrfach ungesättigten Verbindungen, radikalisch initiiert in Polymerisat ein-polymerisiert wird.

Dies gilt insbesondere dann, wenn das Verfahren der radikalischen Polymerisation ein Verfahren zur Herstellung von Wasser "superabsorbierendem" Polymerisat ist, wie es z.B. in Hygieneartikeln wie Babywindeln zum Einsatz kommt (vgl. DE 102011076931 A1 und den in selbiger Schrift zitierten Stand der Technik).

Demgemäß umfasst vorliegende Erfindung insbesondere die folgenden erfindungsgemäßen Ausführungsformen:
1. Verfahren zur Herstellung von Acrylsäure aus Ethylenoxid und Kohlenmonoxid, das wenigstens folgende Verfahrensschritte umfasst:
   - eine carbonylierende Umsetzung von in einem nicht protischen Lösungsmittel gelöstem Ethylenoxid mit Kohlenmonoxid bei erhöhtem Druck und erhöhter Temperatur in Anwesenheit eines Katalysatorsystems, das wenigstens eine Kobaltquelle umfasst, in einer Reaktionszone A unter Erhalt eines Poly-3-hydroxypropionat enthaltenden Produktgemischs A,
   - eine Abtrennung von Poly-3-hydroxypropionat vom Produktgemisch A in einer Trennzone A, und
   - eine Thermolyse von in der Trennzone A abgetrenntem Poly-3-hydroxypropionat in einer Thermolysezone A unter Bildung von Acrylsäure,
      dadurch gekennzeichnet, dass die Abtrennung von Poly-3-hydroxypropionat vom Produktgemisch A in der Trennzone A wenigstens eine der nachfolgenden Verfahrensmaßnahmen umfasst:
      - einen Zusatz von Wasser und/oder einer wässrigen Lösung als wässrige Fällflüssigkeit zu einer oder mehreren Teilmengen des Produktgemischs A und/oder zur Gesamtmenge des Produktgemischs A, um in einer Teilmenge des Produktgemischs A oder in der Gesamtmenge des Produktgemischs A gelöst enthaltenes Poly-3-hydroxypropionat auszufällen;
      - ein Waschen von vom Produktgemisch A in der Trennzone A abgetrenntem Poly-3-hydroxypropionat mit Wasser und/oder mit einer wässrigen Lösung als wässrige Waschflüssigkeit.
2. Verfahren gemäß Ausführungsformen 1, dadurch gekennzeichnet, dass das nicht protische Lösungsmittel wenigstens ein Lösungsmittel aus der Gruppe bestehend aus gesättigten Kohlenwasserstoffen, aromatischen Kohlenwasserstoffen, halogenierten gesättigten Kohlenwasserstoffen, halogenierten aromatischen Kohlenwasserstoffen, Estern organischer Säuren, Ketonen, Nitrilen, Dialkylamiden, Kohlensäureestern, Sulfoxiden, Sulfonen, N-Alkylpyrrolidonen, cyclischen Ethern und nicht cyclischen Ethern umfasst oder ist.
3. Verfahren gemäß Ausführungsform 1 oder 2, dadurch gekennzeichnet, dass das nicht protische Lösungsmittel wenigstens ein Lösungsmittel aus der Gruppe bestehend aus n-Hexan, n-Heptan, Petrolether, Cyclohexan, Benzol, Toluol, Dichlormethan, n-Butylpropionat, Phenylacetat, Glycerinacetat, Essigsäureethylester, Aceton, Ethylmethylketon, Methylisobutylketon, Benzophenon, Acetonitril, Propionitril, n-Butyronitril, Benzonitril, Dimethylformamid, Dimethylacetamid, Dimethylcarbonat, Diethylcarbonat, Ethylmethylcarbonat, Ethylencarbonat, Propylencarbonat, Dimethylsulfoxid, Sulfolan, N-Methylpyrrolidon, Ethylenoxid, Diethylether, Anisol, Terahydrofuran, 1,4-Dioxan, Diphenylether, Alkylenglykoldialkylether und Polyalkylenglykoldialkylether umfasst oder ist.
4. Verfahren gemäß Ausführungsform 3, dadurch gekennzeichnet, dass der Alkylenglykoldialkylether ein Ethylenglykoldialkylether ist.
5. Verfahren gemäß Ausführungsform 3, dadurch gekennzeichnet, dass der Ethylenglykoldialkylether der Ethylenglykoldimethylether ist.
6. Verfahren gemäß Ausführungsform 3, dadurch gekennzeichnet, dass der Polyalkylenglykolether der Diethylenglykoldiethylether, der Diethylenglykoldimethylether, der Triethyl-englykoldimethylether und/oder der Tetraethylenglykoldimethylether ist.
7. Verfahren gemäß einer der Ausführungsformen 1 bis 3, dadurch gekennzeichnet, dass das nicht protische Lösungsmittel wenigstens ein kovalent gebundenes Sauerstoffatom aufweist.
8. Verfahren gemäß Ausführungsform 7, dadurch gekennzeichnet, dass das Sauerstoffatom ein Ethersauerstoffatom ist.
9. Verfahren gemäß einer der Ausführungsformen 1 bis 3, dadurch gekennzeichnet, dass das nicht protische Lösungsmittel eine oder mehr als eine Substanz umfasst oder ist, die als von Kohlenstoff und Wasserstoff verschiedene Atomsorte höchstens Sauerstoff und/oder Schwefel enthält.
10. Verfahren gemäß einer der Ausführungsformen 1 bis 9, dadurch gekennzeichnet, dass das nicht protische Lösungsmittel bei einem Druck von 1,0133·10⁵ Pa und wenigstens einer im Bereich von 0 °C bis 50 °C liegenden Temperatur flüssig ist.
11. Verfahren gemäß einer der Ausführungsformen 1 bis 10, dadurch gekennzeichnet, dass das nicht protische Lösungsmittel bei einem Druck von 1,0133·10⁵ Pa und wenigstens einer im Bereich von 5 °C bis 40 °C liegenden Temperatur flüssig ist.
12. Verfahren gemäß einer der Ausführungsformen 1 bis 10, dadurch gekennzeichnet, dass das nicht protische Lösungsmittel bei einem Druck von 1,0133·10⁵ Pa und wenigstens einer im Bereich von 10 °C bis 30 °C liegenden Temperatur flüssig ist.
13. Verfahren gemäß einer der Ausführungsformen 1 bis 12, dadurch gekennzeichnet, dass das nicht protische Lösungsmittel wenigstens ein Lösungsmittel umfasst oder ist, dessen relative statische Permittivitätszahl als flüssige Reinsubstanz bei der Temperatur 293,15 K und dem Druck 1,0133·10⁵ Pa im Bereich von 2 bis 35 liegt.
14. Verfahren gemäß einer der Ausführungsformen 1 bis 13, dadurch gekennzeichnet, dass das nicht protische Lösungsmittel wenigstens ein Lösungsmittel umfasst oder ist, dessen relative statische Permittivitätszahl als flüssige Reinsubstanz bei der Temperatur 293,15 K und dem Druck 1,0133·10⁵ Pa im Bereich von 3 bis 20 liegt.
15. Verfahren gemäß einer der Ausführungsformen 1 bis 14, dadurch gekennzeichnet, dass das nicht protische Lösungsmittel wenigstens ein Lösungsmittel umfasst oder ist, dessen relative statische Permittivitätszahl als flüssige Reinsubstanz bei der Temperatur 293,15 K und dem Druck 1,0133·10⁵ Pa im Bereich von 4 bis 15 liegt.
16. Verfahren gemäß einer der Ausführungsformen 1 bis 15, dadurch gekennzeichnet, dass das nicht protische Lösungsmittel wenigstens ein Lösungsmittel umfasst oder ist, dessen relative statische Permittivitätszahl als flüssige Reinsubstanz bei der Temperatur 293,15 K und dem Druck 1,0133·10⁵ Pa im Bereich von 5 bis 10 liegt.
17. Verfahren gemäß Ausführungsform 1, dadurch gekennzeichnet, dass das nicht protische Lösungsmittel wenigstens ein Lösungsmittel aus der Gruppe bestehend aus Tetrahydrofuran, Ethylenoxid, 1,4-Dioxan, Ethylenglykoldimethylether, Diethylenglykoldimethylether und Triethylenglykoldimethylether umfasst oder ist.
18. Verfahren gemäß Ausführungsform 1, dadurch gekennzeichnet, dass das nicht protische Lösungsmittel wenigstens ein Lösungsmittel aus der Gruppe bestehend aus Ethylenglykoldimethylether, Diethylenglykoldimethylether und Triethylenglykoldimethylether umfasst oder ist.
19. Verfahren gemäß einer der Ausführungsformen 1 bis 18, dadurch gekennzeichnet, dass das nicht protische Lösungsmittel nur zu wenigstens 90% seines Gewichts ein nicht protisches Lösungsmittel ist.
20. Verfahren gemäß einer der Ausführungsformen 1 bis 18, dadurch gekennzeichnet, dass das nicht protische Lösungsmittel nur zu wenigstens 95% seines Gewichts ein nicht protisches Lösungsmittel ist.
21. Verfahren gemäß einer der Ausführungsformen 1 bis 18, dadurch gekennzeichnet, dass das nicht protische Lösungsmittel nur zu wenigstens 98% seines Gewichts ein nicht protisches Lösungsmittel ist.
22. Verfahren gemäß einer der Ausführungsformen 1 bis 18, dadurch gekennzeichnet, dass das nicht protische Lösungsmittel nur zu wenigstens 99% seines Gewichts ein nicht protisches Lösungsmittel ist.
23. Verfahren gemäß einer der Ausführungsformen 1 bis 22, dadurch gekennzeichnet, dass das vom Produktgemisch A abgetrennte Poly-3-hydroxypropionat ein relatives gewichtsmittleres Molekulargewicht von 1000 bis 20000, oder von 2000 bis 15000 aufweist.
24. Verfahren gemäß einer der Ausführungsformen 1 bis 23, dadurch gekennzeichnet, dass die Polydispersität Q des vom Produktgemisch A abgetrennten Poly-3-hydroxypropionats kleiner als oder gleich groß wie 2,5 ist.
25. Verfahren gemäß einer der Ausführungsformen 1 bis 24, dadurch gekennzeichnet, dass das Katalysatorsystem die wenigstens eine Kobaltquelle in einer solchen Menge umfasst, dass diese, bezogen auf die molare Menge an carbonylierend umzusetzendem Ethylenoxid, 0,005 bis 20 mol-% Co enthält.
26. Verfahren gemäß einer der Ausführungsformen 1 bis 25, dadurch gekennzeichnet, dass das Katalysatorsystem die wenigstens eine Kobaltquelle in einer solchen Menge umfasst, dass diese, bezogen auf die molare Menge an carbonylierend umzusetzendem Ethylenoxid, 0,05 bis 10 mol-% Co enthält.
27. Verfahren gemäß einer der Ausführungsformen 1 bis 26, dadurch gekennzeichnet, dass das Katalysatorsystem die wenigstens eine Kobaltquelle in einer solchen Menge umfasst, dass diese, bezogen auf die molare Menge an carbonylierend umzusetzendem Ethylenoxid, 0,1 bis 8 mol-% Co enthält.
28. Verfahren gemäß einer der Ausführungsformen 1 bis 27, dadurch gekennzeichnet, dass das Katalysatorsystem die wenigstens eine Kobaltquelle in einer solchen Menge umfasst, dass diese, bezogen auf die molare Menge an carbonylierend umzusetzendem Ethylenoxid, 0,5 bis 5 mol-% Co enthält.
29. Verfahren gemäß einer der Ausführungsformen 1 bis 28, dadurch gekennzeichnet, dass wenigstens eine Kobaltquelle ein Salz des Kobalts ist.
30. Verfahren gemäß Ausführungsform 29, dadurch gekennzeichnet, dass das Salz des Kobalts Kobaltformiat, Kobaltacetat, Kobaltacetylacetonat und/oder Kobaltsulfat ist.
31. Verfahren gemäß einer der Ausführungsformen 1 bis 28, dadurch gekennzeichnet, dass wenigstens eine Kobaltquelle feinteiliges Kobaltmetall ist.
32. Verfahren gemäß einer der Ausführungsformen 1 bis 28, dadurch gekennzeichnet, dass wenigstens eine Kobaltquelle eine Kobaltcarbonylverbindung ist.
33. Verfahren gemäß einer der Ausführungsformen 1 bis 28, dadurch gekennzeichnet, dass wenigstens eine Kobaltquelle Dicobaltoctacarbonyl ist.
34. Verfahren gemäß einer der Ausführungsformen 1 bis 33, dadurch gekennzeichnet, dass das Katalysatorsystem wenigstens eine Brönstedsäure als co-Katalysator A umfasst.
35. Verfahren gemäß Ausführungsform 34, dadurch gekennzeichnet, dass die wenigstens eine Brönstedsäure wenigstens eine Säure aus der Gruppe bestehend aus anorganischen Säuren, organischen Carbonsäuren, organischen Sulfonsäuren, Wasser und hydroxyaromatischen Verbindungen ist.
36. Verfahren gemäß Ausführungsform 35, dadurch gekennzeichnet, dass die wenigstens eine Brönstedsäure wenigstens eine Säure aus der Gruppe bestehend aus Wasser, Salzsäure, Schwefelsäure, Phosphorsäure, Ameisensäure, Essigsäure, Adipinsäure, Glutarsäure, Trichloressigsäure, Trifluoressigsäure, Benzoesäure, 2,4,6-Trimethylbenzoesäure, p-Toluolsulfonsäure, Phenol, 1-Naphthol und 2-Naphthol ist.
37. Verfahren gemäß Ausführungsform 36, dadurch gekennzeichnet, dass die wenigstens eine Brönstedsäure Phenol und/oder Essigsäure ist.
38. Verfahren gemäß einer der Ausführungsformen 34 bis 37, dadurch gekennzeichnet, dass das Katalysatorsystem die wenigstens eine Kobaltquelle und den wenigstens einen co-Katalysator A in solchen Mengen enthält, dass das Verhältnis M_{A} : M_{Co}, gebildet aus der im Katalysatorsystem insgesamt enthaltenen molaren Menge M_{A} an co-Katalysatoren A und der im Katalysatorsystem insgesamt enthaltenen molaren Menge M_{Co} an Kobalt, 5 : 1 bis 1 : 5 beträgt.
39. Verfahren gemäß einer der Ausführungsformen 34 bis 38, dadurch gekennzeichnet, dass das Katalysatorsystem die wenigstens eine Kobaltquelle und den wenigstens einen co-Katalysator A in solchen Mengen enthält, dass das Verhältnis M_{A} : M_{Co}, gebildet aus der im Katalysatorsystem insgesamt enthaltenen molaren Menge M_{A} an co-Katalysatoren A und der im Katalysatorsystem insgesamt enthaltenen molaren Menge M_{Co} an Kobalt, 4 : 1 bis 1 : 4 beträgt.
40. Verfahren gemäß einer der Ausführungsformen 34 bis 39, dadurch gekennzeichnet, dass das Katalysatorsystem die wenigstens eine Kobaltquelle und den wenigstens einen co-Katalysator A in solchen Mengen enthält, dass das Verhältnis M_{A} : M_{Co}, gebildet aus der im Katalysatorsystem insgesamt enthaltenen molaren Menge M_{A} an co-Katalysatoren A und der im Katalysatorsystem insgesamt enthaltenen molaren Menge M_{Co} an Kobalt, 3 : 1 bis 1 : 3 beträgt.
41. Verfahren gemäß einer der Ausführungsformen 34 bis 40, dadurch gekennzeichnet, dass das Katalysatorsystem die wenigstens eine Kobaltquelle und den wenigstens einen co-Katalysator A in solchen Mengen enthält, dass das Verhältnis M_{A} : M_{Co}, gebildet aus der im Katalysatorsystem insgesamt enthaltenen molaren Menge M_{A} an co-Katalysatoren A und der im Katalysatorsystem insgesamt enthaltenen molaren Menge M_{Co} an Kobalt, 2 : 1 bis 1 : 2 beträgt.
42. Verfahren gemäß einer der Ausführungsformen 1 bis 41, dadurch gekennzeichnet, dass das Katalysatorsystem wenigstens eine Brönstedbase als co-Katalysator B umfasst.
43. Verfahren gemäß Ausführungsform 42, dadurch gekennzeichnet, dass der pK_{B}-Wert der wenigstens einen Brönstedbase ≥ 7 beträgt.
44. Verfahren gemäß Ausführungsform 42, dadurch gekennzeichnet, dass der pK_{B}-Wert der wenigstens einen Brönstedbase ≥ 8 beträgt.
45. Verfahren gemäß Ausführungsform 42, dadurch gekennzeichnet, dass der pK_{B}-Wert der wenigstens einen Brönstedbase ≥ 9 beträgt.
46. Verfahren gemäß Ausführungsform 42, dadurch gekennzeichnet, dass der pK_{B}-Wert der wenigstens einen Brönstedbase ≥ 10 beträgt.
47. Verfahren gemäß einer der Ausführungsformen 42 bis 46, dadurch gekennzeichnet, dass die wenigstens eine Brönstedbase eine aromatische oder eine nicht aromatische zyklische Verbindung ist, die im Ring neben Kohlenstoffatomen wenigstens ein Stickstoffatom aufweist.
48. Verfahren gemäß Ausführungsform 42, dadurch gekennzeichnet, dass die wenigstens eine Brönstedbase wenigstens eine Brönstedbase aus der Gruppe bestehend aus den Halogenidanionen J⁻, Cl⁻ und F⁻, dem Acetatanion, Pyrrol, N-Methyl-2-pyrrolidon, 3- Pyrrolidon, Piperidin, N-Methylpiperidin, Indol, Indolin, Imidazol, Pyrazol, N,N-Dimethylformamid, Acetanilin, N-Methyl-Imidazol, N-Methyl-2-pyrrolidon, 3- Pyrrolidon, Piperidin, N-Methylpiperidin, Indol, Indolin, Imidazol, Pyrazol, N,N-Dimethylformamid, Acetanilid, N-Methyl-Imidazol, 2-Picolin, 3-Picolin, 4-Picolin, Pyrimidin, das Amid der Nicotinsäure, Pyrazin, Chinolin, Isochinolin, Chinoxalin, 1,10-Phenanthrolin, die Bipyridine und Pyridin ist.
49. Verfahren gemäß einer der Ausführungsformen 42 bis 48, dadurch gekennzeichnet, dass das Katalysatorsystem die wenigstens eine Kobaltquelle und den wenigstens einen co-Katalysator B in solchen Mengen enthält, dass das Verhältnis M_{B} : M_{Co}, gebildet aus der im Katalysatorsystem insgesamt enthaltenen molaren Menge M_{B} an co-Katalysatoren B und der im Katalysatorsystem insgesamt enthaltenen molaren Menge M_{Co} an Kobalt, 5 : 1 bis 1: 5 beträgt.
50. Verfahren gemäß einer der Ausführungsformen 42 bis 49, dadurch gekennzeichnet, dass das Katalysatorsystem die wenigstens eine Kobaltquelle und den wenigstens einen co-Katalysator B in solchen Mengen enthält, dass das Verhältnis M_{B} : M_{Co}, gebildet aus der im Katalysatorsystem insgesamt enthaltenen molaren Menge M_{B} an co-Katalysatoren B und der im Katalysatorsystem insgesamt enthaltenen molaren Menge M_{Co} an Kobalt, 4 : 1 bis 1: 4 beträgt.
51. Verfahren gemäß einer der Ausführungsformen 42 bis 50, dadurch gekennzeichnet, dass das Katalysatorsystem die wenigstens eine Kobaltquelle und den wenigstens einen co-Katalysator B in solchen Mengen enthält, dass das Verhältnis M_{B} : M_{Co}, gebildet aus der im Katalysatorsystem insgesamt enthaltenen molaren Menge M_{B} an co-Katalysatoren B und der im Katalysatorsystem insgesamt enthaltenen molaren Menge M_{Co} an Kobalt, 3 : 1 bis 1: 3 beträgt.
52. Verfahren gemäß einer der Ausführungsformen 42 bis 51, dadurch gekennzeichnet, dass das Katalysatorsystem die wenigstens eine Kobaltquelle und den wenigstens einen co-Katalysator B in solchen Mengen enthält, dass das Verhältnis M_{B} : M_{Co}, gebildet aus der im Katalysatorsystem insgesamt enthaltenen molaren Menge M_{B} an co-Katalysatoren B und der im Katalysatorsystem insgesamt enthaltenen molaren Menge M_{Co} an Kobalt, 2 : 1 bis 1: 2 beträgt.
53. Verfahren gemäß einer der Ausführungsformen 1 bis 52, dadurch gekennzeichnet, dass das Katalysatorsystem wenigstens eine Brönstedsäure als co-Katalysator A und wenigstens eine Brönstedbase als co-Katalysator B umfasst.
54. Verfahren gemäß Ausführungsform 53, dadurch gekennzeichnet, dass der co-Katalysator A Phenol und der co-Katalysator B Pyridin ist.
55. Verfahren gemäß Ausführungsform 53 oder 54, dadurch gekennzeichnet, dass das Katalysatorsystem den wenigstens einen co-Katalysator A und den wenigstens einen co-Katalysator B in solchen Mengen enthält, dass das Verhältnis M_{A} : M_{B}, gebildet aus der im Katalysatorsystem insgesamt enthaltenen molaren Menge M_{A} an co-Katalysatoren A und der im Katalysatorsystem insgesamt enthaltenen molaren Menge M_{B} an co-Katalysatoren B, 1 : 4 bis 4: 1 beträgt.
56. Verfahren gemäß einer der Ausführungsformen 53 bis 55, dadurch gekennzeichnet, dass das Katalysatorsystem den wenigstens einen co-Katalysator A und den wenigstens einen co-Katalysator B in solchen Mengen enthält, dass das Verhältnis M_{A} : M_{B}, gebildet aus der im Katalysatorsystem insgesamt enthaltenen molaren Menge M_{A} an co-Katalysatoren A und der im Katalysatorsystem insgesamt enthaltenen molaren Menge M_{B} an co-Katalysatoren B, 1 : 2 bis 2: 1 beträgt.
57. Verfahren gemäß einer der Ausführungsformen 53 bis 56, dadurch gekennzeichnet, dass das Katalysatorsystem den wenigstens einen co-Katalysator A und den wenigstens einen co-Katalysator B in solchen Mengen enthält, dass das Verhältnis M_{A} : M_{B}, gebildet aus der im Katalysatorsystem insgesamt enthaltenen molaren Menge M_{A} an co-Katalysatoren A und der im Katalysatorsystem insgesamt enthaltenen molaren Menge M_{B} an co-Katalysatoren B, 1 : 1 beträgt.
58. Verfahren gemäß einer der Ausführungsformen 1 bis 57, dadurch gekennzeichnet, dass das Katalysatorsystem wenigstens eine Verbindung als wenigstens einen co-Katalysator C umfasst, die sowohl wenigstens eine nucleophile brönstedbasische Funktionalität wie ein co-Katalysator B, als auch wenigstens eine im Sinne von Brönsted saure Funktionalität wie ein co-Katalysator A aufweist.
59. Verfahren gemäß Ausführungsform 58, dadurch gekennzeichnet, dass der wenigstens eine co-Katalysator C ein aromatischer Stickstoff-Heterozyklus ist, der zusätzlich wenigstens eine Hydroxylgruppe und/oder wenigsten eine Carboxylgruppe kovalent gebunden aufweist.
60. Verfahren gemäß Ausführungsform 59, dadurch gekennzeichnet, dass der aromatische Stickstoff-Heterozyklus an ein oder an mehr als ein anderes aromatisches und/oder aliphatisches Ringsystem anelliert ist.
61. Verfahren gemäß Ausführungsform 60, dadurch gekennzeichnet, dass die wenigstens eine Hydroxylgruppe und/oder Carboxylgruppe an das anellierte Ringsystem kovalent gebunden ist.
62. Verfahren gemäß einer der Ausführungsformen 58 oder 59, dadurch gekennzeichnet, dass der wenigstens eine co-Katalysator C wenigstens eine Verbindung aus der Gruppe bestehend aus 2-Hydroxpyridin, 3-Hydroxypyridin, 4-Hydroxypyridin, 3,4-Dihydroxypyridin, 3-Hydroxychinolin, 4-Hydroxy-2-Methylpyridin, 3-Hydroxy-4-Methylpyridin, 2,6-Dihydroxy-pyridin, 2-Hydroxychinolin, 1-Hydroxyisochinolin, 3-Hydroxychinolin, 2,3-Dihydroxy-chinoxalin, 8-Hydroxychinolin, 2-Pyridylmethanol, 3-Pyridylmethanol, 2-(2-Pyridyl)ethanol und Nicotinsäure ist.
63. Verfahren gemäß einer der Ausführungsformen 58 bis 62, dadurch gekennzeichnet, dass das nicht protische Lösungsmittel Diglyme umfasst, die wenigstens eine Kobaltquelle Dicobaltoctacarbonyl umfasst, und das Katalysatorsystem zusätzlich 3-Hydroxypyridin als wenigstens einen co-Katalysator C umfasst.
64. Verfahren gemäß einer der Ausführungsformen 58 bis 63, dadurch gekennzeichnet, dass das Katalysatorsystem die wenigstens eine Kobaltquelle und den wenigstens einen co-Katalysator C in solchen Mengen enthält, dass das Verhältnis M_{C} : M_{Co}, gebildet aus der im Katalysatorsystem insgesamt enthaltenen molaren Menge M_{C} an co-Katalysatoren C und der im Katalysatorsystem insgesamt enthaltenen molaren Menge M_{Co} an Kobalt, 5 : 1 bis 1: 5 beträgt.
65. Verfahren gemäß einer der Ausführungsformen 58 bis 64, dadurch gekennzeichnet, dass das Katalysatorsystem die wenigstens eine Kobaltquelle und den wenigstens einen co-Katalysator C in solchen Mengen enthält, dass das Verhältnis M_{C} : M_{Co}, gebildet aus der im Katalysatorsystem insgesamt enthaltenen molaren Menge M_{C} an co-Katalysatoren C und der im Katalysatorsystem insgesamt enthaltenen molaren Menge M_{Co} an Kobalt, 4 : 1 bis 1: 4 beträgt.
66. Verfahren gemäß einer der Ausführungsformen 58 bis 65, dadurch gekennzeichnet, dass das Katalysatorsystem die wenigstens eine Kobaltquelle und den wenigstens einen co-Katalysator C in solchen Mengen enthält, dass das Verhältnis M_{C} : M_{Co}, gebildet aus der im Katalysatorsystem insgesamt enthaltenen molaren Menge M_{C} an co-Katalysatoren C und der im Katalysatorsystem insgesamt enthaltenen molaren Menge M_{Co} an Kobalt, 3 : 1 bis 1: 3 beträgt.
67. Verfahren gemäß einer der Ausführungsformen 58 bis 66, dadurch gekennzeichnet, dass das Katalysatorsystem die wenigstens eine Kobaltquelle und den wenigstens einen co-Katalysator C in solchen Mengen enthält, dass das Verhältnis M_{C} : M_{Co}, gebildet aus der im Katalysatorsystem insgesamt enthaltenen molaren Menge M_{C} an co-Katalysatoren C und der im Katalysatorsystem insgesamt enthaltenen molaren Menge M_{Co} an Kobalt, 2 : 1 bis 1: 2 beträgt.
68. Verfahren gemäß einer der Ausführungsformen 1 bis 67, dadurch gekennzeichnet, dass das Katalysatorsystem als Kobaltquelle wenigstens ein Salz des Anions Co(CO)₄ und/oder dessen Brönstedsäure HCo(CO)₄ umfasst.
69. Verfahren gemäß einer der Ausführungsformen 1 bis 68, dadurch gekennzeichnet, dass das Katalysatorsystem wenigstens eines der in Tabelle 1 der vorliegenden Schrift aufgeführten Katalysatorsysteme umfasst.
70. Verfahren gemäß einer der Ausführungsformen 1 bis 69, dadurch gekennzeichnet, dass die carbonylierende Umsetzung bei einer Reaktionstemperatur im Bereich von 25 bis 150 °C durchgeführt wird.
71. Verfahren gemäß einer der Ausführungsformen 1 bis 70, dadurch gekennzeichnet, dass die carbonylierende Umsetzung bei einer Reaktionstemperatur im Bereich von 35 bis 120 °C durchgeführt wird.
72. Verfahren gemäß einer der Ausführungsformen 1 bis 71, dadurch gekennzeichnet, dass die carbonylierende Umsetzung bei einer Reaktionstemperatur im Bereich von 50 bis 120 °C durchgeführt wird.
73. Verfahren gemäß einer der Ausführungsformen 1 bis 72, dadurch gekennzeichnet, dass die carbonylierende Umsetzung bei einer Reaktionstemperatur im Bereich von 60 bis 100 °C durchgeführt wird.
74. Verfahren gemäß einer der Ausführungsformen 1 bis 73, dadurch gekennzeichnet, dass die carbonylierende Umsetzung bei einer Reaktionstemperatur im Bereich von 70 bis 90 °C durchgeführt wird.
75. Verfahren gemäß einer der Ausführungsformen 1 bis 74, dadurch gekennzeichnet, dass die carbonylierende Umsetzung bei einem Arbeitsdruck von 1,0133·10⁵ Pa bis 2,5 · 10⁷ Pa durchgeführt wird.
76. Verfahren gemäß einer der Ausführungsformen 1 bis 75, dadurch gekennzeichnet, dass die carbonylierende Umsetzung bei einem Arbeitsdruck von 2 · 105 Pa bis 2 · 10⁷ Pa durchgeführt wird.
77. Verfahren gemäß einer der Ausführungsformen 1 bis 76, dadurch gekennzeichnet, dass die carbonylierende Umsetzung bei einem Arbeitsdruck von 5 · 10⁵ Pa bis 1,5 · 10⁷ Pa durchgeführt wird.
78. Verfahren gemäß einer der Ausführungsformen 1 bis 77, dadurch gekennzeichnet, dass die carbonylierende Umsetzung bei einem Arbeitsdruck von 1 · 10⁶ Pa bis 1 · 10⁷ Pa durchgeführt wird.
79. Verfahren gemäß einer der Ausführungsformen 1 bis 78, dadurch gekennzeichnet, dass die carbonylierende Umsetzung bei einem Arbeitsdruck von 2 · 10⁶ Pa bis 9 · 10⁶ Pa durchgeführt wird.
80. Verfahren gemäß einer der Ausführungsformen 1 bis 79, dadurch gekennzeichnet, dass die carbonylierende Umsetzung bei einem Arbeitsdruck von 4 · 10⁶ Pa bis 8 · 10⁶ Pa durchgeführt wird.
81. Verfahren gemäß einer der Ausführungsformen 1 bis 80, dadurch gekennzeichnet, dass das für die carbonylierende Umsetzung verwendete Kohlenmonoxid, bezogen auf sein Gesamtvolumen, ≤ 1 Vol.-% an von CO verschiedenen Bestandteilen enthält.
82. Verfahren gemäß einer der Ausführungsformen 1 bis 80, dadurch gekennzeichnet, dass das für die carbonylierende Umsetzung verwendete Kohlenmonoxid, bezogen auf sein Gesamtvolumen, ≤ 0,1 Vol.-% an von CO verschiedenen Bestandteilen enthält.
83. Verfahren gemäß einer der Ausführungsformen 1 bis 80, dadurch gekennzeichnet, dass das für die carbonylierende Umsetzung verwendete Kohlenmonoxid, bezogen auf sein Gesamtvolumen, ≤ 0,01 Vol.-% an von CO verschiedenen Bestandteilen enthält.
84. Verfahren gemäß einer der Ausführungsformen 1 bis 80, dadurch gekennzeichnet, dass das für die carbonylierende Umsetzung verwendete Kohlenmonoxid, bezogen auf sein Gesamtvolumen, ≤ 0,001 Vol.-% an von CO verschiedenen Bestandteilen enthält.
85. Verfahren gemäß einer der Ausführungsformen 1 bis 80, dadurch gekennzeichnet, dass das für die carbonylierende Umsetzung verwendete Kohlenmonoxid in einem Gemisch aus Kohlenmonoxid und wenigstens einem Inertgas enthalten ist.
86. Verfahren gemäß Ausführungsform 85, dadurch gekennzeichnet, dass das Inertgase molekularer Stickstoff und/oder ein Edelgas ist.
87. Verfahren gemäß einer der Ausführungsformen 1 bis 86, dadurch gekennzeichnet, dass die carbonylierende Umsetzung bei einem Partialdruck des CO von 1,0133·10⁵ Pa bis 2,5 · 10⁷ Pa durchgeführt wird.
88. Verfahren gemäß einer der Ausführungsformen 1 bis 87, dadurch gekennzeichnet, dass die carbonylierende Umsetzung bei einem Partialdruck des CO von 2 · 10⁵ Pa bis 2 · 10⁷ Pa durchgeführt wird.
89. Verfahren gemäß einer der Ausführungsformen 1 bis 88, dadurch gekennzeichnet, dass die carbonylierende Umsetzung bei einem Partialdruck des CO von 5 · 10⁵ Pa bis 1,5 · 10⁷ Pa durchgeführt wird.
90. Verfahren gemäß einer der Ausführungsformen 1 bis 89, dadurch gekennzeichnet, dass die carbonylierende Umsetzung bei einem Partialdruck des CO von 1 · 10⁶ Pa bis 1 · 10⁷ Pa durchgeführt wird.
91. Verfahren gemäß einer der Ausführungsformen 1 bis 90, dadurch gekennzeichnet, dass die carbonylierende Umsetzung bei einem Partialdruck des CO von 2 · 10⁶ Pa bis 9 · 106 Pa durchgeführt wird.
92. Verfahren gemäß einer der Ausführungsformen 1 bis 91, dadurch gekennzeichnet, dass die carbonylierende Umsetzung bei einem Partialdruck des CO von 4 · 10⁶ Pa bis 8 · 106 Pa durchgeführt wird.
93. Verfahren gemäß einer der Ausführungsformen 1 bis 92, dadurch gekennzeichnet, dass der Umsatz an Ethylenoxid bei der carbonylierenden Umsetzung ≥ 90 mol-% beträgt.
94. Verfahren gemäß einer der Ausführungsformen 1 bis 92, dadurch gekennzeichnet, dass der Umsatz an Ethylenoxid bei der carbonylierenden Umsetzung ≥ 95 mol-% beträgt.
95. Verfahren gemäß einer der Ausführungsformen 1 bis 92, dadurch gekennzeichnet, dass der Umsatz an Ethylenoxid bei der carbonylierenden Umsetzung ≥ 98 mol-% beträgt.
96. Verfahren gemäß einer der Ausführungsformen 1 bis 92, dadurch gekennzeichnet, dass der Umsatz an Ethylenoxid bei der carbonylierenden Umsetzung ≥ 99 mol-% beträgt.
97. Verfahren gemäß einer der Ausführungsformen 1 bis 92, dadurch gekennzeichnet, dass der Umsatz an Ethylenoxid bei der carbonylierenden Umsetzung ≥ 99,9 mol-% beträgt.
98. Verfahren gemäß einer der Ausführungsformen 1 bis 97, dadurch gekennzeichnet, dass die carbonylierende Umsetzung unter Ausschluss von molekularem Sauerstoff durchgeführt wird.
99. Verfahren gemäß einer der Ausführungsformen 1 bis 98, dadurch gekennzeichnet, dass der pH-Wert der wässrigen Fällflüssigkeit, bezogen auf eine Temperatur von 25 °C und einen Druck von 1,0133·10⁵ Pa, ≤ 7,5 beträgt.
100. Verfahren gemäß einer der Ausführungsformen 1 bis 98, dadurch gekennzeichnet, dass der pH-Wert der wässrigen Fällflüssigkeit, bezogen auf eine Temperatur von 25 °C und einen Druck von 1,0133·10⁵ Pa, ≤ 7,0 beträgt.
101. Verfahren gemäß einer der Ausführungsformen 1 bis 98, dadurch gekennzeichnet, dass der pH-Wert der wässrigen Fällflüssigkeit, bezogen auf eine Temperatur von 25 °C und einen Druck von 1,0133·10⁵ Pa, ≤ 6 beträgt.
102. Verfahren gemäß einer der Ausführungsformen 1 bis 98, dadurch gekennzeichnet, dass der pH-Wert der wässrigen Fällflüssigkeit, bezogen auf eine Temperatur von 25 °C und einen Druck von 1,0133·10⁵ Pa, ≤ 5 beträgt.
103. Verfahren gemäß einer der Ausführungsformen 1 bis 98, dadurch gekennzeichnet, dass der pH-Wert der wässrigen Fällflüssigkeit, bezogen auf eine Temperatur von 25 °C und einen Druck von 1,0133·105 Pa, ≤ 4 beträgt.
104. Verfahren gemäß einer der Ausführungsformen 1 bis 103, dadurch gekennzeichnet, dass der pH-Wert der wässrigen Fällflüssigkeit, bezogen auf eine Temperatur von 25 °C und einen Druck von 1,0133·105 Pa, ≥ 0 beträgt.
105. Verfahren gemäß einer der Ausführungsformen 1 bis 104, dadurch gekennzeichnet, dass der pH-Wert der wässrigen Fällflüssigkeit, bezogen auf eine Temperatur von 25 °C und einen Druck von 1,0133·10⁵ Pa, ≥ 1 beträgt.
106. Verfahren gemäß einer der Ausführungsformen 1 bis 105, dadurch gekennzeichnet, dass der pH-Wert der wässrigen Fällflüssigkeit, bezogen auf eine Temperatur von 25 °C und einen Druck von 1,0133·10⁵ Pa, ≥ 2 beträgt.
107. Verfahren gemäß einer der Ausführungsformen 1 bis 106, dadurch gekennzeichnet, dass der pH-Wert des Produktgemischs A oder einer Teilmenge des Produktgemischs A nach erfolgtem Zusatz einer wässrigen Fällflüssigkeit, bezogen auf eine Temperatur von 25 °C und einen Druck von 1,0133·10⁵ Pa, ≤ 7,5 beträgt.
108. Verfahren gemäß einer der Ausführungsformen 1 bis 106, dadurch gekennzeichnet, dass der pH-Wert des Produktgemischs A oder einer Teilmenge des Produktgemischs A nach erfolgtem Zusatz einer wässrigen Fällflüssigkeit, bezogen auf eine Temperatur von 25 °C und einen Druck von 1,0133·10⁵ Pa, ≤ 7,0 beträgt.
109. Verfahren gemäß einer der Ausführungsformen 1 bis 106, dadurch gekennzeichnet, dass der pH-Wert des Produktgemischs A oder einer Teilmenge des Produktgemischs A nach erfolgtem Zusatz einer wässrigen Fällflüssigkeit, bezogen auf eine Temperatur von 25 °C und einen Druck von 1,0133·10⁵ Pa, ≤ 6 beträgt.
110. Verfahren gemäß einer der Ausführungsformen 1 bis 106, dadurch gekennzeichnet, dass der pH-Wert des Produktgemischs A oder einer Teilmenge des Produktgemischs A nach erfolgtem Zusatz einer wässrigen Fällflüssigkeit, bezogen auf eine Temperatur von 25 °C und einen Druck von 1,0133·10⁵ Pa, ≤ 5 beträgt.
111. Verfahren gemäß einer der Ausführungsformen 1 bis 106, dadurch gekennzeichnet, dass der pH-Wert des Produktgemischs A oder einer Teilmenge des Produktgemischs A nach erfolgtem Zusatz einer wässrigen Fällflüssigkeit, bezogen auf eine Temperatur von 25 °C und einen Druck von 1,0133·105 Pa, ≤ 4 beträgt.
112. Verfahren gemäß einer der Ausführungsformen 1 bis 111, dadurch gekennzeichnet, dass der pH-Wert des Produktgemischs A oder einer Teilmenge des Produktgemischs A nach erfolgtem Zusatz einer wässrigen Fällflüssigkeit, bezogen auf eine Temperatur von 25 °C und einen Druck von 1,0133·10⁵ Pa, ≥ 0 beträgt.
113. Verfahren gemäß einer der Ausführungsformen 1 bis 112, dadurch gekennzeichnet, dass der pH-Wert des Produktgemischs A oder einer Teilmenge des Produktgemischs A nach erfolgtem Zusatz einer wässrigen Fällflüssigkeit, bezogen auf eine Temperatur von 25 °C und einen Druck von 1,0133·10⁵ Pa, ≥ 1 beträgt.
114. Verfahren gemäß einer der Ausführungsformen 1 bis 113, dadurch gekennzeichnet, dass der pH-Wert des Produktgemischs A oder einer Teilmenge des Produktgemischs A nach erfolgtem Zusatz einer wässrigen Fällflüssigkeit, bezogen auf eine Temperatur von 25 °C und einen Druck von 1,0133·105 Pa, ≥ 2 beträgt.
115. Verfahren gemäß einer der Ausführungsformen 1 bis 114, dadurch gekennzeichnet, dass die wässrige Fällflüssigkeit die wässrige Lösung einer anorganischen Säure und/oder einer organischen Säure ist.
116. Verfahren gemäß Ausführungsform 115, dadurch gekennzeichnet dass die wässrige Fällflüssigkeit wenigstens eine wässrige Lösung aus der Gruppe bestehend aus wässriger Schwefelsäurelösung, wässriger Kohlensäurelösung, wässriger Salzsäurelösung, wässriger Phosphorsäurelösung, wässriger Acrylsäurelösung, wässriger Oxalsäurelösung, wässriger Ameisensäurelösung, wässriger Essigsäurelösung, wässriger Propionsäurelösung, wässriger Fumarsäurelösung, wässriger Maleinsäurelösung und wässriger Methansulfonsäurelösung ist.
117. Verfahren gemäß einer der Ausführungsformen 1 bis 114, dadurch gekennzeichnet, dass die wässrige Fällflüssigkeit eine wässrige Essigsäurelösung ist.
118. Verfahren gemäß einer der Ausführungsformen 1 bis 117, dadurch gekennzeichnet, dass der Wassergehalt der wässrigen Fällflüssigkeit, bezogen auf ihr Gewicht, wenigstens 10 Gew.-% oder wenigstens 20 Gew.-% beträgt.
119. Verfahren gemäß einer der Ausführungsformen 1 bis 117, dadurch gekennzeichnet, dass der Wassergehalt der wässrigen Fällflüssigkeit, bezogen auf ihr Gewicht, wenigstens 30 Gew.-% beträgt.
120. Verfahren gemäß einer der Ausführungsformen 1 bis 117, dadurch gekennzeichnet, dass der Wassergehalt der wässrigen Fällflüssigkeit, bezogen auf ihr Gewicht, wenigstens 40 Gew.-% beträgt.
121. Verfahren gemäß einer der Ausführungsformen 1 bis 117, dadurch gekennzeichnet, dass der Wassergehalt der wässrigen Fällflüssigkeit, bezogen auf ihr Gewicht, wenigstens 50 Gew.-% beträgt.
122. Verfahren gemäß einer der Ausführungsformen 1 bis 117, dadurch gekennzeichnet, dass der Wassergehalt der wässrigen Fällflüssigkeit, bezogen auf ihr Gewicht, wenigstens 60 Gew.-% beträgt.
123. Verfahren gemäß einer der Ausführungsformen 1 bis 117, dadurch gekennzeichnet, dass der Wassergehalt der wässrigen Fällflüssigkeit, bezogen auf ihr Gewicht, wenigstens 70 Gew.-% beträgt.
124. Verfahren gemäß einer der Ausführungsformen 1 bis 117, dadurch gekennzeichnet, dass der Wassergehalt der wässrigen Fällflüssigkeit, bezogen auf ihr Gewicht, wenigstens 80 Gew.-% beträgt.
125. Verfahren gemäß einer der Ausführungsformen 1 bis 117, dadurch gekennzeichnet, dass der Wassergehalt der wässrigen Fällflüssigkeit, bezogen auf ihr Gewicht, wenigstens 90 Gew.-% beträgt.
126. Verfahren gemäß einer der Ausführungsformen 1 bis 117, dadurch gekennzeichnet, dass der Wassergehalt der wässrigen Fällflüssigkeit, bezogen auf ihr Gewicht, wenigstens 95 Gew.-% beträgt.
127. Verfahren gemäß einer der Ausführungsformen 1 bis 117, dadurch gekennzeichnet, dass der Wassergehalt der wässrigen Fällflüssigkeit, bezogen auf ihr Gewicht, wenigstens 97 Gew.-% beträgt.
128. Verfahren gemäß einer der Ausführungsformen 1 bis 117, dadurch gekennzeichnet, dass der Wassergehalt der wässrigen Fällflüssigkeit, bezogen auf ihr Gewicht, wenigstens 99 Gew.-% beträgt.
129. Verfahren gemäß einer der Ausführungsformen 1 bis 128, dadurch gekennzeichnet, dass der Zusatz der wässrigen Fällflüssigkeit im Beisein wenigstens eines Oxidationsmittels (für Co in Oxidationsstufen < +2) vorgenommen wird.
130. Verfahren gemäß Ausführungsform 129, dadurch gekennzeichnet, dass der Zusatz der wässrigen Fällflüssigkeit in Gegenwart von Luft oder in Gegenwart eines von Luft verschiedenen molekularen Sauerstoff enthaltenden Gases vorgenommen wird.
131. Verfahren gemäß einer der Ausführungsformen 1 bis 130, dadurch gekennzeichnet, dass vor, während und/oder nach dem Zusatz der wässrigen Fällflüssigkeit einer Teilmenge des Produktgemischs A, der wässrigen Fällflüssigkeit selbst, der Gesamtmenge des Produktgemischs A und/oder dem entstehenden Gemisch aus einer Teilmenge bzw. der Gesamtmenge des Produktgemischs A und der wässrigen Fällflüssigkeit ein oder mehr als ein Oxidationsmittel zugesetzt wird.
132. Verfahren gemäß Ausführungsform 131, dadurch gekennzeichnet dass ein oder mehr als ein Oxidationsmittel aus der Gruppe bestehend aus Ozon, Wasserstoffperoxid, molekularem Sauerstoff, Perchlorat, Perchlorsäure und Salpetersäure zugesetzt wird.
133. Verfahren gemäß einer der Ausführungsformen 1 bis 132, dadurch gekennzeichnet, dass die wässrige Fällflüssigkeit molekularen Sauerstoff gelöst enthält.
134. Verfahren gemäß einer der Ausführungsformen 1 bis 133, dadurch gekennzeichnet, dass die wässrige Fällflüssigkeit mit molekularem Sauerstoff gesättigt ist.
135. Verfahren gemäß einer der Ausführungsformen 1 bis 134, dadurch gekennzeichnet, dass dem Produktgemisch A und/oder einer Teilmenge des Produktgemischs A zusätzlich zu der wässrigen Fällflüssigkeit ein molekularen Sauerstoff enthaltendes Gas zugeführt wird.
136. Verfahren gemäß Ausführungsform 135, dadurch gekennzeichnet, dass das molekularen Sauerstoff enthaltende Gas Luft ist oder Luft enthält.
137. Verfahren gemäß Ausführungsform 135 oder 136, dadurch gekennzeichnet, dass die Zufuhr des molekularen Sauerstoff enthaltenden Gases in das aus dem Produktgemisch A oder aus einer Teilmenge des Produktgemischs A durch den Zusatz der wässrigen Fällflüssigkeit entstehende wässrige Gemisch hinein erfolgt.
138. Verfahren gemäß Ausführungsform 137, dadurch gekennzeichnet, dass die Zufuhr des molekularen Sauerstoff enthaltenden Gases bei einer Temperatur des wässrigen Gemischs von 10 bis 95 °C, oder von 20 bis 95 °C, oder von 30 °C bis 95 °C erfolgt.
139. Verfahren gemäß Ausführungsform 137 oder 138, dadurch gekennzeichnet, dass die Zufuhr des molekularen Sauerstoff enthaltenden Gases bei einer Temperatur des wässrigen Gemischs von 40 °C bis 90 °C erfolgt.
140. Verfahren gemäß einer der Ausführungsformen 137 oder 139, dadurch gekennzeichnet, dass die Zufuhr des molekularen Sauerstoff enthaltenden Gases bei einer Temperatur des wässrigen Gemischs von 50 °C bis 80 °C oder von 50°C bis 60 °C erfolgt.
141. Verfahren gemäß einer der Ausführungsformen 137 bis 140, dadurch gekennzeichnet, dass die Temperatur des wässrigen Gemischs während und/oder nach der Zufuhr des molekularen Sauerstoff enthaltenden Gases verringert wird.
142. Verfahren gemäß einer der Ausführungsformen 1 bis 141, dadurch gekennzeichnet, dass der pH-Wert der wässrigen Waschflüssigkeit, bezogen auf eine Temperatur von 25 °C und einen Druck von 1,0133·10⁵ Pa, ≤ 7,5 beträgt.
143. Verfahren gemäß einer der Ausführungsformen 1 bis 141, dadurch gekennzeichnet, dass der pH-Wert der wässrigen Waschflüssigkeit, bezogen auf eine Temperatur von 25 °C und einen Druck von 1,0133·10⁵ Pa, ≤ 7,0 beträgt.
144. Verfahren gemäß einer der Ausführungsformen 1 bis 141, dadurch gekennzeichnet, dass der pH-Wert der wässrigen Waschflüssigkeit, bezogen auf eine Temperatur von 25 °C und einen Druck von 1,0133·10⁵ Pa, ≤ 6 beträgt.
145. Verfahren gemäß einer der Ausführungsformen 1 bis 141, dadurch gekennzeichnet, dass der pH-Wert der wässrigen Waschflüssigkeit, bezogen auf eine Temperatur von 25 °C und einen Druck von 1,0133·10⁵ Pa, ≤ 5 beträgt.
146. Verfahren gemäß einer der Ausführungsformen 1 bis 141, dadurch gekennzeichnet, dass der pH-Wert der wässrigen Waschflüssigkeit, bezogen auf eine Temperatur von 25 °C und einen Druck von 1,0133·10⁵ Pa, ≤ 4 beträgt.
147. Verfahren gemäß einer der Ausführungsformen 1 bis 146, dadurch gekennzeichnet, dass der pH-Wert der wässrigen Waschflüssigkeit, bezogen auf eine Temperatur von 25 °C und einen Druck von 1,0133·10⁵ Pa, ≥ 0 beträgt.
148. Verfahren gemäß einer der Ausführungsformen 1 bis 147, dadurch gekennzeichnet, dass der pH-Wert der wässrigen Waschflüssigkeit, bezogen auf eine Temperatur von 25 °C und einen Druck von 1,0133·10⁵ Pa, ≥ 1 beträgt.
149. Verfahren gemäß einer der Ausführungsformen 1 bis 148, dadurch gekennzeichnet, dass der pH-Wert der wässrigen Waschflüssigkeit, bezogen auf eine Temperatur von 25 °C und einen Druck von 1,0133·105 Pa, ≥ 2 beträgt.
150. Verfahren gemäß einer der Ausführungsformen 1 bis 149, dadurch gekennzeichnet, dass die wässrige Waschflüssigkeit die wässrige Lösung einer anorganischen Säure und/oder einer organischen Säure ist.
151. Verfahren gemäß Ausführungsform 150, dadurch gekennzeichnet dass die wässrige Waschflüssigkeit wenigstens eine wässrige Lösung aus der Gruppe bestehend aus wässriger Schwefelsäurelösung, wässriger Kohlensäurelösung, wässriger Salzsäurelösung, wässriger Phosphorsäurelösung, wässriger Acrylsäurelösung, wässriger Oxalsäurelösung, wässriger Ameisensäurelösung, wässriger Essigsäurelösung, wässriger Propions-äurelösung, wässriger Fumarsäurelösung, wässriger Maleinsäurelösung und wässriger Methansulfonsäurelösung ist.
152. Verfahren gemäß einer der Ausführungsformen 1 bis 151, dadurch gekennzeichnet, dass die wässrige Waschflüssigkeit eine wässrige Essigsäurelösung ist.
153. Verfahren gemäß einer der Ausführungsformen 1 bis 152, dadurch gekennzeichnet, dass der Wassergehalt der wässrigen Waschflüssigkeit, bezogen auf ihr Gewicht, wenigstens 10 Gew.-% oder wenigstens 20 Gew.-% beträgt.
154. Verfahren gemäß einer der Ausführungsformen 1 bis 152, dadurch gekennzeichnet, dass der Wassergehalt der wässrigen Waschflüssigkeit, bezogen auf ihr Gewicht, wenigstens 30 Gew.-% beträgt.
155. Verfahren gemäß einer der Ausführungsformen 1 bis 152, dadurch gekennzeichnet, dass der Wassergehalt der wässrigen Waschflüssigkeit, bezogen auf ihr Gewicht, wenigstens 40 Gew.-% beträgt.
156. Verfahren gemäß einer der Ausführungsformen 1 bis 152, dadurch gekennzeichnet, dass der Wassergehalt der wässrigen Waschflüssigkeit, bezogen auf ihr Gewicht, wenigstens 50 Gew.-% beträgt.
157. Verfahren gemäß einer der Ausführungsformen 1 bis 152, dadurch gekennzeichnet, dass der Wassergehalt der wässrigen Waschflüssigkeit, bezogen auf ihr Gewicht, wenigstens 60 Gew.-% beträgt.
158. Verfahren gemäß einer der Ausführungsformen 1 bis 152, dadurch gekennzeichnet, dass der Wassergehalt der wässrigen Waschflüssigkeit, bezogen auf ihr Gewicht, wenigstens 70 Gew.-% beträgt.
159. Verfahren gemäß einer der Ausführungsformen 1 bis 152, dadurch gekennzeichnet, dass der Wassergehalt der wässrigen Waschflüssigkeit, bezogen auf ihr Gewicht, wenigstens 80 Gew.-% beträgt.
160. Verfahren gemäß einer der Ausführungsformen 1 bis 152, dadurch gekennzeichnet, dass der Wassergehalt der wässrigen Waschflüssigkeit, bezogen auf ihr Gewicht, wenigstens 90 Gew.-% beträgt.
161. Verfahren gemäß einer der Ausführungsformen 1 bis 152, dadurch gekennzeichnet, dass der Wassergehalt der wässrigen Waschflüssigkeit, bezogen auf ihr Gewicht, wenigstens 95 Gew.-% beträgt.
162. Verfahren gemäß einer der Ausführungsformen 1 bis 152, dadurch gekennzeichnet, dass der Wassergehalt der wässrigen Waschflüssigkeit, bezogen auf ihr Gewicht, wenigstens 97 Gew.-% beträgt.
163. Verfahren gemäß einer der Ausführungsformen 1 bis 152, dadurch gekennzeichnet, dass der Wassergehalt der wässrigen Waschflüssigkeit, bezogen auf ihr Gewicht, wenigstens 99 Gew.-% beträgt.
164. Verfahren gemäß einer der Ausführungsformen 1 bis 163, dadurch gekennzeichnet, dass das Waschen von vom Produktgemisch A abgetrenntem Poly-3-hydroxypropionat im Beisein wenigstens eines Oxidationsmittels (für Co in Oxidationsstufen < +2) erfolgt.
165. Verfahren gemäß einer der Ausführungsformen 1 bis 164, dadurch gekennzeichnet, dass das Waschen des vom Produktgemisch A abgetrenntem Poly-3-hydroxypropionats in Gegenwart von Luft und/oder einem von Luft verschiedenen molekularen Sauerstoff enthaltenden Gas vorgenommen wird.
166. Verfahren gemäß Ausführungsform 164, dadurch gekennzeichnet, dass das wenigstens eine Oxidationsmittel ein Oxidationsmittel aus der Gruppe bestehend aus Ozon, Wasserstoffperoxid, molekularem Sauerstoff, Perchlorat, Perchlorsäure und Salpetersäure ist.
167. Verfahren gemäß einer der Ausführungsformen 1 bis 166, dadurch gekennzeichnet, dass die wässrige Waschflüssigkeit molekularen Sauerstoff oder Luft gelöst enthält.
168. Verfahren gemäß einer der Ausführungsformen 1 bis 167, dadurch gekennzeichnet, dass die wässrige Waschflüssigkeit mit molekularem Sauerstoff oder mit Luft gesättigt ist.
169. Verfahren gemäß einer der Ausführungsformen 1 bis 168, dadurch gekennzeichnet, dass das Waschen von vom Produktgemisch A abgetrenntem Poly-3-hydroxypropionat so erfolgt, dass wässrige Waschflüssigkeit durch das vom Produktgasgemisch A abgetrennte Poly-3-hydroxypropionat hindurchgesaugt und/oder hindurchgedrückt wird.
170. Verfahren gemäß Ausführungsform 169, dadurch gekennzeichnet, dass die Temperatur der wässrigen Waschflüssigkeit 10 °C bis 95 °C beträgt.
171. Verfahren gemäß Ausführungsform 169 oder 170, dadurch gekennzeichnet, dass die Temperatur der wässrigen Waschflüssigkeit 20 °C bis 90 °C beträgt.
172. Verfahren gemäß einer der Ausführungsform 169 bis171, dadurch gekennzeichnet, dass die Temperatur der wässrigen Waschflüssigkeit 30 °C bis 80 °C beträgt.
173. Verfahren gemäß einer der Ausführungsformen 169 bis 172, dadurch gekennzeichnet, dass die Temperatur der wässrigen Waschflüssigkeit 40 °C bis 70 °C beträgt.
174. Verfahren gemäß einer der Ausführungsformen 169 oder 173, dadurch gekennzeichnet, dass die Temperatur der wässrigen Waschflüssigkeit 50 °C bis 60 °C beträgt.
175. Verfahren gemäß einer der Ausführungsformen 1 bis 168, dadurch gekennzeichnet, dass das Waschen von vom Produktgemisch A abgetrenntem Poly-3-hydroxypropionat mit der wässrigen Waschflüssigkeit so erfolgt, dass das vom Produktgemisch A abgetrennte Poly-3-hydroxypropionat in der wässrigen Waschflüssigkeit suspendiert und von der dabei resultierenden wässrigen Suspension nachfolgend durch die Anwendung wenigstens einer mechanischen Trennoperation wieder abgetrennt wird.
176. Verfahren gemäß Ausführungsform 175, dadurch gekennzeichnet, dass die wässrige Suspension des Poly-3-hydroxypropionats von einem molekularen Sauerstoff enthaltenden Gas durchströmt wird.
177. Verfahren gemäß Ausführungsform 176, dadurch gekennzeichnet, dass das molekularen Sauerstoff enthaltende Gas Luft, molekularer Sauerstoff und/oder ein Gemisch aus molekularem Sauerstoff und wenigstens einem Inertgas ist.
178. Verfahren gemäß einer der Ausführungsformen 175 bis 177, dadurch gekennzeichnet, dass die wässrige Suspension des Poly-3-hydroxypropionats bei einer Temperatur von 10 °C bis 95 °C durchmischt wird.
179. Verfahren gemäß Ausführungsform 178, dadurch gekennzeichnet, dass die wässrige Suspension des Poly-3-hydroxypropionats bei einer Temperatur von 20 °C bis 90 °C durchmischt wird.
180. Verfahren gemäß Ausführungsform 178 oder 179, dadurch gekennzeichnet, dass die wässrige Suspension des Poly-3-hydroxypropionats bei einer Temperatur von 30 °C bis 80 °C durchmischt wird.
181. Verfahren gemäß einer der Ausführungsformen 178 bis 180, dadurch gekennzeichnet, dass die wässrige Suspension des Poly-3-hydroxypropionats bei einer Temperatur von 40 °C bis 70 °C durchmischt wird.
182. Verfahren gemäß einer der Ausführungsformen 178 bis 181, dadurch gekennzeichnet, dass die wässrige Suspension des Poly-3-hydroxypropionats bei einer Temperatur von 50 °C bis 60 °C durchmischt wird.
183. Verfahren gemäß einer der Ausführungsformen 175 bis 182, dadurch gekennzeichnet, dass die wenigstens eine mechanische Trennoperation eine Filtration und/oder eine Zentrifugation ist.
184. Verfahren gemäß einer der Ausführungsformen 1 bis 183, dadurch gekennzeichnet, dass das (in einer Trennzone A) abgetrennte Poly-3-hydroxypropionat vorab seiner Thermolyse getrocknet wird.
185. Verfahren gemäß Ausführungsform 184, dadurch gekennzeichnet, dass das (in einer Trennzone A) abgetrennte Poly-3-hydroxypropionat vorab seiner Trocknung mit Methanol gewaschen wird.
186. Verfahren gemäß einer der Ausführungsformen 1 bis 185, dadurch gekennzeichnet, dass die Thermolyse des (in einer Trennzone A abgetrennten) Poly-3-Hydroxypropionats aus seiner festen Substanz, oder aus seiner Schmelze, oder aus seiner Lösung in einem Lösungsmittel, oder aus seiner Suspension in einem Dispergiermittel, oder aus seiner Emulsion in einem Dispergiermittel heraus erfolgt.
187. Verfahren gemäß einer der Ausführungsformen 1 bis 185, dadurch gekennzeichnet, dass dem (in einer Trennzone A abgetrennten) Poly-3-hydroxypropionat wenigstens ein die Thermolyse katalysierender Spaltkatalysator zugesetzt wird (wahlweise z.B. in einer Menge von, bezogen auf das Gewicht der Masse des Poly-3-hydroxypropionats, von 0,01 bis 15 Gew.-%, oder von 0,05 bis 10 Gew.-%, oder von 0,1 bis 5 Gew.-%, oder von 0,4 bis 4 Gew.-%, oder von 1,5 bis 3,5 Gew.-%).
188. Verfahren gemäß Ausführungsform 186, dadurch gekennzeichnet, dass der festen Substanz, oder der Schmelze, oder der Lösung, oder der Suspension, oder der Emulsion wenigstens ein die Thermolyse katalysierender Spaltkatalysator zugesetzt wird.
189. Verfahren gemäß Ausführungsform 187 oder 188, dadurch gekennzeichnet, dass der wenigstens eine Spaltkatalysator eine molekulare organische Wirkverbindung ist, die neben Kohlenstoff und Wasserstoff als von diesen verschiedene Heteroatome wenigstens ein Stickstoffatom sowie wahlweise wenigstens ein oder mehr als ein Sauerstoffatom mit der Maßgabe kovalent gebunden aufweisen, dass
   - die organische Wirkverbindung kein über Stickstoff und Sauerstoff hinausgehendes, von Kohlenstoff und Wasserstoff verschiedenes, Heteroatom aufweist, und
   - wenigstens ein Stickstoffatom ein tertiäres Stickstoffatom ist.
190. Verfahren gemäß Ausführungsform 189, dadurch gekennzeichnet, dass die wenigstens eine molekulare organische Wirkverbindung mehr als ein tertiäres Stickstoffatom aufweist.
191. Verfahren gemäß Ausführungsform 190, dadurch gekennzeichnet, dass die wenigstens eine molekulare organische Wirkverbindung wenigstens zwei oder wenigstens drei tertiäre Stickstoffatome aufweist.
192. Verfahren gemäß einer der Ausführungsformen 189 bis 191, dadurch gekennzeichnet, dass die wenigstens eine molekulare organische Wirkverbindung wenigstens ein tertiäres Stickstoffatom aufweist, das zu drei voneinander verschiedenen Kohlenstoffatomen eine kovalente Bindung aufweist
193. Verfahren gemäß einer der Ausführungsformen 189 bis 192, dadurch gekennzeichnet, dass die wenigstens eine molekulare organische Wirkverbindung wenigstens zwei tertiäre Stickstoffatome oder wenigstens drei tertiäre Stickstoffatome aufweist, die jeweils zu drei voneinander verschiedenen Kohlenstoffatomen eine kovalente Bindung aufweisen.
194. Verfahren gemäß einer der Ausführungsformen 189 bis 192, dadurch gekennzeichnet, dass wenigstens ein tertiäres Stickstoffatom ein iminisches Stickstoffatom ist.
195. Verfahren gemäß einer der Ausführungsformen 189 bis 194, dadurch gekennzeichnet, dass die wenigstens eine molekulare organische Wirkverbindung kein Stickstoffatom aufweist, an das ein oder mehr als ein Wasserstoffatom kovalent gebunden ist und/oder kein Sauerstoffatom aufweist, an das ein Wasserstoffatom kovalent gebunden ist.
196. Verfahren gemäß einer der Ausführungsformen 189 bis 195, dadurch gekennzeichnet, dass das Molgewicht der wenigstens einen molekularen organischen Wirkverbindung ≥ 59,1 g/mol und ≤ 600 g/mol beträgt.
197. Verfahren gemäß einer der Ausführungsformen 189 bis 196, dadurch gekennzeichnet, dass das Molgewicht der wenigstens einen molekularen organischen Wirkverbindung ≥ 75 g/mol und ≤ 500 g/mol beträgt.
198. Verfahren gemäß einer der Ausführungsformen 189 bis 197, dadurch gekennzeichnet, dass das Molgewicht der wenigstens einen molekularen organischen Wirkverbindung ≥ 100 g/mol und ≤ 400 g/mol beträgt.
199. Verfahren gemäß einer der Ausführungsformen 189 bis 198, dadurch gekennzeichnet, dass das Molgewicht der wenigstens einen molekularen organischen Wirkverbindung ≥ 125 g/mol und ≤ 300 g/mol beträgt.
200. Verfahren gemäß einer der Ausführungsformen 189 bis 199, dadurch gekennzeichnet, dass das Molgewicht der wenigstens einen molekularen organischen Wirkverbindung ≥ 150 g/mol und ≤ 250 g/mol beträgt.
201. Verfahren gemäß einer der Ausführungsformen 189 bis 200, dadurch gekennzeichnet, dass der auf einen Druck von 1,0133·10⁵ Pa bezogene Siedepunkt der wenigstens einen molekularen organischen Wirkverbindung ≥ 180 °C beträgt.
202. Verfahren gemäß einer der Ausführungsformen 189 bis 200, dadurch gekennzeichnet, dass der auf einen Druck von 1,0133·10⁵ Pa bezogene Siedepunkt der wenigstens einen molekularen organischen Wirkverbindung ≥ 185 °C beträgt.
203. Verfahren gemäß einer der Ausführungsformen 189 bis 200, dadurch gekennzeichnet, dass der auf einen Druck von 1,0133·10⁵ Pa bezogene Siedepunkt der wenigstens einen molekularen organischen Wirkverbindung ≥ 190 °C beträgt.
204. Verfahren gemäß einer der Ausführungsformen 189 bis 203, dadurch gekennzeichnet, dass der auf einen Druck von 1,0133·10⁵ Pa bezogene Siedepunkt der wenigstens einen molekularen organischen Wirkverbindung ≤ 350 °C beträgt.
205. Verfahren gemäß einer der Ausführungsformen 189 bis 204, dadurch gekennzeichnet, dass der auf einen Druck von 1,0133·10⁵ Pa bezogene Siedepunkt der wenigstens einen molekularen organischen Wirkverbindung ≤ 330 °C beträgt.
206. Verfahren gemäß einer der Ausführungsformen 189 bis 205, dadurch gekennzeichnet, dass der auf einen Druck von 1,0133·10⁵ Pa bezogene Siedepunkt der wenigstens einen molekularen organischen Wirkverbindung ≤ 310 °C beträgt.
207. Verfahren gemäß einer der Ausführungsformen 189 bis 206, dadurch gekennzeichnet, dass der auf einen Druck von 1,0133·10⁵ Pa bezogene Siedepunkt der wenigstens einen molekularen organischen Wirkverbindung ≤ 290 °C beträgt.
208. Verfahren gemäß einer der Ausführungsformen 189 bis 207, dadurch gekennzeichnet, dass der auf einen Druck von 1,0133·10⁵ Pa bezogene Siedepunkt der wenigstens einen molekularen organischen Wirkverbindung ≤ 270 °C beträgt.
209. Verfahren gemäß einer der Ausführungsformen 189 bis 208, dadurch gekennzeichnet, dass der auf einen Druck von 1,0133·10⁵ Pa bezogene Siedepunkt der wenigstens einen molekularen organischen Wirkverbindung ≤ 250 °C beträgt.
210. Verfahren gemäß einer der Ausführungsformen 189 bis 209, dadurch gekennzeichnet, dass der auf einen Druck von 1,0133·10⁵ Pa bezogene Siedepunkt der wenigstens einen molekularen organischen Wirkverbindung ≤ 230 °C beträgt.
211. Verfahren gemäß einer der Ausführungsformen 189 bis 210, dadurch gekennzeichnet, dass der auf einen Druck von 1,0133·10⁵ Pa bezogene Siedepunkt der wenigstens einen molekularen organischen Wirkverbindung ≤ 200 °C beträgt.
212. Verfahren gemäß Ausführungsform 189, dadurch gekennzeichnet, dass die wenigstens eine molekulare organische Wirkverbindung ausgewählt ist aus der Gruppe bestehend aus Trimethylamin, Triethylamin, Tri-n-Hexylamin, Tri-n-Butylamin, N-Ethyl-N,N-diisopropylamin, Pentamethyldiethylentriamin, N,N,N',N'-Tetramethyl-1,6-hexandiamin, dem Bis(2-Dimethylaminoethyl)ether, dem 2,2'-Dimorpholinodiethylether, N,N-Diethylethanolamin, N,N,N',N'-Tetramethyl-1,3-propandiamin, N,N-Dimethylcyclohexylamin, N,N-Dimethyl-1,3-diaminopropan, N-Methylimidazol, 1,2-Dimethylimidazol, 2-Hydroxypyridin, 3-Hydroxypyridin und 4-Hydroxypyridin.
213. Verfahren gemäß Ausführungsform 189, dadurch gekennzeichnet, dass die wenigstens eine molekulare organische Wirkverbindung wenigstens ein Poly-3-hydroxypropionat, das mit der Hydroxylgruppe des 2-Hydroxypyridins, oder des 3-Hydroxypyridins, oder des 4-Hydroxypyridins verethert ist, ist.
214. Verfahren gemäß Ausführungsform 213, dadurch gekennzeichnet, dass die wenigstens eine molekulare organische Wirkverbindung wenigstens ein Poly-3-hydroxypropionat der allgemeinen Strukturen 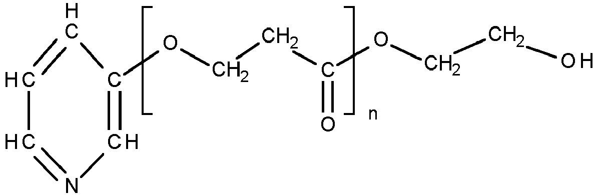 und 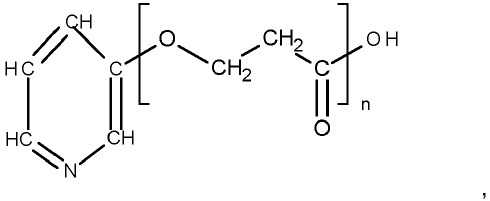 mit n = eine ganze Zahl ≥ 1 ist.
215. Verfahren gemäß Ausführungsform 214, dadurch gekennzeichnet, dass n ≤ 250, oder ≤ 200, oder ≤ 150 ist.
216. Verfahren gemäß einer der Ausführungsformen 189 bis 215, dadurch gekennzeichnet, dass die Thermolyse des (in einer Trennzone A abgetrennten) Poly-3-hydroxypropionats aus dessen fester Substanz, oder aus dessen Schmelze, oder aus einer Lösung desselben in einem Lösungsmittel, oder aus einer Suspension desselben in einem Dispergiermittel, oder aus einer Emulsion desselben in einem Dispergiermittel heraus erfolgt, die, bezogen auf das Gewicht des enthaltenen Poly-3-hydroxypropionats, 0,01 bis 15 Gew.-% der wenigstens einen molekularen organischen Wirkverbindung enthält.
217. Verfahren gemäß einer der Ausführungsformen 189 bis 216, dadurch gekennzeichnet, dass die Thermolyse des Poly-3-hydroxypropionats aus dessen fester Substanz, oder aus dessen Schmelze, oder aus einer Lösung desselben in einem Lösungsmittel, oder aus einer Suspension desselben in einem Dispergiermittel, oder aus einer Emulsion desselben in einem Dispergiermittel heraus erfolgt, die, bezogen auf das Gewicht des enthaltenen Poly-3-hydroxypropionats, 0,05 bis 10 Gew.-% der wenigstens einen molekularen organischen Wirkverbindung enthält.
218. Verfahren gemäß einer der Ausführungsformen 189 bis 217, dadurch gekennzeichnet, dass die Thermolyse des Poly-3-hydroxypropionats aus dessen fester Substanz, oder aus dessen Schmelze, oder aus einer Lösung desselben in einem Lösungsmittel, oder aus einer Suspension desselben in einem Dispergiermittel, oder aus einer Emulsion desselben in einem Dispergiermittel heraus erfolgt, die, bezogen auf das Gewicht des enthaltenen Poly-3-hydroxypropionats, 0,1 bis 5 Gew.-% der wenigstens einen molekularen organischen Wirkverbindung enthält.
219. Verfahren gemäß einer der Ausführungsformen 189 bis 218, dadurch gekennzeichnet, dass die Thermolyse des Poly-3-hydroxypropionats aus dessen fester Substanz, oder aus dessen Schmelze, oder aus einer Lösung desselben in einem Lösungsmittel, oder aus einer Suspension desselben in einem Dispergiermittel, oder aus einer Emulsion desselben in einem Dispergiermittel heraus erfolgt, die, bezogen auf das Gewicht des enthaltenen Poly-3-hydroxypropionats, 0,5 bis 4 Gew.-% der wenigstens einen molekularen organischen Wirkverbindung enthält.
220. Verfahren gemäß einer der Ausführungsformen 189 bis 219, dadurch gekennzeichnet, dass die Thermolyse des Poly-3-hydroxypropionats aus dessen fester Substanz, oder aus dessen Schmelze, oder aus einer Lösung desselben in einem Lösungsmittel, oder aus einer Suspension desselben in einem Dispergiermittel, oder aus einer Emulsion desselben in einem Dispergiermittel heraus erfolgt, die, bezogen auf das Gewicht des enthaltenen Poly-3-hydroxypropionats, 1,5 bis 3,5 Gew.-% der wenigstens einen molekularen organischen Wirkverbindung enthält.
221. Verfahren gemäß einer der Ausführungsformen 1 bis 220, dadurch gekennzeichnet, dass die Thermolyse bei einer Temperatur des Poly-3-hydroxypropionats von 50 °C bis 350 °C, oder von 100 °C bis 300 °C, oder von 150 °C bis 220 °C, oder von 160 °C bis 200 °C durchgeführt wird.
222. Verfahren gemäß Ausführungsform 186 oder gemäß einer der Ausführungsformen 188 bis 221, dadurch gekennzeichnet, dass die Thermolyse bei einer Temperatur der festen Substanz, oder der Schmelze, oder der Lösung, oder der Suspension, oder der Emulsion von 50 °C bis 350 °C durchgeführt wird.
223. Verfahren gemäß Ausführungsform 186 oder gemäß einer der Ausführungsformen 188 bis 222, dadurch gekennzeichnet, dass die Thermolyse bei einer Temperatur der festen Substanz, oder der Schmelze, oder der Lösung, oder der Suspension, oder der Emulsion von 100 °C bis 300 °C durchgeführt wird.
224. Verfahren gemäß Ausführungsform 186 oder gemäß einer der Ausführungsformen 188 bis 223, dadurch gekennzeichnet, dass die Thermolyse bei einer Temperatur der festen Substanz, oder der Schmelze, oder der Lösung, oder der Suspension, oder der Emulsion von 150 °C bis 220 °C durchgeführt wird.
225. Verfahren gemäß Ausführungsform 186 oder gemäß einer der Ausführungsformen 188 bis 224, dadurch gekennzeichnet, dass die Thermolyse bei einer Temperatur der festen Substanz, oder der Schmelze, oder der Lösung, oder der Suspension, oder der Emulsion von 160 °C bis 200 °C durchgeführt wird.
226. Verfahren gemäß einer der Ausführungsformen 1 bis 225, dadurch gekennzeichnet, dass die Thermolyse bei Atmosphärendruck, oberhalb von Atmosphärendruck oder unterhalb von Atmosphärendruck durchgeführt wird.
227. Verfahren gemäß einer der Ausführungsformen 1 bis 226, dadurch gekennzeichnet, dass die Thermolyse bei einem Arbeitsdruck im Bereich von 10² bis 10⁷ Pa durchgeführt wird.
228. Verfahren gemäß einer der Ausführungsformen 1 bis 227, dadurch gekennzeichnet, dass die Thermolyse bei einem Arbeitsdruck im Bereich von 10³ bis 10⁶ Pa durchgeführt wird.
229. Verfahren gemäß einer der Ausführungsformen 1 bis 228, dadurch gekennzeichnet, dass die Thermolyse bei einem Arbeitsdruck im Bereich von 2 · 10³ bis 5 · 10⁵ Pa durchgeführt wird.
230. Verfahren gemäß einer der Ausführungsformen 1 bis 229, dadurch gekennzeichnet, dass die Thermolyse bei einem Arbeitsdruck im Bereich von 5 · 10³ bis 3 · 105 Pa durchgeführt wird.
231. Verfahren gemäß Ausführungsform 186 oder gemäß einer der Ausführungsformen 188 bis 230, dadurch gekennzeichnet, dass während der Thermolyse ein Strippgas über die feste Substanz, oder durch die Schmelze, oder durch die Lösung, oder durch die Suspension, oder durch die Emulsion des Poly-3-hydroxypropionats geführt wird.
232. Verfahren gemäß Ausführungsform 231, dadurch gekennzeichnet, dass das Strippgas molekularen Sauerstoff enthält oder frei von molekularem Sauerstoff ist.
233. Verfahren gemäß einer der Ausführungsformen 1 bis 232, dadurch gekennzeichnet, dass die Thermolyse des Poly-3-hydroxypropionats im Beisein wenigstens eines Polymerisationsinhibitors erfolgt (bezogen auf das Gewicht der Masse des Poly-3-hydroxypropionats z.B. im Beisein von 10 bis 1000 Gew.ppm, oder 50 bis 500 Gew.ppm, oder 150 bis 350 Gew.ppm wenigstens eines Polymerisationsinhibitors).
234. Verfahren gemäß Ausführungsform 186 oder gemäß einer der Ausführungsformen 188 bis 232, dadurch gekennzeichnet, dass die Thermolyse des Poly-3-hydroxypropionats aus dessen fester Substanz, oder aus dessen Schmelze, oder aus dessen Lösung, oder aus dessen Suspension, oder aus dessen Emulsion heraus erfolgt, und diese wenigstens einen Polymerisationsinhibitor zugesetzt enthält.
235. Verfahren gemäß Ausführungsform 233 oder 234, dadurch gekennzeichnet, dass der wenigstens eine Polymerisationsinhibitor wenigstens ein Polymerisationsinhibitor aus der Gruppe bestehend aus o-, m- und p-Kresol, 2-tert.-Butyl-4-methylphenol, 6-tert.-Butyl-2,4-dimethylphenol, 2,6-Di-tert.-Butyl-4-methylphenol, 2-tert.-Butylphenol, 4-tert.-Butylphenol, 2,4-di-tert.-Butylphenol, 2-Methyl-4-tert.-Butylphenol, Hydrochinon, Brenzcatechin, Resorcin, 2-Methylhydrochinon und 2,5-Di-tert.-Butylhydrochinon, para-Aminophenol, para-Nitrosophenol, 2-Methoxyphenol, 2-Ethoxyphenol, 4-Methoxyphenol, Mono- und Di-tert.-Butyl-4-methoxyphenol, α-Tocopherol, 4-Hydroxy-2,2,6,6-tetramethyl-piperidin-N-oxyl, 2,2,6,6-Tetramethyl-piperidin-N-oxyl, 4,4',4"-Tris(2,2,6,6-tetramethyl-piperidin-N-oxyl)-phosphit, 3-Oxo-2,2,5,5-tetramethyl-pyrrolidin-N-oxyl, N,N-Diphenylamin, N-Nitroso-diphenylamin, N,N'-Dialkyl-para-phenylendiamin, wobei die Alkylreste gleich oder verschieden sein können und jeweils unabhängig voneinander aus 1 bis 4 Kohlenstoffatomen bestehen und geradkettig oder verzweigt sein können, N,N-Diethylhydroxylamin, Triphenylphosphin, Triphenylphosphit, hypophosphorige Säure, Triethylphosphit, Diphenylsulfid, Phenothiazin, sowie alle vorgenannten Inhibitoren gegebenenfalls in Kombination mit Metallsalzen wie beispielsweise den Chloriden, Dithiocarbonaten, Sulfaten, Salicylaten oder Acetaten von Kupfer, Mangan, Cer, Nickel und/oder Chrom, ist.
236. Verfahren gemäß Ausführungsform 234 oder 235, dadurch gekennzeichnet, dass die Gesamtmenge an zugesetztem Polymerisationsinhibitor, bezogen auf die in der festen Substanz, oder in der Schmelze, oder in der Lösung, oder in der Suspension, oder in der Emulsion enthaltene Gesamtmenge an Poly-3-hydroxypropionat, 10 bis 1000 Gew.ppm beträgt.
237. Verfahren gemäß einer der Ausführungsformen 1 bis 236, dadurch gekennzeichnet, dass die Acrylsäure aus der bei der Thermolyse des Poly-3-hydroxypropionats gebildeten Acrylsäure enthaltenden Gasphase durch absorptive und/oder kondensative Maßnahmen in die flüssige Phase überführt wird.
238. Verfahren gemäß Ausführungsform 237, dadurch gekennzeichnet, dass die Acrylsäure unter Anwendung wenigstens eines thermischen Trennverfahrens von der flüssigen Phase in einer im Vergleich zur flüssigen Phase erhöhten Reinheit abgetrennt wird, und das wenigstens eine thermische Trennverfahren wenigstens eine Rektifikation und/oder Kristallisation der in der flüssigen Phase enthaltenen Acrylsäure umfasst.
239. Verfahren gemäß Ausführungsform 238, dadurch gekennzeichnet, dass die Kristallisation eine Suspensionskristallisation unter Erhalt einer Acrylsäurekristalle enthaltenden Kristallsuspension ist.
240. Verfahren gemäß Ausführungsform 239, dadurch gekennzeichnet, dass sich ein Trennverfahren anschließt, bei dem die Acrylsäurekristalle von der Kristallsuspension in einer Waschschmelzewaschkolonne abgetrennt werden.
241. Verfahren gemäß Ausführungsform 240, dadurch gekennzeichnet, dass die Waschschmelzewaschkolonne eine hydraulische Waschschmelzewaschkolonne ist.
242. Verfahren gemäß einer der Ausführungsformen 1 bis 241, dadurch gekennzeichnet, dass sich an das Verfahren zur Herstellung von Acrylsäure ein Verfahren der radikalischen Polymerisation anschließt, in welchem die hergestellte Acrylsäure als solche und/oder in Form ihrer konjugierten Brönsted-Base sowie wahlweise im Gemisch mit anderen einfach und/oder mehrfach ungesättigten Verbindungen radikalisch initiiert in Polymerisat einpolymerisiert wird.
243. Verfahren gemäß einer der Ausführungsformen 1 bis 242, dadurch gekennzeichnet, dass der Co-Gehalt des (in einer Trennzone A abgetrennten) Poly-3-hydroxypropionats bei der Thermolyse 0 bis 1 Gew.-% beträgt.
244. Verfahren gemäß einer der Ausführungsformen 1 bis 243, dadurch gekennzeichnet, dass der Co-Gehalt des Poly-3-hydroxypropionats bei der Thermolyse 10⁻⁶ bis 1 Gew.-% beträgt.
245. Verfahren gemäß einer der Ausführungsformen 1 bis 244, dadurch gekennzeichnet, dass der Co-Gehalt des Poly-3-hydroxypropionats bei der Thermolyse 10⁻⁵ bis 1 Gew.-% beträgt.
246. Verfahren gemäß einer der Ausführungsformen 1 bis 245, dadurch gekennzeichnet, dass der Co-Gehalt des Poly-3-hydroxypropionats bei der Thermolyse 10⁻⁴ bis 1 Gew.-% beträgt.
247. Verfahren gemäß einer der Ausführungsformen 1 bis 246, dadurch gekennzeichnet, dass der Co-Gehalt des Poly-3-hydroxypropionats bei der Thermolyse 0,001 bis 0,75 Gew.-% beträgt.
248. Verfahren gemäß einer der Ausführungsformen 1 bis 247, dadurch gekennzeichnet, dass der Co-Gehalt des Poly-3-hydroxypropionats bei der Thermolyse 0,01 bis 0,75 Gew.-% beträgt.
249. Verfahren gemäß einer der Ausführungsformen 1 bis 248, dadurch gekennzeichnet, dass der Co-Gehalt des Poly-3-hydroxypropionats bei der Thermolyse 0,05 bis 0,75 Gew.-% beträgt.
250. Verfahren gemäß einer der Ausführungsformen 1 bis 249, dadurch gekennzeichnet, dass der Co-Gehalt des Poly-3-hydroxypropionats bei der Thermolyse 0,1 bis 0,5 Gew.-% beträgt.
251. Verfahren gemäß einer der Ausführungsformen 1 bis 242, dadurch gekennzeichnet, dass der Co-Gehalt des Poly-3-hydroxypropionats bei der Thermolyse ≤ 1 Gew.-% beträgt.
252. Verfahren gemäß einer der Ausführungsformen 1 bis 242, dadurch gekennzeichnet, dass der Co-Gehalt des Poly-3-hydroxypropionats bei der Thermolyse ≤ 0,5 Gew.-% beträgt.
253. Verfahren gemäß einer der Ausführungsformen 1 bis 242, dadurch gekennzeichnet, dass der Co-Gehalt des Poly-3-hydroxypropionats bei der Thermolyse ≤ 0,1 Gew.-% beträgt.
254. Verfahren gemäß einer der Ausführungsformen 1 bis 242, dadurch gekennzeichnet, dass der Co-Gehalt des Poly-3-hydroxypropionats bei der Thermolyse ≤ 0,01 Gew.-% beträgt.
255. Verfahren gemäß einer der Ausführungsformen 1 bis 242, dadurch gekennzeichnet, dass der Co-Gehalt des Poly-3-hydroxypropionats bei der Thermolyse ≤ 0,001 Gew.-% beträgt.
256. Verfahren gemäß einer der Ausführungsformen 1 bis 242, dadurch gekennzeichnet, dass der Co-Gehalt des Poly-3-hydroxypropionats bei der Thermolyse ≤ 10⁻⁵ Gew.-% beträgt.
257. Verfahren gemäß einer der Ausführungsformen 187 bis 256, dadurch gekennzeichnet, dass die wenigstens eine, die Thermolyse als Spaltkatalysator katalysierende, organische Wirkverbindung, Pentamethyldiethylentriamin ist.
258. Verfahren gemäß einer der Ausführungsformen 187 bis 256, dadurch gekennzeichnet, dass die wenigstens eine, die Thermolyse als Spaltkatalysator katalysierende, organische Wirkverbindung, N,N,N',N'-Tetramethyl-1,6-hexandiamin ist.
259. Verfahren gemäß einer der Ausführungsformen 187 bis 256, dadurch gekennzeichnet, dass die wenigstens eine, die Thermolyse als Spaltkatalysator katalysierende, organische Wirkverbindung, Bis(2-Dimethylaminoethyl)ether ist.
260. Verfahren gemäß einer der Ausführungsformen 187 bis 256, dadurch gekennzeichnet, dass die wenigstens eine, die Thermolyse als Spaltkatalysator katalysierende, organische Wirkverbindung, 2,2'-Dimorpholinodiethylether ist.
261. Verfahren gemäß einer der Ausführungsformen 187 bis 256, dadurch gekennzeichnet, dass die wenigstens eine, die Thermolyse als Spaltkatalysator katalysierende, organische Wirkverbindung, N,N-Diethylethanolamin ist.
262. Verfahren gemäß einer der Ausführungsformen 187 bis 256, dadurch gekennzeichnet, dass die wenigstens eine, die Thermolyse als Spaltkatalysator katalysierende, organische Wirkverbindung, N,N,N',N'-Tetramethyl-1,3-propandiamin ist.
263. Verfahren gemäß einer der Ausführungsformen 187 bis 256, dadurch gekennzeichnet, dass die wenigstens eine, die Thermolyse als Spaltkatalysator katalysierende, organische Wirkverbindung, N,N-Dimethylcyclohexylamin ist.
264. Verfahren gemäß einer der Ausführungsformen 187 bis 256, dadurch gekennzeichnet, dass die wenigstens eine, die Thermolyse als Spaltkatalysator katalysierende, organische Wirkverbindung, N,N-Dimethyl-1,3-diaminopropan ist.
265. Verfahren gemäß einer der Ausführungsformen 187 bis 256, dadurch gekennzeichnet, dass die wenigstens eine, die Thermolyse als Spaltkatalysator katalysierende, organische Wirkverbindung, N-Methylimidazol ist.
266. Verfahren gemäß einer der Ausführungsformen 187 bis 256, dadurch gekennzeichnet, dass die wenigstens eine, die Thermolyse als Spaltkatalysator katalysierende, organische Wirkverbindung, Dimethylimidazol ist.
267. Verfahren gemäß einer der Ausführungsformen 187 bis 256, dadurch gekennzeichnet, dass die wenigstens eine, die Thermolyse als Spaltkatalysator katalysierende, organische Wirkverbindung, 3-Hydroxypyridin ist.
268. Verfahren gemäß einer der Ausführungsformen 187 bis 256, dadurch gekennzeichnet, dass die wenigstens eine, die Thermolyse als Spaltkatalysator katalysierende, organische Wirkverbindung, 2-Hydroxypyridin ist.
269. Verfahren gemäß einer der Ausführungsformen 187 bis 256, dadurch gekennzeichnet, dass die wenigstens eine, die Thermolyse als Spaltkatalysator katalysierende, organische Wirkverbindung, 4-Hydroxypyridin ist.
270. Verfahren gemäß einer der Ausführungsformen 1 bis 269, dadurch gekennzeichnet, dass die Polydispersität Q des vom Produktgemisch A abgetrennten Poly-3-hydroxypropionats (in der Thermolysezone A) kleiner als oder gleich groß wie 2,0 ist.
271. Verfahren gemäß einer der Ausführungsformen 1 bis 269, dadurch gekennzeichnet, dass die Polydispersität Q des vom Produktgemisch A abgetrennten Poly-3-hydroxypropionats (in der Thermolysezone A) kleiner als oder gleich groß wie 1,5 ist.
272. Verfahren gemäß einer der Ausführungsformen 1 bis 271, dadurch gekennzeichnet, dass die Polydispersität Q des vom Produktgemisch A abgetrennten Poly-3-hydroxypropionats (in der Thermolysezone A) 1,2 bis 2 beträgt.
273. Verfahren gemäß einer der Ausführungsformen 1 bis 272, dadurch gekennzeichnet, dass die Polydispersität Q des vom Produktgemisch A abgetrennten Poly-3-hydroxypropionats (in der Thermolysezone A) 1,5 bis 1,8 beträgt.
274. Verfahren gemäß einer der Ausführungsformen 1 bis 273, dadurch gekennzeichnet, dass das vom Produktgemisch A abgetrennte Poly-3-hydroxypropionat (in der Thermolysezone A) ein relatives gewichtsmittleres Molekulargewicht von 3000 bis 12000 aufweist.
275. Verfahren gemäß einer der Ausführungsformen 1 bis 274, dadurch gekennzeichnet, dass das vom Produktgemisch A abgetrennte Poly-3-hydroxypropionat (in der Thermolysezone A) ein relatives gewichtsmittleres Molekulargewicht von 4000 bis 10000 aufweist.
276. Verfahren gemäß einer der Ausführungsformen 234 bis 275, dadurch gekennzeichnet, dass die Gesamtmenge an zugesetztem Polymerisationsinhibitor, bezogen auf die in der festen Substanz, oder in der Schmelze, oder in der Lösung, oder in der Suspension, oder in der Emulsion enthaltene Gesamtmenge an Poly-3-hydroxypropionat, 50 bis 500 Gew.ppm beträgt.
277. Verfahren gemäß einer der Ausführungsformen 234 bis 276, dadurch gekennzeichnet, dass die Gesamtmenge an zugesetztem Polymerisationsinhibitor, bezogen auf die in der festen Substanz, oder in der Schmelze, oder in der Lösung, oder in der Suspension, oder in der Emulsion enthaltene Gesamtmenge an Poly-3-hydroxypropionat, 150 bis 350 Gew.ppm beträgt.
278. Verfahren gemäß einer der Ausführungsformen 1 bis 277, dadurch gekennzeichnet, dass die Co-Gehalte in den Verfahren gemäß einer der Ausführungsformen 243 bis 256 die Gesamtgehalte des Poly-3-hydroxypropionats an Co⁺² sind.
279. Verfahren gemäß einer der Ausführungsformen 1 bis 278, dadurch gekennzeichnet, dass die Co-Gehalte in den Verfahren gemäß einer der Ausführungsformen 243 bis 256 die Gesamtgehalte des Poly-3-hydroxypropionats an Co⁺¹ sind.
280. Verfahren gemäß einer der Ausführungsformen 1 bis 279, dadurch gekennzeichnet, dass die Co-Gehalte in den Verfahren gemäß einer der Ausführungsformen 243 bis 256 die Gesamtgehalte des Poly-3-hydroxypropionats an Co⁰ sind.
281. Verfahren gemäß einer der Ausführungsformen 1 bis 280, dadurch gekennzeichnet, dass die Co-Gehalte in den Verfahren gemäß einer der Ausführungsformen 243 bis 256 die Gesamtgehalte des Poly-3-hydroxypropionats an Co⁻¹ sind.
282. Verfahren gemäß einer der Ausführungsformen 1 bis 281, dadurch gekennzeichnet, dass die wässrige Fällflüssigkeit wenigstens ein von einer anorganischen und/oder organischen Säure verschiedenes organisches Lösungsmittel enthält.
283. Verfahren gemäß einer der Ausführungsformen 1 bis 282, dadurch gekennzeichnet, dass die wässrige Waschflüssigkeit wenigstens ein von einer anorganischen und/oder organischen Säure verschiedenes organisches Lösungsmittel enthält.
284. Verfahren gemäß Ausführungsform 282 oder 283, dadurch gekennzeichnet, dass das organische Lösungsmittel wenigstens ein organisches Lösungsmittel aus der Gruppe bestehend aus Methanol, Ethanol, Aceton, Methylethylketon, Dimethylsulfoxid, N-methyl-2-pyrrolidon, Formamid, N,N-Dimethylformamid, Tetrahydrofuran und 1,4-Dioxan ist.

### Beispiele und Vergleichsbeispiele (Versuche "1." bis "23.")

(in den nachfolgenden Versuchen "1." Bis "23." zur Darstellung von Beispielen und Vergleichsbeispielen jeweils erstmals ausgeführte und spezifizierte Einsatzmaterialien sowie Untersuchungsmethoden wurden in darauffolgenden Versuchen an entsprechender Stelle in entsprechender Weise eingesetzt, soweit dort nicht ausdrücklich etwas anderes explizit erwähnt ist; alle Fällungen und Wäschen von Poly-3-hydroxypropionat wurden an Luft durchgeführt)

### 1. Nicht erfindungsgemäße Herstellung von Poly-3-hydroxypropionat durch carbonylierende Umsetzung von in Diglyme gelöstem Ethylenoxid im Beisein eines Co umfassenden Katalysatorsystems (Vergleichsbeispiel 1)

Die carbonylierende Umsetzung erfolgte in einem mit einem Blattrührer rührbaren Autoklaven A (die Bewegung des Blattrührers erfolgte über eine Magnet-Kupplung), dessen Reaktionsraum von außen wahlweise beheizt oder gekühlt werden konnte. Alle den Reaktionsraum berührenden Oberflächen waren aus Hastelloy HC4 gefertigt. Der Reaktionsraum des Autoklaven hatte eine kreiszylindrische Geometrie. Die Höhe des Kreiszylinders lag bei 335 mm. Der Innendurchmesser des Kreiszylinders war 107 mm. Die Einhüllende des Reaktionsraums hatte eine Wanddicke von 19 mm (Hastelloy HC4). Der Kopf des Autoklaven war mit einem Gaseingang-/Gasausgang-Ventil V bestückt, das in den Reaktionsraum hinein öffnete. Die Temperatur im Reaktionsraum wurde mit Hilfe eines Thermoelements ermittelt. Die Regelung der Reaktionstemperatur erfolgte elektronisch kontrolliert. Der Innendruck im Reaktionsraum wurde mit einem entsprechenden Sensor kontinuierlich verfolgt.

Der Reaktionsraum des Autoklaven wurde zunächst mit Argon inertisiert (Gehalte des Ar: ≥ 99,999 Vol.-% Ar, ≤ 2 Vol.ppm O₂, ≤ 3 Vol.ppm H₂O und ≤ 0,5 Vol.ppm Gesamtmenge an Kohlenwasserstoffen).

Anschließend wurde der auf 10 °C temperierte Autoklav A unter Argon mit 8,2 g Dicobaltoctacarbonyl (Co₂(CO)₈; Lieferfirma: Sigma-Aldrich; Spezifikation: 1-10 % Hexan, ≥ 90 % Co, Bestellnummer: 60811), 4,4 g 3-Hydroxypyridin (Lieferfirma: Sigma-Aldrich; Spezifikation: 99 % Gehalt, Bestellnummer: H57009) und 802,4 g Diglyme (Lieferfirma: Sigma-Aldrich; Spezifikation: 99% Gehalt, Bestellnummer: M1402) befüllt und der Autoklav anschließend verschlossen. Die Temperatur der beiden Feststoffe war 25 °C und die Temperatur des Diglyme war 10 °C. Dann wurde unter Beibehalt der Innentemperatur von 10 °C durch das Ventil V Kohlenmonoxid in den Autoklaven gepresst, bis der Druck im Reaktionsraum 1,5 · 10⁶ Pa betrug (Kohlenmonoxid der BASF SE, Spezifikation: 99,2 % CO). Anschließend wurde die Temperatur im Reaktionsraum auf 35 °C erhöht, um die Dichtigkeit des Autoklaven A zu verifizieren (über einen Zeitraum von 90 min). Dann wurde die Atmosphäre im Reaktionsraum durch Öffnen des Ventils V auf einen Innendruck von 10⁶ Pa entspannt. Die Temperatur im Innenraum betrug danach 30 °C.

Anschließend wurden durch das Ventil V 51,0 g Ethylenoxid (1,5 g/min) in den Reaktionsraum gepumpt (Lieferfirma: BASF SE; Spezifikation: 99,9 % Reinheit). Dabei verringerte sich die Temperatur im Reaktionsraum auf 25 °C. Danach wurde wieder Kohlenmonoxid in den Autoklav gepresst, bis der Druck im Reaktionsraum 6 · 106 Pa erreichte (unter Beibehalt der Innentemperatur von 25 °C).

Nun wurde unter Rühren (700 U/min) die Temperatur im Reaktionsraum des Autoklaven A innerhalb von 45 min im Wesentlichen linear auf 75 °C erhöht. Diese Temperatur wurde unter Rühren 8 h beibehalten. Der Druck im Reaktionsraum fiel in diesem Zeitraum auf 5 · 106 Pa ab. Dann wurde die Beheizung des Autoklaven A abgeschaltet. Innerhalb von 5 h und 50 min kühlte die Temperatur im gerührten Reaktionsraum im Wesentlichen exponentiell auf 25 °C ab (nach 50 min war die Innentemperatur auf 60 °C, nach 150 min auf 40 °C und nach 235 min auf 30 °C abgefallen). Der zugehörige Druck im Reaktionsraum war 4,3 · 106 Pa. Nun wurde der Autoklav A auf Normaldruck entspannt und der Reaktionsraum 3 mal nacheinander mit Argon (10⁶ Pa) gespült.

Im Reaktionsraum befanden sich 860,2 g einer dunkelrot-braunen Lösung als Produktgemisch A. Sie wurde dem Autoklaven entnommen und anschließend in einem verschlossenen Glaskolben während 12 h in einem eine Temperatur von 7 °C aufweisenden Kühlraum sich selbst überlassen. Das dabei ausfallende Poly-3-hydroxypropionat wurde abfiltriert und der Filterkuchen mit 252 g Methanol (Lieferfirma: BASF SE; Spezifikation: 99,8 % Reinheit) gewaschen. Das Methanol hatte eine Temperatur von 25 °C und wurde durch den Filterkuchen gesaugt. Der so gewaschene Filterkuchen wurde dann 10 h bei reduziertem Druck getrocknet (10 hPa, Trocknungstemperatur: 25 °C).

Die so vom Produktgemisch A als erste Fraktion abgetrennten 8,1 g Poly-3-hydroxypropio-nat enthielten, auf das Gewicht ihrer Masse bezogen, noch 2,8 Gew.-% an Co (der Einwaagegehalt des Produktgemischs A an Co, bezogen auf das Gewicht der theoretisch maximal möglichen Bildungsmenge an Poly-3-hydroxypropionat, war 3,39 Gew.-%).

Sein gewichtsmittleres relatives Molekulargewicht Mw war 7270 (=̂ einem gewichtsmittleren Molgewicht von 7270 g/mol).

Das beim Abfiltrieren des Poly-3-hydroxypropionats anfallende Filtrat wurde gaschromatographisch analysiert (dazu wurde ein Gaschromatograph der Firma Hewlett Packard (HP Model 5890 Series II) mit Flammenionisationsdetektor verwendet). Es enthielt (angegeben als Flächenprozent der Gesamtfläche der GC-Peaks) 0,7 % Ethylenoxid, 97,1 % Diglyme, 0,2 % des Nebenprodukts β-Propiolacton und 0,3 % des Nebenprodukts Bernsteinsäureanhydrid.

Es wurde mit dem bei der Wäsche des abfiltrierten Poly-3-hydroxypropionats durch das selbige gesaugte Methanol vereinigt. Das so erzeugte Gemisch wurde für 12 h in einem eine Temperatur von 7 °C aufweisenden Kühlraum sich selbst überlassen. Das dabei ausfallende Poly-3-hydroxypropionat wurde wieder abfiltriert und der dabei resultierende Filterkuchen mit 250 g Methanol gewaschen. Das Methanol hatte eine Temperatur von 25 °C und wurde durch den Filterkuchen gesaugt. Der gewaschene Filterkuchen wurde wieder bei 10 hPa und 25 °C während 10 h getrocknet.

Die Menge des so vom Produktgemisch A als zweite Fraktion abgetrennten Poly-3-hydroxypropionats betrug 18,7 g. Bezogen auf das Gewicht seiner Masse enthielt es noch 1,1 Gew.-% an Co. Sein gewichtsmittleres relatives Molekulargewicht Mw war 5190 (=̂ einem gewichtsmittleren Molgewicht von 5190 g/mol).

Insgesamt wurden 26,8 g Poly-3-hydroxypropionat vom Produktgemisch A abgetrennt. Dies sind 32,1 % der theoretisch möglichen maximalen Ausbeute.

Die Bestimmung der Co-Gehalte erfolgte durch optische lonenemissionsspektroskopie mit induktiv gekoppeltem Plasma (ICP-OES).

Als Messgerät wurde ein Varian 720-ES ICP-OES Spektrometer verwendet. Die Wellenlänge der zur Analyse herangezogenen Spektrallinie des Co betrug 237,86 nm.

Zur Probenvorbereitung wurden jeweils 0,1 g der zu untersuchenden Probe mit einem Gemisch aus konzentrierter Schwefelsäure, konzentrierter Salpetersäure und konzentrierter Perchlorsäure (als stark oxidierende Säuren) in einem Quarzglas verascht (unter Anwendung von Temperaturen bis zu 320 °C wurden die Säuren quantitativ abgeraucht). Der dabei verbleibende Rückstand wurde in konzentrierte Salzsäure aufgenommen und unter Erhitzen und Zugabe von Wasser gelöst. Die resultierende Lösung wurde anschließend analysiert.

Die Molekulargewichtsbestimmungen erfolgten durch Größenausschlußchromatographie (SEC/GPC). Die Elutionskurve wurde mit Hilfe von Polymethylmethacrylat (PMMA) Eichkurven in die eigentliche Verteilungskurve umgerechnet. Die Kalibrierung erfolgte mit eng verteilten PMMA-Standards, deren relative Molekulargewichte im Bereich von M = 800 bis M = 1 820 000 lagen. Die Werte außerhalb dieses Elutionsbereichs wurden extrapoliert.

### 2. Nicht erfindungsgemäße Herstellung von Poly-3-hydroxypropionat durch carbonylierende Umsetzung von in Diglyme gelöstem Ethylenoxid im Beisein eines Co umfassenden Katalysatorsystems (Vergleichsbeispiel 2).

Es wurde wie im Vergleichsbeispiel 1 verfahren, jedoch mit nachfolgenden Unterschieden:
- die Einsatzmenge an Dicobaltoctacarbonyl war 16,0 g;
- die Einsatzmenge an 3-Hydroxypyridin war 8,7 g;
- die Einsatzmenge an Diglyme betrug 1001,2 g;
- die Einsatzmenge an Ethylenoxid betrug 97,8 g;
- die Dichtigkeitsprüfung des Autoklaven erstreckte sich nur über 50 min und erfolgte bei einer auf 28 °C erhöhten Temperatur im Reaktionsraum;
- die Zufuhr des Ethylenoxids erfolgte ebenso unter Beibehaltung der 28 °C im Reaktionsraum wie die abschließende Zufuhr an Kohlenmonoxid (6 · 10⁶ Pa);
- am Ende der 8 h Reaktionsdauer lag der Druck im Reaktionsraum des Autoklaven bei 3 · 10⁶ Pa;
- die Abkühlung auf 25 °C erstreckte sich über einen Zeitraum von 6 h;
   die Innentemperatur von 60 °C war nach 66 min erreicht;
   die Innentemperatur von 40 °C war nach 165 min erreicht;
   die Innentemperatur von 30 °C war nach 255 min erreicht;
   der Druck im Reaktionsraum beim Erreichen der 25 °C war 2,8 · 106 Pa;
- im Reaktionsraum befanden sich 1106,3 g einer dunkelrot-braunen Lösung als Produktgemisch A.

Das Produktgemisch A wurde in einem verschlossenen Glaskolben während 12 h in einem eine Temperatur von 7 °C aufweisenden Kühlraum sich selbst überlassen. Das dabei ausfallende Poly-3-hydroxypropionat wurde abfiltriert und der Filterkuchen mit 300 g Methanol der Temperatur 25 °C gewaschen. Der gewaschene Filterkuchen wurde 10 h getrocknet (10 hPa, 25 °C). Die so vom Produktgemisch A abgetrennten 41,1 g Poly-3-hydroxypropionat (erste Fraktion) enthielten auf das Gewicht ihrer Masse bezogen noch 1,6 Gew.-% an Co (der Einwaagegehalt des Produktgemischs A an Co, bezogen auf das Gewicht der maximal möglichen Bildungsmenge an Poly-3-hydroxypropionat war 2,97 Gew.-%). Das gewichtsmittlere relative Molekulargewicht war Mw = 7220.

Das beim Abfiltrieren des Poly-3-hydroxypropionats anfallende Filtrat wurde gaschromatographisch analysiert. Es enthielt (angegeben als Flächenprozent der Gesamtfläche der GC-Peaks) 0,9 % Ethylenoxid, 92,7 % Diglyme, 1,0 % des Nebenprodukts β-Propiolacton und 0,6 % des Nebenprodukts Bernsteinsäureanhydrid.

Es wurde mit dem bei der Wäsche des abfiltrierten Poly-3-hydroxypropionats (erste Fraktion) durch das selbige gesaugte Methanol vereinigt. Das so erzeugte Gemisch wurde für 12 h in einem eine Temperatur von 7 °C aufweisenden Kühlraum sich selbst überlassen. Das dabei ausfallende Poly-3-hydroxypropionat wurde wieder abfiltriert und der dabei resultierende Filterkuchen mit 300 g Methanol der Temperatur 25 °C gewaschen (das Methanol wurde wie immer durch den Filterkuchen hindurchgesaugt). Der gewaschene Filterkuchen wurde wieder bei 10 hPa und 25 °C während 10 h getrocknet.

Die Masse des so vom Produktgemisch A als zweite Fraktion getrennten Poly-3-hydroxy-propionats betrug 88,0 g. Bezogen auf das Gewicht seiner Masse enthielt es noch 1,6 Gew.-% an Co. Sein gewichtsmittleres relatives Molekulargewicht Mw war 5640.

Das beim Abfiltrieren der zweiten Fraktion an Poly-3-hydroxypropionat anfallende Filtrat wurde mit dem bei der Wäsche der zweiten Fraktion an Poly-3-hydroxypropionat durch das selbige gesaugte Methanol vereinigt. Das so erzeugte Gemisch wurde für 12 h in einem eine Temperatur von 7 °C aufweisenden Kühlraum sich selbst überlassen. Das dabei anfallende Poly-3-hydroxypropionat wurde wieder abfiltriert (dritte Fraktion) und der dabei resultierende Filterkuchen mit 300 g Methanol der Temperatur 25 °C gewaschen. Der gewaschene Filterkuchen wurde wieder bei 10 hPa und 25 °C während 10 h getrocknet.

Die Masse des so vom Produktgemisch A als dritte Fraktion abgetrennten Poly-3-hydroxy-propionats betrug 5,8 g. Bezogen auf das Gewicht seiner Masse enthielt es noch 1,8 Gew.-% an Co. Sein gewichtsmittleres relatives Molekulargewicht M_{w} war 5240. Das beim Abfiltrieren der dritten Fraktion an Poly-3-hydroxypropionat anfallende Filtrat wurde mit dem bei der Wäsche der dritten Fraktion an Poly-3-hydroxypropionat durch das selbige gesaugte Methanol vereinigt. Das resultierende Gemisch wurde für 12 h in einem eine Temperatur von 7 °C aufweisenden Kühlraum sich selbst überlassen. Das dabei ausfallende Poly-3-hydroxypropionat wurde wieder abfiltriert (vierte Fraktion) und der dabei resultierende Filterkuchen mit 300 g Methanol der Temperatur 25 °C gewaschen. Der gewaschene Filterkuchen wurde wieder bei 10 hPa und 25 °C während 10 h getrocknet.

Die Masse des so vom Produktgemisch A als dritte Fraktion abgetrennten Poly-3-hydroxy-propionats betrug 5,3 g. Bezogen auf das Gewicht seiner Masse enthielt es 2,7 Gew.-% an Co. Sein gewichtsmittleres relatives Molekulargewicht Mw war 4230.

Der erhöhte Co-Gehalt der dritten Fraktion wird darauf zurückgeführt, dass im resultierenden Lösungsmittelgemisch jetzt offensichtlich zuvor noch gelöstes Co als separates Co-Salz mitfällt.

Insgesamt wurden 140,2 g Poly-3-hydroxypropionat vom Produktgemisch A abgetrennt. Das sind 87,6 % der theoretisch möglichen maximalen Ausbeute.

### 3. Nicht erfindungsgemäße Herstellung von Poly-3-hydroxypropionat durch carbonylierende Umsetzung von in Diglyme gelöstem Ethylenoxid im Beisein eines Co umfassenden Katalysatorsystems (Vergleichsbeispiel 3)

Die carbonylierende Umsetzung erfolgte in einem mit einem Stabrührer rührbaren Autoklaven B (die Bewegung des Stabrührers erfolgte über eine Magnet-Kupplung), dessen Reaktionsraum von außen wahlweise beheizt oder gekühlt werden konnte. Alle den Reaktionsraum berührenden Oberflächen waren aus Hastelloy HC4 gefertigt. Der Reaktionsraum des Autoklaven hatte eine kreiszylindrische Geometrie. Die Höhe des Kreiszylinders war 90 mm. Der Innendurchmesser des Kreiszylinders lag bei 59,1 mm. Die Einhüllende des Reaktionsraums hatte eine Wanddicke von 15,5 mm (Hastelloy HC4).

Der Kopf des Autoklaven war mit einem Gaseingang-/Gasausgang-Ventil V bestückt, das in den Reaktionsraum hinein öffnete. Die Temperatur im Reaktionsraum wurde mit Hilfe eines Thermoelements ermittelt. Die Regelung der Reaktionstemperatur erfolgte elektronisch kontrolliert. Der Innendruck im Reaktionsraum wurde mit einem entsprechenden Sensor kontinuierlich verfolgt. Die Spezifikationen voneinander entsprechenden Einsatzstoffen entsprach jenen aus Vergleichsbeispiel 1.

Zunächst wurde der Reaktionsraum des Autoklaven mit molekularem Stickstoff inertisiert (Gehalte des N₂: ≥ 99,99 Vol.-% N₂, ≤ 20 Vol.ppm O₂ und ≤ 5 Vol.ppm H₂O).

Anschließend wurde der auf 10 °C temperierte Autoklav B unter N₂ mit 1,58 g Dicobaltoctacarbonyl, 0,87 g 3-Hydroxypyridin und 88,10 g Diglyme befüllt und der Autoklav B anschließend verschlossen. Die Temperatur der beiden Feststoffe war 25 °C und die Temperatur des Diglyme war 10 °C. Dann wurde unter Beibehalt der Innentemperatur von 10 °C durch das Ventil V Kohlenmonoxid in den Autoklaven gepresst, bis der Druck im Reaktionsraum 1,5 · 106 Pa betrug. Anschließend wurde die Temperatur im Reaktionsraum auf 25 °C erhöht, um die Dichtigkeit des Autoklaven B zu verifizieren (über einen Zeitraum von 30 min). Dann wurde die Atmosphäre im Reaktionsraum des Autoklaven B durch Öffnen des Ventils V auf 2 · 105 Pa entspannt. Anschließend wurden durch das Ventil V 11,66 g Ethylenoxid (0,3 g/min) in den Reaktionsraum gepumpt. Danach betrug die Temperatur im Reaktionsraum 19 °C. Schließlich wurde wieder Kohlenmonoxid in den Autoklaven gepresst, bis der Druck im Reaktionsraum 6 · 106 Pa erreichte (bei einer Innentemperatur von 19 °C). Nun wurde die Temperatur im Reaktionsraum des Autoklaven B unter Rühren (700 U/min) innerhalb von 45 min im Wesentlichen linear auf 75 °C erhöht. Diese Temperatur wurde unter Rühren 4 h beibehalten. Dann wurde die Beheizung des Autoklaven B abgeschaltet und der Autoklav (ohne Rühren im Reaktionsraum) in Eiswasser auf 0 °C abgekühlt. Nun wurde der Autoklav B auf Normaldruck entspannt und das Produktgemisch A durch 30 minütiges sich selbst überlassen ohne Rühren ausgegast. Danach wurde der Reaktionsraum des Autoklaven B 3 mal nacheinander mit molekularem Stickstoff (10⁶ Pa) gespült. Die Temperatur von 0 °C im Reaktionsraum des Autoklaven B wurde während der gesamten Zeit stetig beibehalten.

Das im Reaktionsraum befindliche Produktgemisch A war eine rotbraune Suspension (106,8 g). Das bei der vergleichsweise geringen Temperatur von 0 °C ausgefallene Poly-3-hydroxypropionat wurde abfiltriert und mit 60 g Methanol gewaschen (das Methanol wies eine Temperatur von 0 °C auf und wurde durch den Filterkuchen gesaugt). Die methanolische Waschlösung wurde verworfen. Der gewaschene Filterkuchen wurde 10 h bei reduziertem Druck getrocknet (10 hPa, Trocknungstemperatur: 25 °C). Die so vom Produktgemisch A abgetrennten 5,7 g Poly-3-hydroxypropionat enthielten auf das Gewicht ihrer Masse bezogen noch 1,95 Gew.-% an Co (der Einwaagegehalt des Produktgemischs A an Co, bezogen auf das Gewicht der theoretisch maximal möglichen Bildungsmenge an Poly-3-hydroxypropionat, war 2,83 Gew.-%).

Das beim Abfiltrieren des Poly-3-hydroxypropionats aus dem Produktgemisch A anfallende Filtrat wurde gaschromatographisch analysiert. Es enthielt (angegeben als Flächenprozente der Gesamtfläche der GC-Peaks) 0,5 % Ethylenoxid, 92,7 % Diglyme, 0 % β-Propiolacton und 0,8 % des Nebenprodukts Bernsteinsäureanhydrid.

### 4. Nicht erfindungsgemäße Herstellung von Poly-3-hydroxypropionat durch carbonylierende Umsetzung von in Diglyme gelöstem Ethylenoxid im Beisein eines Co umfassenden Katalysatorsystems (Vergleichsbeispiele 4a, 4b und 4c)

Vergleichsbeispiel 3 wurde zweimal wiederholt und die beiden bei der Filtration des ausgefallenen Poly-3-hydroxypropionats vom jeweiligen Produktgemisch A anfallenden Filtrate zu einem Gesamtfiltrat vereinigt.

Vom Gesamtfiltrat wurden vier identische Teilmengen zu jeweils 30 g entnommen. Die vierte Teilmenge bildete die Grundlage für das Beispiel 1 dieser Schrift. Die anderen drei Teilmengen des Gesamtfiltrats wurden jeweils mit 16 g einer der folgenden, jeweils eine Temperatur von 25 °C aufweisenden, Fällflüssigkeiten FF versetzt: Methanol (= Vergleichsbeispiel 4a), Cyclohexan (Lieferfirma: Sigma-Aldrich; Spezifikation: ≤ 0,01 % Wasser (Karl Fischer), ≤ 0,001 % nicht flüchtige Stoffe; Bestellnummer: 34496) (= Vergleichsbeispiel 4b) und tert.-Butylmethylether = Methyl-tert.-butylether (MTBE) (Lieferfirma: Sigma-Aldrich; Spezifikation: < 0,003 % water, 99,8 % Gehalt; Bestellnummer: 306975) (= Vergleichsbeispiel 4c). Das dabei resultierende Gemisch wurde für 12 h in einem eine Temperatur von 7 °C aufweisenden Kühlraum sich selbst überlassen.

Das jeweils ausfallende Poly-3-hydroxypropionat wurde abfiltriert und der dabei jeweils resultierende Filterkuchen jeweils mit 20 g der entsprechenden Fällflüssigkeit als Waschflüssigkeit gewaschen (die Waschflüssigkeit wurde jeweils mit einer Temperatur von 25 °C durch den Filterkuchen gesaugt). Der gewaschene Filterkuchen wurde bei 10 hPa und 25 °C während 10 h getrocknet.

Die nachfolgende Tabelle 2 zeigt in Abhängigkeit von der verwendeten Fällflüssigkeit FF die Masse des so jeweils abgetrennten Poly-3-hydroxypropionats (P3HP), die bezogen auf das Gewicht dieser Masse noch enthaltene Menge an Co (in Gew.-%) und das jeweilige zugehörige relative gewichtsmittlere Molekulargewicht Mw. Außerdem weist die Tabelle 2 noch die Farbe des jeweils abgetrennten Poly-3-hydroxypropionats aus.

**Tabelle 2**

| **VBsp.** | **FF** | **Co (Gew.-%)** | **P3HP (g)** | **Farbe P3HP** | **M_{W}** |
|---|---|---|---|---|---|
| 4a | Methanol | 1,7 | 1,9 | hellbraun | 4085 |
| 4b | Cyclohexan | 3,5 | 2,9 | rosa-braun | 4221 |
| 4c | MTBE | 4,6 | 2,2 | rotbraun | 4719 |

Zur Bestimmung der vorhandenen Endgruppen sowie der Struktur des abgetrennten Feststoffs wurde selbiger im Fall der Verwendung von Methanol als Fällflüssigkeit sowohl durch Massenspektrometrie mit Matrix-unterstützter Laser-Desorption/Ionisation (MALDI-MS) als auch mit Gelpermeationschromatographie-Massenspektrometrie (GPC-MS) analysiert.

Für die MALDI-MS Untersuchung wurde die zu untersuchende Probenmenge zunächst vollständig in wässrigem Acetonitril (50 Vol.-% Wasser, 50 Vol.-% Acetonitril) gelöst und dann mit 2,5-Dihydroxybenzoesäure und Natriumtrifluoracetat als Matrixsubstanzen (beide ebenfalls im wässrigen Acetonitril gelöst) auf ein MALDI Stahl-Target aufgebracht und das Lösemittel entfernt. Mit einem Stickstoff-Laser (3 ns Pulszeit, Wellenlänge = 337 nm) wurde der Analyt im Gemisch mit der Matrix vom Stahl-Target verdampft und ionisiert.

Zur GPC-MS Untersuchung wurde von einem Extrakt der zu untersuchenden Probenmenge in Tetrahydrofuran (THF) ausgegangen (die Probe löste sich in THF nicht vollständig), dessen gelöst enthaltene Bestandteile vorab ihrer MS-Untersuchung mittels GPC aufgetrennt wurden. Die Ionisation erfolgte mittels Elektrospray-Ionisierung (ESI).

Folgende Strukturen/Endgruppen konnten zugeordnet werden:

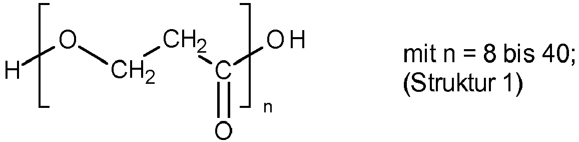

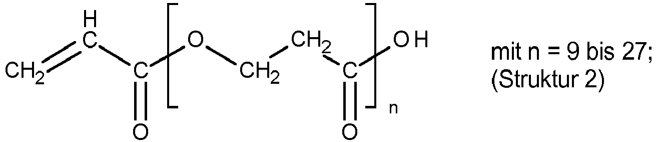

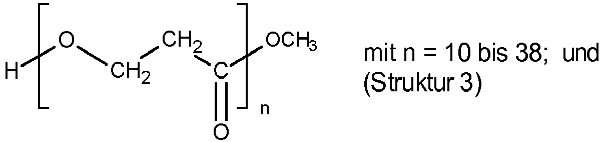

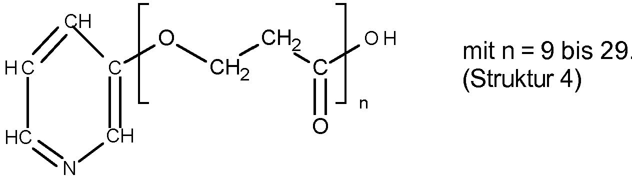

Quantitative Mengenbestimmungen der vorstehenden Strukturen erfolgten mittels ¹H-NMR Spektroskopie an einem DPX 400/1 FT-NMR Spektrometer der Firma Bruker bei einer ¹H-Trägerfrequenz von 400 MHZ.

Die Probenkonzentration betrug 5 mg Poly-3-hydroxypropionat in 1 ml CDCl₃ gelöst. Die Breite des Anregungsimpulses war 8012, 82 Hz. Die Probentemperatur bei der Aufnahme der Spektren betrug stets 26,8 °C. Zur Anregung wurde eine Folge von 30°-Pulsen verwendet. Jeweils 32 Einzelaufnahmen wurden zum Ergebnisspektrum akkumuliert.

Im Ergebnis bestand die untersuchte Probe zu ≥ 99 Gew.-% aus der Struktur 1. Die Protonen der Vinylgruppe in der Struktur 2 waren mit ihrem ¹H-NMR Signal sichtbar. ¹H-NMR Signale von aromatischen Protonen der Struktur 4 konnten nicht detektiert werden.

Dieses Ergebnis wird insbesondere auf im Reaktionsgemisch noch vorhandene Spuren von Wasser zurückgeführt.

### 5. Erfindungsgemäße Herstellung von Poly-3-hydroxypropionat durch carbonylierende Umsetzung von in Diglyme gelöstem Ethylenoxid im Beisein eines Co umfassenden Katalysatorsystems und Wäsche von abgetrenntem Poly-3-hydroxypropionat mit Wasser (Beispiel 1).

Die vierte Teilmenge (30 g) des Gesamtfiltrats aus Vergleichsbeispiel 4 wurde mit 16 g Wasser (das eine Temperatur von 25 °C aufwies) versetzt und das dabei resultierende wässrige Gemisch für 12 h in einem eine Temperatur von 7 °C aufweisenden Kühlraum sich selbst überlassen.

Das dabei anfallende Poly-3-hydroxypropionat wurde abfiltriert und der dabei resultierende Filterkuchen mit 20 g Wasser gewaschen (das Wasser wurde mit einer Temperatur von 25 °C durch den Filterkuchen gesaugt). Der gewaschene Filterkuchen wurde bei 10 hPa und 60 °C während 72 h getrocknet.

Nachfolgende Tabelle 3 zeigt die mit den bereits beschriebenen Untersuchungsmethoden ermittelten Eigenschaften des so von seinem Produktgemisch A abgetrennten Poly-3-hydroxypropionats.

**Tabelle 3**

| **Bsp.** | **FF** | **Co (Gew.-%)** | **P3HP (g)** | **Farbe P3HP** | **M_{W}** |
|---|---|---|---|---|---|
| 1 | Wasser | 0,7 | 2,2 | hellorange-creme | 5598 |

Die Ergebnisse weisen aus, das Wasser eine besonders geeignete Waschflüssigkeit zum Zweck der Decobaltierung des von einem Produktgemisch A abgetrennten Poly-3-hydroxy-propionats ist.

### 6. Erfindungsgemäße Herstellung von Poly-3-hydroxypropionat durch carbonylierende Umsetzung von in Diglyme gelöstem Ethylenoxid im Beisein eines Co umfassenden Katalysatorsystems und Wäsche von abgetrenntem Poly-3-hydroxypropionat mit einer wässrigen Essigsäurelösung (Beispiel 2)

Vergleichsbeispiel 2 wurde mehrfach wiederholt und durch Mischen von verschiedenen abgetrennten Fraktionen ein Poly-3-hydroxypropionat erzeugt, das auf das Gewicht seiner Masse bezogen noch 2 Gew.-% an Co enthielt.
Eine 80 g Probe dieses Poly-3-hydroxypropionats wurde mit 658 g einer 12,5 gew.-%igen Lösung von Essigsäure in Wasser gewaschen (die Temperatur der Essigsäurelösung war 25 °C; sie wurde durch das P3HP hindurchgesaugt).
Anschließend wurde mit 200 g Wasser (Temperatur = 25 °C) und danach mit 200 g Methanol (Temperatur = 25 °C) nachgewaschen und der verbliebene Feststoff bei 10 hPa und 25 °C während 10 h getrocknet.
Der Co-Gehalt des so erzeugten Poly-3-hydroxypropionats lag bei 0,2 Gew.-%.

Das gewichtsmittlere Molekulargewicht vor der Wäsche war Mw = 5930 und nach der Wäsche M_{w} = 5810.

Eine Untersuchung des Schmelzverhaltens (diese erfolgte nach der Methode der dynamischen Differenzkalorimetrie (DSC) an einem Differenzkalorimeter Q2000 der Firma TA (Thermal Analysis) Instruments; die Probenmenge betrug jeweils 8,2 mg und die Heiz-/Kühlrate war 20 K/min) ergab einen Schmelzbereich von 65,7 °C bis 79 °C für das P3HP vor der Wäsche und von 65,4 °C bis 71,6 °C nach der Wäsche.

Die Elementaranalyse des P3HP (sie erfolgte auf der Grundlage der Vollverbrennung der jeweiligen Probe mit anschließender gaschromatographischer Analyse der Verbrennungsprodukte an einem CHN-Analysator der Fa. Elementar Analysensysteme GmbH, vom Typ vario EL cube, und an einem O-Analysator der Fa. EuroVektor vom Typ EA) ergab (Angaben in Gew..-%):
C : 47,8 %;
O:42,6%;
H : 5,6 %; und
N : 0,5 %.

Nach der Wäsche ergab die entsprechende Elementaranalyse:
C : 49,3 %;
O: 43,5 %;
H : 5,7 %; und
N : <0,5 %.

Struktur- und Endgruppenanalyse mittels MALDI-MS und GPC-MS ergab für das gewaschene P3HP folgende Zuordnungen:

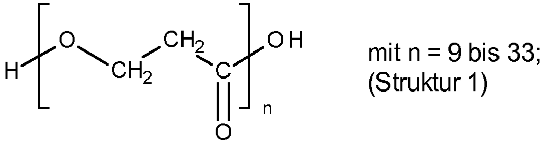

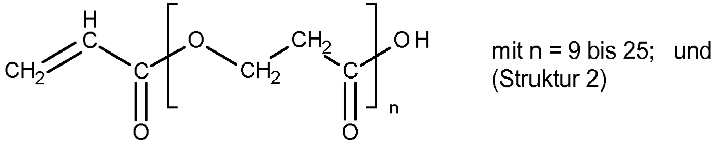

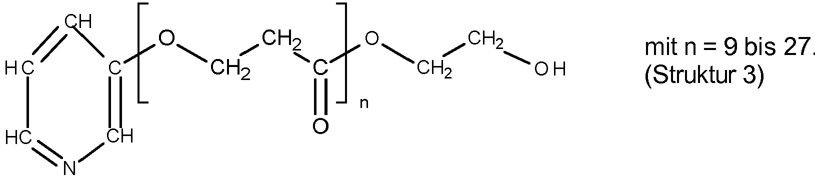

Quantitative Mengenbestimmungen der vorstehenden Strukturen erfolgten mit der bereits beschriebenen ¹H-NMR-Methode.

Im Ergebnis bestand die untersuchte Probe zu ≥ 99 % aus der Struktur 1. Protonen der Vinylgruppe in der Struktur 2 waren mit ihrem ¹H-NMR Signal sichtbar. Ebenso die Protonen der Ethylenglycolendgruppen. ¹H-NMR-Signale von aromatischen Protonen der Struktur 3 konnten nicht detektiert werden.

### 7. Erfindungsgemäße Herstellung von Poly-3-hydroxypropionat durch carbonylierende Umsetzung von in Diglyme gelöstem Ethylenoxid im Beisein eines Co umfassenden Katalysatorsystems und Fällung desselben unter Verwendung einer wässrigen Essigsäurelösung als Fällflüssigkeit (Beispiel 3)

Vergleichsbeispiel 3 wurde wiederholt. Die 106,8 g der als Produktgemisch A erhaltenen rotbraunen wässrigen Suspension wurden jedoch mit 534 g einer 12,5 gew.-%igen Lösung von Essigsäure in Wasser versetzt, deren Temperatur 50 °C betrug. Das resultierende wässrige Gemisch wurde unter Einperlung von Luft (25 °C, 5 l/h) während 1 h bei einer Temperatur von 45 °C gerührt (700 U/min). Dabei erfolgte eine Farbänderung. Der Farbton der flüssigen Phase wechselte nach hellrosa und die Farbe des in selbiger suspendierten Feststoffs nach cremefarben.

Das in der Suspension suspendierte Poly-3-hydroxypropionat wurde abfiltriert und 72 h bei reduziertem Druck getrocknet ( 10 hPa, Trocknungstemperatur: 60 °C).
Die so vom Produktgemisch A abgetrennten 18 g Poly-3-hydroxypropionat enthielten auf das Gewicht ihrer Masse bezogen nur noch 0,05 Gew.-% an Co. Das zugehörige relative gewichtsmittlere Molekulargewicht Mw betrug 5120. Die 18 g abgetrenntes Poly-3-hydroxy-propionat sind 94 % der theoretisch möglichen maximalen Ausbeute.

### 8. Erfindungsgemäße Herstellung von Poly-3-hydroxypropionat durch carbonylierende Umsetzung von in Diglyme gelöstem Ethylenoxid im Beisein eines Co umfassenden Katalysatorsystems und Fällung desselben unter Verwendung einer wässrigen Essigsäurelösung als Fällflüssigkeit (Beispiel 4)

Vergleichsbeispiel 3 wurde wiederholt. Anstelle von 0,87 g 3-Hydroxypyridin wurden jedoch 0,78 g Pyridin als alleiniger co-Katalysator B eingesetzt. Außerdem betrug die eingesetzte Menge an Ethylenoxid nur 10,07 g.

Die 103,6 g der als Produktgemisch A anfallenden wässrigen Suspension (rotbraune Flüssigphase mit orangerotem Feststoff) wurde mit 518 g einer 12,5 gew.-%igen Lösung von Essigsäure in Wasser versetzt, deren Temperatur 50 °C betrug. Das resultierende wässrige Gemisch wurde unter Einperlung von Luft (25 °C, 5 l/h) während 1 h bei einer Temperatur von 45 °C gerührt (700 U/min). Dabei erfolgte eine Farbänderung. Der Farbton der flüssigen Phase wechselte nach hellrosa und die Farbe des in selbiger suspendierten Feststoffs nach cremefarben.

Das in der Suspension suspendierte Poly-3-hydroxypropionat wurde abfiltriert und 72 h bei reduziertem Druck getrocknet (10 hPa, Trocknungstemperatur: 60 °C).

Die so vom Produktgemisch A abgetrennten 8,0 g Poly-3-hydroxypropionat enthielten auf das Gewicht ihrer Masse bezogen nur noch 0,12 Gew.-% an Co. Das zugehörige relative gewichtsmittlere Molekulargewicht Mw war 4022. Die 8,0 g abgetrenntes Poly-3-hydroxypropionat sind 48,7 % der theoretisch möglichen maximalen Ausbeute.

### 9. Nicht erfindungsgemäße Herstellung von Poly-3-hydroxypropionat durch carbonylierende Umsetzung von in Diglyme gelöstem Ethylenoxid im Beisein eines Co umfassenden Katalysatorsystems (Vergleichsbeispiele 5a, 5b, 5c, und 5d)

Vergleichsbeispiel 2 wurde wiederholt, jedoch mit nachfolgenden geringfügigen Unterschieden:
- die Einsatzmenge an Dicobaltoctacarbonyl war 16,1 g;
- die Einsatzmenge an Diglyme war 944,1 g;
- die Einsatzmenge an Ethylenoxid war 99,8 g.

Im Reaktionsraum befanden sich jeweils ca. 1112,1 g einer dunkelrot-braunen Lösung als Produktgemisch A.

Von diesem Produktgemische A wurden vier identische Teilmengen zu jeweils 250 g entnommen.
Die erste Teilmenge wurde für 12 h in einem eine Temperatur von 7 °C aufweisenden Kühlraum sich selbst überlassen. Das dabei ausfallende Poly-3-hydroxypropionat wurde abfiltriert und der resultierende Filterkuchen bei 10 hPa und 60 °C während 10 h getrocknet. (= Vergleichsbeispiel 5a).

Die zweite Teilmenge wurde mit 75 g Methanol (25 °C) als Fällflüssigkeit versetzt und das resultierende Gemisch für 12 h in einem eine Temperatur von 7 °C aufweisenden Kühlraum sich selbst überlassen. Das dabei ausfallende Poly-3-hydroxypropionat wurde abfiltriert und der resultierende Filterkuchen bei 10 hPa und 60 °C während 10 h getrocknet.

### (= Vergleichsbeispiel 5b).

Beim Vergleichsbeispiel 5c wurde wie beim Vergleichsbeispiel 5b verfahren, der Filterkuchen vorab seiner Trocknung jedoch noch mit 50 g Methanol, dessen Temperatur 60 °C betrug, gewaschen (das Methanol wurde durch den Filterkuchen hindurch gesaugt).

Beim Vergleichsbeispiel 5d wurde wie beim Vergleichsbeispiel 5c verfahren, die verwendete Menge der Waschflüssigkeit Methanol betrug jedoch 75 g.

Die nachfolgende Tabelle 4 zeigt für das jeweilige Vergleichsbeispiel die Masse des jeweils abgetrennten Poly-3-hydroxypropionats (P3HP), die bezogen auf das Gewicht dieser Masse noch enthaltene Menge an Co (in Gew.-%) und das jeweils zugehörige relative gewichtsmittlere Molekulargewicht M_{w} sowie die zugehörige Polydispersität Q. Außerdem weist die Tabelle 4 noch die Farbe des jeweils abgetrennten Poly-3-hydroxypropionat aus.

**Tabelle 4**

| **Vgl.Bsp.** | **Co (Gew.-%)** | **P3HP (g)** | **Farbe P3HP** | **M_{W}(Q)** |
|---|---|---|---|---|
| 5a | 3,0 | 3,4 | rotbraun | 6221 (1,9) |
| 5b | 2,5 | 22,4 | altrosa-braun | 6610 (1,8) |
| 5c | 2,2 | 21,5 | altrosa | 6742 (1,8) |
| 5d | 1,8 | 18,6 | sandfarben | 7584 (1,9) |

### 10. Erfindungsgemäße Herstellung von Poly-3-hydroxypropionat durch carbonylierende Umsetzung von in Diglyme gelöstem Ethylenoxid im Beisein eines Co umfassenden Katalysatorsystems und unter Verwendung einer wässrigen Essigsäurelösung als Fällflüssigkeit (Beispiel 5)

Vergleichsbeispiel 5a wurde wiederholt, zu den 250 g an Produktgemisch A wurden jedoch noch 50 g einer 12,5 gew.-%igen Lösung von Essigsäure in Wasser hinzugefügt, bevor das resultierende Gemisch für 12 h in einem eine Temperatur von 7 °C aufweisenden Kühlraum sich selbst überlassen wurde. Das dabei ausgefallene Poly-3-hydroxypropionat wurde abfiltriert und der resultierende Filterkuchen noch mit 50 g Methanol gewaschen (dieses hatte eine Temperatur von 25 °C und wurde durch den Filterkuchen hindurchgesaugt).

Nachfolgende Tabelle 5 zeigt die Masse des abgetrennten Poly-3-hydroxypropionats (P3HP), die bezogen auf das Gewicht dieser Masse noch enthaltene Menge an Co (in Gew.-%), das zugehörige relative gewichtsmittlere Molekulargewicht Mwsowie die zugehörige Polydispersität Q. Außerdem weist die Tabelle 5 noch die Farbe des abgetrennten Poly-3-hydroxypropionats aus.

**Tabelle 5**

| **Bsp.** | **Co (Gew.-%)** | **P3HP (g)** | **Farbe P3HP** | **M_{W}(Q)** |
|---|---|---|---|---|
| 5 | 0,76 | 19,9 | hellrosa | 6651 (1,6) |

### 11. Erfindungsgemäße Herstellung von Poly-3-hydroxypropionat durch carbonylierende Umsetzung von in Diglyme gelöstem Ethylenoxid im Beisein eines Co umfassenden Katalysatorsystems und unter Verwendung verschiedener wässriger Lösungen als Fäll- und Waschflüssigkeiten (Beispiele 6a, 6b, 6c, und 6d)

Vergleichsbeispiel 5 wurde identisch wiederholt und von dem dabei resultierenden Produktgemisch A wurden vier identische Teilmengen zu jeweils 250 g entnommen.

Jede Teilmenge wurde mit 75 g einer wässrigen Fällflüssigkeit versetzt und das jeweils resultierende Gemisch für 12 h in einem eine Temperatur von 7 °C aufweisenden Kühlraum sich selbst überlassen.

Das dabei jeweils ausfallende Poly-3-hydroxypropionat wurde abfiltriert und der resultierende Filterkuchen mit 75 g der als Fällflüssigkeit verwendeten Flüssigkeitssorte gewaschen (die Temperatur der Waschflüssigkeit war jeweils 25 °C, die Waschflüssigkeit wurde jeweils durch den Filterkuchen hindurch gesaugt).

Abschließend wurde der gewaschene Filterkuchen jeweils bei 10 hPa und 60 °C während 72 h getrocknet.
Die nachfolgende Tabelle 6 zeigt die für das jeweilige Beispiel verwendete wässrige Fäll-/Waschflüssigkeit FF, die Masse des jeweils abgetrennten Poly-3-hydroxypropionats (P3HP), die bezogen auf das Gewicht dieser Masse jeweils noch enthaltene Menge an Co (in Gew.-%) und das jeweilige zugehörige relative gewichtsmittlere Molekulargewicht Mw sowie die jeweilige Polydispersität Q. Außerdem weist die Tabelle 6 noch die Farbe des jeweils abgetrennten Poly-3-hydroxypropionats aus.

**Tabelle 6**

| **Bsp.** | **FF** | **Co (Gew.-%)** | **P3HP (g)** | **Farbe P3HP** | **M_{W}(Q)** |
|---|---|---|---|---|---|
| 6a | Wasser | 1,1 | 23,4 | creme-beige | 5684 (1,8) |
| 6b | 15 gew.-%ige wässrige Essigsäurelösung | 0,3 | 22,3 | rosa | 6141 (1,7) |
| 6c | 15 gew.-%ige wässrige Ammoniaklösung | 1,0 | 16,2 | himbeerrot | 4052 (1,7) |
| 6d | 2 gew.-%ige wässrige Lösung von Tetranatrium-ethylendiamintetraacetat | 1,0 | 10,2 | grün-braun | 6653 (1,7) |

### 12. Nicht erfindungsgemäße Herstellung von Poly-3-hydroxypropionat durch ringöffnende Polymerisation von β-Propiolacton in Abwesenheit von Co (Vergleichsbeispiel 6; die Synthese erfolgte in Anlehnung an die US 4,357,462 A und an "Die Polymerisation von Lactonen, Teil 1: Homopolymerisation 4-, 6- und 7-gliedriger Lactone mit kationischen Initiatoren" in "Die Makromolekulare Chemie - New York - Hüthig & Wepf Verlag, Vol. 56, 1962, Seiten 179 ff")

In 300 ml Methylenchlorid (= Lösungsmittel; Lieferfirma: BASF SE; Spezifikation: Reinheit 98-100 %), das über Molekularsieb (3Ä) als Trocknungsmittel gelagert worden war, wurde 1 ml Bortrifluorid-Etherat (= Katalysator; BF₃ x (CH₃-CH₂-O-CH₂-CH₃)₂; Lieferfirma: Fluka; Spezifikation: purum, Bestellnummer: 15719) gelöst (in einem 3-Halskolben aus Glas mit 750 ml Innenvolumen, es wurde magnetisch gerührt, die Innentemperatur war 20 °C).

Mit einem Siliconölbad wurde die Lösung zum Sieden gebracht (bei Normaldruck). Anschließend wurden zu der unter Rückfluss siedenden Lösung unter Rühren 24,9 g β-Propiolacton (Lieferfirma: Alfa Aesar; Spezifikation: 97 %, Bestellnummer: B23197, LOT 10140573) innerhalb von 20 min kontinuierlich zugetropft.

Nach beendeter Zugabe wurde das Reaktionsgemisch unter Rühren noch 8 h unter Rückfluss gehalten. Während der voranschreitenden Reaktion änderte die Lösung ihre Farbe von farblos über gelb zu orange.

Danach wurde das Lösungsmittel bei vermindertem Druck und einer Temperatur des Ölbads von 65 °C innerhalb von 30 min unter Rühren destillativ entfernt.

Es verblieben 27,2 g eines orangefarbenen Öls, das auf 25 °C abgekühlt wurde und bei dieser Temperatur wachsartig erstarrte. Zur Abtrennung des Katalysatorsystems wurden 400 ml Methanol (25 °C) hinzugegeben, die Temperatur des Gemischs auf 50 °C erwärmt und das Gemisch bei dieser Temperatur 1 h und 50 min gerührt, bis sich der Feststoff vollständig gelöst hatte. Dann wurde die Lösung wieder auf 25 °C abgekühlt, wobei ein farbloser Niederschlag ausfiel.

Dieser wurde abfiltriert und der Filterkuchen zweimal hintereinander mit jeweils 10 ml Methanol gewaschen (die Temperatur des Methanols war 25 °C; das Methanol wurde durch den Filterkuchen gesaugt) und dann für 8 h bei 25 °C und 10 hPa getrocknet. Es verblieben 12,4 g eines farblosen Pulvers. Sein gewichtsmittleres relatives Molekulargewicht M_{w} war 3000, bei einer Polydispersität Q von 1,4.

Die zugehörigen ¹H-, ¹³C-NMR-Spektren sowie das ATR-FT-IR Spektrum entsprachen Poly-3-hydroxypropionat mit einer Reinheit von > 95 Gew.-%.

Die ¹H- und ¹³C-NMR Spektren wurden an einem DRX 500 FT-NMR Spektrometer der Firma Bruker an Lösungen des Poly-3-hydroxypropionats in CDCl3 aufgenommen. Die Magnetfeldstärke entsprach einer ¹H-Trägerfrequenz von 500 MHz.

Die ATR-Infrarotspektren wurden mit einem Vertex 70 Spektrometer der Firma Bruker mit ATR ("attenuated total reflection") und der Methode der FT-IR-Spektroskopie aufgenommen. Untersucht wurde das feste Poly-3-hydroxpropionat. Die Proben wurden zu diesem Zweck zusätzlich für 12 h bei 60 °C und 10 hPA getrocknet und anschließend fein pulverisiert, um einen optimalen Kontakt mit dem ATR-Kristall (in dem die Totalreflexion erfolgte) zu ermöglichen.

### 13. Nicht erfindungsgemäße Herstellung von Poly-3-hydroxypropionat durch ringöffnende Polymerisation von β-Propiolacton in Abwesenheit von Co (Vergleichsbeispiel 7; die Synthese erfolgte in Anlehnung an die US 4,357,462 A und an "Die Polymerisation von Lactonen, Teil 1: Homopolymerisation 4-, 6- und 7-gliedriger Lactone mit kationischen Initiatoren" in "Die Makromolekulare Chemie - New York - Hüthig & Wepf Verlag, Vol. 56, 1962, Seiten 179 ff")

In 150 ml Toluol (= Lösungsmittel; Lieferant: BASF SE; Spezifikation: 99,9 %), das über Molekularsieb (3Ä) als Trocknungsmittel gelagert worden war, wurden 0,5 mg Titantetrachlorid (= Katalysator; Lieferfirma: Acros, Spezifikation: 99,9 %, Bestellnummer: 197231000) gelöst (in einem 3-Halskolben aus Glas mit 250 ml Innenvolumen, es wurde magnetisch gerührt, die Innentemperatur war 20 °C).

Zu der erhaltenen orangefarbenen Lösung wurden 5 g β-Propiolacton unter Rühren so langsam zugetropft, dass die Temperatur der Reaktionslösung nicht über 25 °C stieg. Nach beendeter Zugabe wurde das Reaktionsgemisch unter Rühren mit einem Siliconölbad auf 80 °C erwärmt und 1 h bei dieser Temperatur gehalten, wobei sich eine zweite, orangefarbene flüssige ölige Phase ausbildete. Die Temperatur des Reaktionsgemischs wurde auf 100 °C erhöht und das Gemisch bei dieser Temperatur weitere 4 h gerührt. Dann wurde auf 25 °C abgekühlt, wobei die separierte orangefarbene ölige Phase wachsartig erstarrte.

Durch Dekantieren wurde die flüssige Toluolphase vom Wachs abgetrennt und verworfen. Dann wurden zu dem orangefarbenen Wachs als Trennmittel 100 ml Methanol (25 °C) hinzugefügt, das Gemisch auf 50 °C erwärmt und bei dieser Temperatur 1 h gerührt, bis der Feststoff vollständig gelöst war. Schließlich wurde die Lösung wieder auf 25 °C abgekühlt, wobei ein beiger Niederschlag ausfiel.

Dieser wurde abfiltriert und zweimal hintereinander mit jeweils 10 ml Methanol (25 °C) gewaschen (das Methanol wurde durch den Filterkuchen gesaugt).

Der verbleibende Filterkuchen wurde bei 60 °C und 300 Pa für 60 h getrocknet. Es verblieben 3,4 g eines schwach gelblichen Pulvers. Sein gewichtsmittleres relatives Molekulargewicht Mwwar 7200, bei einer Polydispersität Q von 1,8.

Die zugehörigen ¹H-, ¹³C-NMR-Spektren sowie das ATR-FT-IR-Spektrum entsprachen Poly-3-hydroxypropionat mit einer Reinheit von >95 Gew.-%.

### 14. Thermolyse des nicht erfindungsgemäß hergestellten Poly-3-hydroxypropionats (P3HP) aus Vergleichsbeispiel 6 (Vergleichsbeispiel 8)

a) Die aus Glas gefertigte Spaltvorrichtung (die Thermolysezone A) bestand aus einem Spaltrundkolben (25 ml Innenvolumen, drei Hälse) mit einer diesem aufgesetzten Destillationsbrücke mit Thermometer, Liebigkühler, einem Produktkolben (10 ml Innenvolumen, ein Hals) und einem Schlauchanschluss für Abgas, der zur Atmosphäre offen war.
   In den Spaltrundkolben wurden 3,0 g des Poly-3-hydroxypropionats aus Vergleichsbeispiel 7 eingewogen. Über den zweiten Hals des Spaltkolbens wurde diesem während der gesamten Thermolyse ein Strom aus molekularem Stickstoff (≥ 99,9 Vol.-% N₂; Stromstärke: 1,4 l/h; Temperatur: 25 °C) als Strippgas zugeführt. Dieser durchströmte die Spaltvorrichtung und verließ diese wieder als Bestandteil des aus selbigem heraus über eine Kühlfalle, deren Temperatur bei - 78 °C gehalten wurde, geführten Abgases über den Abgasschlauch. Der mit dem P3HP befüllte Spaltkolben wurde bis zum mittleren Hals in ein auf 180 °C vorgeheiztes Siliconölbad abgesenkt und bei einem Arbeitsdruck von 1,0133 · 105 Pa (Normaldruck) durch das Ölbad erwärmt. Mit einem Magnetrührer wurde der Inhalt des Spaltkolbens gerührt.
   Als die Temperatur im Spaltkolben 60 °C erreichte, begann das P3HP zu schmelzen. Als die Innentemperatur 80 °C erreichte, war das Poly-3-hydroxypropionat vollständig geschmolzen.
   Nach Erreichen der Innentemperatur von 175 °C wurde diese unter Rühren für 300 min beibehalten.
   Der Liebigkühler wurde mit Wasser, das eine Zuströmtemperatur von 20 °C aufwies, im Gegenstrom gekühlt.
   Vom Stickstoffstrom transportierte kondensierbare Spaltprodukte würden im Liebigkühler kondensiert und das Kondensat im Produktkolben, der ebenfalls bei einer Temperatur von 20 °C gehalten wurde, aufgefangen.
   Innerhalb der vorgenannten 300 min fiel kein Kondensat im Produktkolben an.
b) Eine Probe von 34,86 mg des Poly-3-hydroxypropionats aus Vergleichsbeispiel 6 wurde in einen Tiegel aus Al₂O₃ eingewogen und simultan ihr Verhalten bei Temperaturerhöhung mit der Methode der Thermogravimetrie und mit der Methode der Dynamischen Differenzkalorimetrie untersucht ("simultane TG-DSC Analyse").

Die Untersuchung erfolgte mit einer Thermischen Analyse-Apparatur "NETZSCH STA 449 F3 Jupiter^{®}" der Firma Netzsch Gerätebau GmbH.
Mittels FT-IR-Spektroskopie wurde das bei der mit der thermischen Analyse einhergehenden Thermolyse gebildete Spaltgas auf seine Hauptkomponenten untersucht.

Im Rahmen der Untersuchung wurde die Probe zunächst für 10 min auf 35 °C temperiert und dann unter einem Ar-Strom (40 ml/min) die Probentemperatur mit der konstanten Geschwindigkeit von 5 K/min auf 610 °C erhöht.
Detektiert wurden als Funktion der Temperatur die Probenmasse und der Wärmestrom durch die Probe (d.h., die Dynamische Differenzkalorimetrie war als Dynamische Wärmestromdifferenzkalorimetrie ausgeführt).

Das erhaltene Thermogramm wies unter Einbezug der FT-IR-Spektroskopie die folgenden drei endothermen Prozesse aus:
1. Das Schmelzen der P3HP ohne Masseverlust;
   onset-Temperatur (oTs): 70,1 °C;
   peak-Temperatur (pTs): 93,6 °C.
   oTs = die Temperatur, bei der das Schmelzen der Probe nachweislich einsetzt; pTs = die Temperatur, bei der der Schmelzvorgang seine höchste Rate aufweist;
2. Thermolyse der Probe zu Acrylsäure;
   onset-Temperatur (oT_{T}): 286,5 °C;
   peak-Temperatur (pT_{T}): 340,0 °C;
   oT_{T} = die Temperatur, bei der die Thermolyse nachweislich einsetzt;
   pT_{T} = die Temperatur, bei der die Thermolyse ihre maximale Spaltrate aufweist;
   Masseverlust: 98,8 % der Ausgangsmasse;
   das Spaltgas enthielt als Hauptkomponente Acrylsäure und Spuren von CO₂.
3. Zersetzung der Restmasse oberhalb 400 °C;
   keine onset- oder peak-Temperatur bestimmbar, da bei 610 °C das Ende des Messbereichs erreicht war;
   Masseverlust bis zum Ende des Messbereichs: 0,5 % der Ausgangsmasse.

### 15. Thermolyse des nicht erfindungsgemäß hergestellten Poly-3-hydroxpropionats (P3HP) aus Vergleichsbeispiel 6, im Beisein von 3-Hydroxypyridin als Spaltkatalysator (Vergleichsbeispiel 9)

Es wurde wie im Versuch "14.a)" verfahren, jedoch mit dem Unterschied, dass nach dem Aufschmelzen des P3HP 97 mg 3-Hydroxypyridin zur Schmelze gegeben wurden. Bereits 15 min nach Erreichen der Innentemperatur von 175 °C im Spaltkolben fiel im Produktkolben das erste Kondensat an (der Produktkolben enthielt in diesem Versuch "15." und in allen nachfolgenden Thermolyseversuchen keinen Polymerisationsinhibitor zugesetzt). Nach insgesamt 90 min bei 175 °C Innentemperatur wurde die im Spaltkolben noch vorhandene Restschmelze fest. Daraufhin wurde der Spaltversuch abgebrochen. In der Destillationsbrücke anhaftende Kondensattropfen wurden durch Erwärmen derselben mit einem Heißluftgebläse (Föhn) verdampft, im Liebigkühler verflüssigt und im Produktkolben aufgefangen.

Die im Produktkolben enthaltene Kondensatmenge war 2,48 g.
Gemäß gaschromatographischer Analyse enthielt das Kondensat (bezogen auf sein Gewicht) 95,5 % Gew.-% Acrylsäure, 3,6 % Gew.-% Diacrylsäure (Michael Addukt) und 0,8 Gew.-% höhere Michael-Addukte der Acrylsäure an sich selbst.
Aldehyde waren im Kondensat nicht nachweisbar. Das Kondensat enthielt kein 3-Hydroxy-pyridin.

Die Masse des verbliebenen hellbraunen, klebrigen Rückstands im Spaltkolben betrug 330 mg (11 Gew.-% der Einsatzmenge an P3HP).
Die vom Strippgas mitgestrippten Michael-Addukte können hier (und in allen nachfolgenden Fällen) bei Bedarf in einfacher Weise dadurch zurückgehalten werden, dass der Stoffstrom über eine unter Rückfluss betriebene Rektifikationskolonne (z.B. eine Vigreux-Kolonne) zum Produktkolben geführt wird. Die Spaltausbeute an Acrylsäure kann dabei entsprechend erhöht werden.

### 16. Thermolyse des nicht erfindungsgemäß hergestellten Poly-3-hydroxypropionats (P3HP) aus Vergleichsbeispiel 6, im Beisein von Pentamethylethylentriamin (Lupragen^{®} N301) als Spaltkatalysator (Vergleichsbeispiel 10)

a) Es wurde wie im Versuch "14.a)" verfahren, jedoch mit dem Unterschied, dass nach dem Aufschmelzen des P3HP 87 mg Pentamethylethylentriamin (Lieferfirma: BASF SE; Spezifikation: >98 %, Handelsname: Lupragen^{®} N301) zur Schmelze gegeben wurden. Bereits 15 min nach Erreichen der Innentemperatur von 175 °C im Spaltkolben fiel im Produktkolben das erste Kondensat an. Nach insgesamt 120 min bei 175 °C Innentemperatur wurde die im Spaltkolben noch vorhandene Restschmelze fest (klebrigfest). Daraufhin wurde der Spaltversuch abgebrochen. In der Destillationsbrücke anhaftende Kondensattropfen wurden durch Erwärmen derselben mit einem Heißluftgebläse (Föhn) verdampft, im Liebigkühler verflüssigt und im Produktkolben aufgefangen
   Die im Produktkolben enthaltene Kondensatmenge war 2,71 g.
   Das Kondensat erhielt 95,7 Gew.-% Acrylsäure, 3,3 Gew.-% Diacrylsäure (Michael-Addukt) und 0,5 Gew.-% höhere Michael-Addukte der Acrylsäure an sich selbst. Aldehyde waren im Kondensat nicht nachweisbar. Das Kondensat enthielt kein Pentamethylethylentriamin. Die Masse des verbliebenen hellbraunen klebrigen Rückstands im Spaltkolben betrug 150 mg (5 Gew.-% der Einsatzmenge an P3HP).
b) Es wurde wie im Versuch "14.b)" verfahren, jedoch mit dem Unterschied, dass die Probenmenge an P3HP 36,65 mg war und dieser Probenmenge vorab der Thermischen Analyse, bezogen auf ihr Gewicht, 0,68 Gew.-% Pentamethylentriamin zugesetzt wurden.

Das resultierende Thermogramm wies unter Einbezug der FT-IR-Spektroskopie die folgenden drei endothermen Prozesse aus.
1. das Schmelzen des P3HP ohne Masseverlust;
   onset-Temperatur: 69,6 °C;
   peak-Temperatur: 93,3 °C.
2. Thermolyse der Probe zu Acrylsäure;
   onset-Temperatur: 208,7 °C;
   peak-Temperatur: 259,7 °C;
   Masseverlust: 98,9 % der Ausgangsmasse;
   das Spaltgas enthält als Hauptkomponente Acrylsäure und Spuren von CO₂.
3. Zersetzung der Restmasse oberhalb von 300 °C;
   keine onset- und peak-Temperatur bestimmbar;
   Masseverlust bis zum Ende des Messbereichs: 0,3 % der Ausgangsmasse.

Bei einer Wiederholung der Versuche "14. a) und b)", des Versuchs "15." und der Versuche "16. a) und b)", jedoch unter Verwendung des nicht erfindungsgemäß hergestellten Poly-3-hydroxypropionats (P3HP) aus Vergleichsbeispiel 7 (anstelle aus Vergleichsbeispiel 6) als zu spaltendes Material, zeigte dieses ein dem nicht erfindungsgemäß hergestellten Poly-3-hydroxypropionat (P3HP) aus Vergleichsbeispiel 6 entsprechendes Spaltverhalten.

### 17. Thermolyse des nicht erfindungsgemäß hergestellten Poly-3-hyroxypropionats (P3HP) aus Versuch "6." (Vergleichsbeispiel 11)

In Versuch "6." wurde Vergleichsbeispiel 2 mehrfach wiederholt und durch Mischen von verschiedenen abgetrennten Fraktionen ein Poly-3-hyroxypropionat erzeugt, das auf das Gewicht seiner Masse bezogen noch 2 Gew.-% Co enthielt.
Dieses Poly-3-hyroxypropionat war Gegenstand dieses Versuchs 17.
a) Es wurde wie im Versuch "14.a)" verfahren, in den Spaltkolben wurden jedoch 3,0 g des 2 Gew.-% Co enthaltenden Poly-3-hydroxypropionats eingewogen. 30 Minuten nach Erreichen der Innentemperatur von 175 °C im Spaltkolben fiel im Produktkolben das erste Kondensat an. Nach insgesamt 90 min bei 175 °C Innentemperatur wurde die im Spaltkolben noch vorhandene Restschmelze extrem zäh-viskos, so dass der Spaltversuch abgebrochen wurde. In der Destillationsbrücke anhaftende Kondensattropfen wurden durch Erwärmen derselben mit einem Heißluftgebläse (Föhn) verdampft, im Liebigkühler verflüssigt und im Produktkolben aufgefangen.
   Die im Produktkolben enthaltene Kondensatmenge war 2,14 g.
   Das Kondensat enthielt 95,3 Gew.-% Acrylsäure, 3,7 Gew.-% Diacrylsäure (Michael-Addukt) und 0,5 Gew.-% höhere Michael-Addukte der Acrylsäure an sich selbst. Aldehyde waren im Kondensat nicht nachweisbar.
   Die Masse des im Spaltkolben verbliebenen dunkelbraunen, bei 25 °C glasartig spröden Rückstands betrug 710 mg (24 Gew.-% der Einsatzmenge an P3HP).
   Eine Elementaranalyse des Spaltrückstand ergab die folgenden, auf das Gewicht der Masse desselben bezogenen Gehalte: 12 Gew.-% Co, 46,6 Gew.-% C, 4,5 Gew.-% H, 2,9 Gew.-% N und 34 Gew.-% O.
   Dieses Ergebnis korreliert mit einem Stoffgemisch aus 12 Gew.-% Co, 19,7 Gew.-% 3-Hydroxypyridin und 68,3 Gew.-% eines Stoffes mit der Elementzusammensetzung 50,1 Gew.-% C, 5,1 Gew.-% H und 44,9 Gew.-% O. Letztere entspricht in befriedigender Weise der theoretischen Elementzusammensetzung von P3HP: 50,0 Gew.-% C, 5,59 Gew.-% H und 44,4 Gew.-% O.
   Eine Wiederholung des Versuchs "17.a)" unter Zusatz unterschiedlicher Mengen an Phenothiazin bzw. 4-Hydroxy-2,2,6,6-tetramethylpiperidin-1-N-oxyl als Polymerisationsinhibitoren führte weder zu einer Verbesserung der Ausbeute an Acrylsäure noch zu einer Abnahme des verbleibenden Spaltrückstands.
b) Es wurde wie im Versuch "14.b)" verfahren, jedoch mit dem Unterschied, dass es sich bei der untersuchten Probe um 37,70 mg des 2 Gew.-% Co enthaltenden Poly-3-hydro-xypropionats handelte.

Das resultierende Thermogramm wies unter Einbezug der FT-IR-Spektroskopie die folgenden drei endothermen Prozesse aus:
- 1.: das Schmelzen des P3HP (mit einem Masseverlust von 0,4 % der Einwaagemasse);
onset-Temperatur: 62,9 °C; peak-Temperatur: 76,0 °C.
- 2.: Thermolyse der Probe zu Acrylsäure;
onset-Temperatur: 204,3 °C;
peak-Temperatur: 235,1 °C;
Masseverlust: 86,0 % der Ausgangsmasse;
das Spaltgas enthielt als Hauptkomponente Acrylsäure und in Spuren CO₂ sowie Methan.
- 3.: Zersetzung der Restmasse oberhalb von 300 °C;
keine onset- oder peak-Temperatur bestimmbar;
Masseverlust bis zum Ende des Messbereichs: 4,7 % der Ausgangsmasse.

### 18. Thermolyse des nicht erfindungsgemäß hergestellten Poly-3-Hydroxypropionats (P3HP) aus Versuch "6.", im Beisein von Pentamethylethylentriamin (Vergleichsbeispiel 12)

a) Es wurde wie in Versuch "17.a)" verfahren, zusätzlich zu den 3,0 g des 2 Gew.-% Co enthaltenden Poly-3-hydroxypropionats wurden jedoch nach dessen Aufschmelzen noch 87 mg Pentamethylethylentriamin in den Spaltkolben gegeben. Bereits 15 min nach Erreichen der Innentemperatur von 175 °C im Spaltkolben fiel im Produktkolben das erste Kondensat an. Nach insgesamt 90 min bei 175 °C Innentemperatur wurde die im Spaltkolben noch vorhandene Restschmelze ausgeprägt zäh-viskos, so dass der Spaltversuch abgebrochen wurde. In der Destillationsbrücke anhaftende Kondensattropfen wurden durch Erwärmen derselben mit einem Heißluftgebläse (Föhn) verdampft, im Liebigkühler verflüssigt und im Produktkolben aufgefangen.
   Die im Produktkolben enthaltene Kondensatmenge war 2,21 g. Das Kondensat enthielt 96,1 Gew.-% Acrylsäure, 3,2 Gew.-% Diacrylsäure (Michael-Addukt) und 0,6 Gew.-% höhere Michael-Addukte der Acrylsäure an sich selbst. Aldehyde waren im Kondensat nicht nachweisbar. Das Kondensat enthielt kein Pentamethylethylentriamin.
   Die Masse des im Spaltkolben verbliebenen dunkelbraunen, bei 25 °C glasartig spröden Rückstands betrug 690 mg (23 Gew.-% der Einsatzmenge an P3HP). D.h., das als Spaltkatalysator zugegebene Pentamethylethylentriamin vermag im Beisein des Co im Vergleich mit Versuch "17.a)" den Spaltrückstand nicht wesentlich zu verringern.
b) Es wurde wie im Versuch "17.b)" verfahren, jedoch mit dem Unterschied, dass die Probenmenge an P3HP 35,43 mg des 2 Gew.-% Co enthaltenden Poly-3-hydroxypropionats war und dieser Probenmenge vorab der Thermischen Analyse, bezogen auf ihre Gewicht, 0,58 Gew.-% Pentamethylethylentriamin zugesetzt wurden.

Das resultierende Thermogramm wies unter Einbezug der FT-IR-Spektroskopie die folgenden drei endothermen Prozesse aus:
1. Das Schmelzen des P3HP (mit einem Masseverlust von 0,4 % der Einwaagemasse);
   onset-Temperatur: 62,6 °C;
   peak-Temperatur: 75,5 °C.
2. Thermolyse der Probe zu Acrylsäure;
   onset-Temperatur: 191,5 °C;
   peak-Temperatur: 222,6 °C;
   Masseverlust: 88,4 % der Ausgangsmasse;
   das Spaltgas enthielt als Hauptkomponente Acrylsäure und in Spuren CO₂ sowie Methan.
3. Zersetzung der Restmasse oberhalb von 290 °C;
   keine onset- und peak-Temperatur bestimmbar;
   Masseverlust bis zum Ende des Messbereichs: 4,6 % der Ausgangsmasse.
   D.h., das zugegebene Pentamethylethylentriamin erniedrigt im Vergleich mit Versuch "17.b)" trotz des Co-Gehalts die für die Thermolyse erforderliche Aktivierungsenergie erheblich.

### 19. Thermolyse des erfindungsgemäß hergestellten Poly-3-hydroxypropionats (P3HP) aus Beispiel 2 (Beispiel 7)

a) Es wurde wie in Versuch "14.a)" verfahren, in den Spaltkolben wurden jedoch 3,0 g des 0,2 Gew.-% Co enthaltenden Poly-3-hydroxypropionats aus Beispiel 2 eingewogen. 30 Minuten nach Erreichen der Innentemperatur von 175 °C im Spaltkolben fiel im Produktkolben das erste Kondensat an. Nach insgesamt 135 min bei 175 °C Innentemperatur wurde die im Spaltkolben noch vorhandene Restschmelze ausgeprägt zäh-viskos, so dass der Spaltversuch abgebrochen wurde. In der Destillationsbrücke anhaftende Kondensattropfen wurden durch Erwärmen derselben mit einem Heißluftgebläse (Föhn) verdampft, im Liebigkühler verflüssigt und im Produktkolben aufgefangen.
   Die im Produktkolben enthaltene Kondensatmenge war 2,51 g. Das Kondensat enthielt 95,6 Gew.-% Acrylsäure, 3,2 Gew.-% Diacrylsäure (Michael-Addukt) und 0,6 Gew.-% höhere Michael-Addukte der Acrylsäure an sich selbst. Aldehyde waren im Kondensat nicht nachweisbar.
   Die Masse des im Spaltkolben verbliebenen dunkelbraunen, bei 25 °C glasartig spröden Rückstands betrug 360 mg (12 Gew.-% der Einsatzmenge an P3HP).
b) Es wurde wie in Versuch "14.b)" verfahren, jedoch mit dem Unterschied, dass es sich bei der untersuchten Probe um 36,65 mg des 0,2 Gew.-% Co enthaltenden Poly-3-hydroxpropionats aus Beispiel 2 handelte.

Das resultierende Thermogramm wies unter Einbezug der FT-IR-Spektroskopie die folgenden drei endothermen Prozesse aus:
1. Das Schmelzen des P3HP ohne Masseverlust;
   onset-Temperatur: 60,9 °C;
   peak-Temperatur: 86,9 °C.
2. Thermolyse der Probe zu Acrylsäure;
   onset-Temperatur: 197,2 °C;
   peak-Temperatur: 236,4 °C;
   Masseverlust: 97,3 % der Ausgangsmasse;
   das Spaltgas enthielt als Hauptkomponente Acrylsäure und in Spuren CO₂.
3. Zersetzung der Restmasse oberhalb von 290 °C;
   keine onset- und peak-Temperatur bestimmbar;
   Masseverlust bis zum Ende des Messbereichs: 1,0 % der Ausgangsmasse.

### 20. Thermolyse des erfindungsgemäß hergestellten Poly-3-hydroxypropionats (P3HP) aus Beispiel 2, im Beisein von Pentamethylethylentriamin (Beispiel 8)

a) Es wurde wie in Versuch "19.a)" verfahren, zusätzlich zu den 3,0 g des 0,2 Gew.-% Co enthaltenden Poly-3-hydroxypropionats wurden jedoch nach dessen Aufschmelzen noch 87 mg Pentamethylethylentriamin in den Spaltkolben gegeben. Bereits 15 min nach Erreichen der Innentemperatur von 175 °C im Spaltkolben fiel im Produktkolben das erste Kondensat an. Nach insgesamt 90 min bei 175 °C Innentemperatur wurde die im Spaltkolben noch vorhandene Restschmelze ausgeprägt zäh-viskos, so dass der Spaltversuch abgebrochen wurde. In der Destillationsbrücke anhaftende Kondensattropfen wurden durch Erwärmen derselben mit einem Heißluftgebläse (Föhn) verdampft, im Liebigkühler verflüssigt und im Produktkolben aufgefangen.
   Die im Produktkolben enthaltene Kondensatmenge war 2,56 g. Das Kondensat enthielt 96,2 Gew.-% Acrylsäure, 2,9 Gew.-% Diacrylsäure (Michael-Addukt) und 0,5 Gew.-% höhere Michael-Addukte der Acrylsäure an sich selbst. Aldehyde waren im Kondensat nicht nachweisbar. Das Kondensat enthielt kein Pentamethylethylentriamin.
   Die Masse des im Spaltkolben verbliebenen dunkelbraunen, bei 25 °C glasartig spröden Rückstands betrug 240 mg (8 Gew.-% der Einsatzmenge an P3HP).
b) Es wurde wie in Versuch "19.b)" verfahren, jedoch mit dem Unterschied, dass die Probenmenge an P3HP 35,02 mg des 0,2 Gew.-% Co enthaltenden Poly-3-hydroxypropionats aus Beispiel 2 war und dieser Probenmenge vorab der Thermischen Analyse, bezogen auf ihre Gewicht, 0,56 Gew.-% Pentamethylethylentriamin zugesetzt wurden. Das resultierende Thermogramm wies unter Einbezug der FT-IR-Spektroskopie die folgenden drei endothermen Prozesse aus:
   1. das Schmelzen des P3HP ohne Masseverlust;
      onset-Temperatur: 60,6 °C;
      peak-Temperatur: 84,8 °C.
   2. Thermolyse der Probe zu Acrylsäure;
      onset-Temperatur: 192,9 °C;
      peak-Temperatur: 228,3 °C;
      Masseverlust: 97,4 % der Ausgangsmasse;
      das Spaltgas enthielt als Hauptkomponente Acrylsäure und in Spuren CO₂.
   3. Zersetzung der Restmasse oberhalb von 290 °C;
      keine onset- und peak-Temperatur bestimmbar;
      Masseverlust bis zum Ende des Messbereichs: 1,2 % der Ausgangsmasse.

### 21. Thermolyse eines Gemischs von zwei nicht erfindungsgemäß hergestellten Poly-3-hydroxy-propionaten (P3HP): das P3HP aus Vergleichsbeispiel 6 und das bezogen auf das Gewicht seiner Masse 2 Gew.-% Co enthaltende P3HP aus Versuch "6." (Vergleichsbeispiel 13)

Es wurde wie in Versuch "14.a)" verfahren, in den Spaltkolben wurde jedoch ein Gemisch aus 2,5 g des P3HP aus Vergleichsbeispiel 6 und 2,5 g des bezogen auf das Gewicht seiner Masse 2 Gew.-% Co enthaltenden P3HP's aus Versuch "6." eingewogen. 30 Minuten nach Erreichen der Innentemperatur von 175 °C im Spaltkolben fiel im Produktkolben das erste Kondensat an. Nach insgesamt 120 min bei einer Innentemperatur von 175 °C wurde die im Spaltkolben noch vorhandene Restschmelze ausgeprägt zäh-viskos, so dass der Spaltversuch abgebrochen wurde. In der Destillationsbrücke anhaftende Kondensattropfen wurden durch Erwärmen derselben mit einem Heißluftgebläse (Föhn) verdampft, im Liebigkühler verflüssigt und im Produktkolben aufgefangen.

Die im Produktkolben enthaltene Kondensatmenge war 4,15 g. Das Kondensat enthielt 96,8 Gew.-% Acrylsäure, 2,7 Gew.-% Diacrylsäure (Michael-Addukt) und 0,3 Gew.-% höhere Michael-Addukte der Acrylsäure an sich selbst. Aldehyde waren im Kondensat nicht nachweisbar.

Die Masse des im Spaltkolben verbliebenen dunkelbraunen, bei 25 °C glasartig spröden Rückstands betrug 580 mg (12 Gew.-% der Einsatzmenge an P3HP).

### 22. Thermolyse des nicht erfindungsgemäß hergestellten Poly-3-hydroxypropionats (P3HP) aus Vergleichsbeispiel 6, im Beisein von N-Benzylamin als Spaltkatalysator (Vergleichsbeispiel 14)

Es wurde wie im Versuch "14.a)" verfahren, jedoch mit dem Unterschied, dass nach dem Aufschmelzen des P3HP 90 mg N-Benzylamin (Lieferfirma: Sigma-Aldrich; Spezifikation: >99 %, Artikelnummer: 185701) zur Schmelze gegeben wurden. Nach Erreichen der Innentemperatur von 175 °C wurde diese unter Rühren noch 300 min beibehalten. Dann wurde der Thermolyseversuch abgebrochen.

Innerhalb der vorgenannten 300 min fiel kein Kondensat im Produktkolben an.

Der im Spaltkolben verbliebene Inhalt erstarrte bei einer Innentemperatur von 55 °C zu einem leicht beige gefärbten Wachs. Die Menge des Wachses war 3,06 g (99,0 Gew.-% der Einsatzmenge von P3HP und Benzylamin). Das gewichtsmittlere relative Molekulargewicht Mw des P3HP-Anteils war nach dem Experiment 1900, bei einer Polydispersität Q von 2,7.

### 23. Nachweis der Abtrennbarkeit von als Spaltkatalysator mitverwendetem Pentamethylethylentriamin aus dem Spaltrückstand von Versuch "16.a)" durch gaschromatographische Auftrennung von bei der thermischen Behandlung dieses Spaltrückstands gasförmig entweichenden Bestandteilen und anschließender Aufklärung der Struktur dieser Bestandteile durch Massenspektrometrie (Methode der programmierten Pyrolyse GC/MS-Kopplung) und FT-IR

Die thermische Behandlung des Spaltrückstands erfolgte in einem kreiszylindrischen Tiegel aus V2A-Stahl (Höhe: 6,2 mm; Wanddicke: 0,2 mm; Außendurchmesser: 2,5 mm). Die in den Tiegel eingewogene Probenmenge an Spaltrückstand von Versuch "16.a)" war 0,23 mg. Der Tiegel wurde zentriert in ein kreiszylindrisches Rohr aus Quarzglas eingebracht (25 mm Höhe; 5 mm Innendurchmesser; 0,5 mm Wanddicke). Das Quarzglasrohr konnte von außen elektrisch beheizt werden.

Durch das Rohr aus Quarzglas wurde ein Gasstrom aus He geführt (20 ml/min, Eintrittstemperatur in das Quarzglasrohr = 25 °C)), der in Richtung des im Rohr befindlichen Tiegels strömte (die Öffnung des Tiegels blickte in Richtung des He-Stroms), aus selbigem gegebenenfalls austretende gasförmige Bestandteile aufnahm und in Strömungsrichtung in eine gaschromatographische Trennsäule förderte. Die Länge der Trennsäule betrug 30 m, ihr Innendurchmesser war 0,25 mm. Als stationäre Phase wies sie einen Film der Schichtdicke 1 µm aus Polydimethylsiloxan auf (diese Säule war von der Fa. Agilent Technologies unter dem Typ "HP-1 ms" käuflich erworben worden).

Die Starttemperatur der elektrischen Beheizung des Quarzrohres betrug 100 °C. Diese wurde mit einer Rampe von 10 °C/min auf 400 °C erhöht und anschließend bei dieser Temperatur gehalten.

Bis zum Erreichen der 400 °C wurden die mit dem He-Strom in die Trennsäule geförderten, aus der im Tiegel thermisch behandelten Probe gasförmig austretenden Bestandteile, am Eintritt in selbige kryofokussiert. Zu diesem Zweck befand sich die gesamte Trennsäule in einem mit flüssigem Stickstoff gefüllten Dewargefäß.

Anschließend wurde die Temperatur der gesamten Trennsäule auf 40 °C erhöht, und bei dieser Temperatur 2 min gehalten. Dann wurde die Temperatur der gesamten Säule mit einer Heizrate von 6 °C/min bis auf eine Endtemperatur von 320 °C erhöht. Abschließend wurde diese Endtemperatur noch 13 min aufrechterhalten. Während der gesamten Zeit strömte der He-Strom durch das den Tiegel enthaltende beheizte Rohr aus Quarzglas in die Trennsäule und aus der Trennsäule in ein Massenspektrometer.
Zusätzlich wurde in einem weiteren Versuch der aus der Trennsäule ausströmende Gasstrom mittels FT-IR analysiert.

Pentamethylethylentriamin konnte als Hauptbestandteil im He-Strom zweifelsfrei nachgewiesen werden.

## Patentansprüche

1. Verfahren zur Herstellung von Acrylsäure aus Ethylenoxid und Kohlenmonoxid, das wenigstens folgende Verfahrensschritte umfasst:
- eine carbonylierende Umsetzung von in einem nicht protischen Lösungsmittel gelöstem Ethylenoxid mit Kohlenmonoxid bei erhöhtem Druck und erhöhter Temperatur in Anwesenheit eines Katalysatorsystems, das wenigstens eine Kobaltquelle umfasst, in einer Reaktionszone A unter Erhalt eines Poly-3-hydroxypropionat enthaltenden Produktgemischs A,
- eine Abtrennung von Poly-3-hydroxypropionat vom Produktgemisch A in einer Trennzone A, und
- eine Thermolyse von in der Trennzone A abgetrenntem Poly-3-hydroxypropionat in einer Thermolysezone A unter Bildung von Acrylsäure,
**dadurch gekennzeichnet, dass** die Abtrennung von Poly-3-hydroxypropionat vom Produktgemisch A in der Trennzone A wenigstens eine der nachfolgenden Verfahrensmaßnahmen umfasst:
- einen Zusatz von Wasser und/oder einer wässrigen Lösung als wässrige Fällflüssigkeit zu einer oder mehreren Teilmengen des Produktgemischs A und/oder zur Gesamtmenge des Produktgemischs A, um in einer Teilmenge des Produktgemischs A oder in der Gesamtmenge des Produktgemischs A gelöst enthaltenes Poly-3-hydroxypropionat auszufällen;
- ein Waschen von vom Produktgemisch A in der Trennzone A abgetrenntem Poly-3-hydroxypropionat mit Wasser und/oder mit einer wässrigen Lösung als wässrige Waschflüssigkeit.

2. Verfahren nach Anspruch 1, **dadurch gekennzeichnet, dass** das nicht protische Lösungsmittel wenigstens ein Lösungsmittel aus der Gruppe bestehend aus gesättigten Kohlenwasserstoffen, aromatischen Kohlenwasserstoffen, halogenierten gesättigten Kohlenwasserstoffen, halogenierten aromatischen Kohlenwasserstoffen, Estern organischer Säuren, Ketonen, Nitrilen, Dialkylamiden, Kohlensäureestern, Sulfoxiden, Sulfonen, N-Alkylpyrrolidonen, cyclischen Ethern und nicht cyclischen Ethern umfasst oder ist.

3. Verfahren nach Anspruch 1 oder 2, **dadurch gekennzeichnet, dass** das vom Produktgemisch A abgetrennte Poly-3-hydroxypropionat ein relatives gewichtsmittleres Molekulargewicht von 1000 bis 20000 aufweist.

4. Verfahren nach einem der Ansprüche 1 bis 3, **dadurch gekennzeichnet, dass** das Katalysatorsystem die wenigstens eine Kobaltquelle in einer solchen Menge umfasst, dass diese, bezogen auf die molare Menge an carbonylierend umzusetzendem Ethylenoxid, 0,005 bis 20 mol-% Co enthält.

5. Verfahren nach einem der Ansprüche 1 bis 4, **dadurch gekennzeichnet, dass** wenigstens eine Kobaltquelle Dicobaltoctacarbonyl ist.

6. Verfahren nach einem der Ansprüche 1 bis 5, **dadurch gekennzeichnet, dass** das Katalysatorsystem wenigstens eine Verbindung als wenigstens einen coKatalysator C umfasst, die sowohl wenigstens eine nucleophile brönstedbasische Funktionalität, als auch wenigstens eine im Sinne von Brönsted saure Funktionalität wie ein aufweist.

7. Verfahren nach Anspruch 6, **dadurch gekennzeichnet, dass** der wenigstens eine co-Katalysator C wenigstens eine Verbindung aus der Gruppe bestehend aus 2-Hydroxpyridin, 3-Hydroxypyridin, 4-Hydroxypyridin, 3,4-Dihydroxypyridin, 3-Hydroxychinolin, 4-Hydroxy-2-Methylpyridin, 3-Hydroxy-4-Methylpyridin, 2,6-Dihydroxypyridin, 2-Hydroxychinolin, 1-Hydroxyisochinolin, 3-Hydroxychinolin, 2,3-Dihydroxychinoxalin, 8-Hydroxychinolin, 2-Pyridylmethanol, 3-Pyridylmethanol, 2-(2-Pyridyl)ethanol und Nicotinsäure ist.

8. Verfahren nach Anspruch 6 oder 7, **dadurch gekennzeichnet, dass** das nicht protische Lösungsmittel Diglyme umfasst, die wenigstens eine Kobaltquelle Dicobaltoctacarbonyl umfasst, und das Katalysatorsystem zusätzlich 3-Hydroxypyridin als wenigstens einen co-Katalysator C umfasst.

9. Verfahren nach einem der Ansprüche 1 bis 8, **dadurch gekennzeichnet, dass** der pH-Wert der wässrigen Fällflüssigkeit, bezogen auf eine Temperatur von 25°C und einen Druck von 1,0133·10⁵ Pa, ≤ 7,5 beträgt.

10. Verfahren nach einem der Ansprüche 1 bis 9, **dadurch gekennzeichnet, dass** die wässrige Fällflüssigkeit die wässrige Lösung einer anorganischen Säure und/oder einer organischen Säure ist.

11. Verfahren nach einem der Ansprüche 1 bis 10, **dadurch gekennzeichnet, dass** der Zusatz der wässrigen Fällflüssigkeit im Beisein wenigstens eines Oxidationsmittels für Co in Oxidationsstufen < +2 vorgenommen wird.

12. Verfahren nach Anspruch 11, **dadurch gekennzeichnet, dass** der Zusatz der wässrigen Fällflüssigkeit in Gegenwart von Luft oder in Gegenwart eines von Luft verschiedenen molekularen Sauerstoff enthaltenden Gases vorgenommen wird.

13. Verfahren nach einem der Ansprüche 1 bis 12, **dadurch gekennzeichnet, dass** der pH-Wert der wässrigen Waschflüssigkeit, bezogen auf eine Temperatur von 25 °C und einen Druck von 1,0133·10⁵ Pa, ≤ 7,5 beträgt.

14. Verfahren nach einem der Ansprüche 1 bis 13, **dadurch kennzeichnet, dass** dem in einer Trennzone A abgetrennten Poly-3-hydroxypropionat wenigstens ein die Thermolyse katalysierender Spaltkatalysator zugesetzt wird.

15. Verfahren nach Anspruch 14, **dadurch gekennzeichnet, dass** der wenigstens eine Spaltkatalysator eine molekulare organische Wirkverbindung ist, die neben Kohlenstoff und Wasserstoff als von diesen verschiedene Heteroatome wenigstens ein Stickstoffatom sowie wahlweise wenigstens ein oder mehr als ein Sauerstoffatom mit der Maßgabe kovalent gebunden aufweisen, dass
- die organische Wirkverbindung kein über Stickstoff und Sauerstoff hinausgehendes, von Kohlenstoff und Wasserstoff verschiedenes, Heteroatom aufweist,
und
- wenigstens ein Stickstoffatom ein tertiäres Stickstoffatom ist.

16. Verfahren nach einem der Ansprüche 1 bis 15, **dadurch gekennzeichnet, dass** sich an das Verfahren zur Herstellung von Acrylsäure ein Verfahren der radikalischen Polymerisation anschließt, in welchem die hergestellte Acrylsäure als solche und/oder in Form ihrer konjugierten Brönsted-Base sowie wahlweise im Gemisch mit anderen einfach und/oder mehrfach ungesättigten Verbindungen radikalisch initiiert in Polymerisat einpolymerisiert wird.

## Claims

1. A process for preparing acrylic acid from ethylene oxide and carbon monoxide, the process comprising at least the following process steps:
- converting by carbonylating ethylene oxide dissolved in an aprotic solvent with carbon monoxide at elevated pressure and elevated temperature in the presence of a catalyst system comprising at least one cobalt source in a reaction zone A to obtain a product mixture A comprising poly-3-hydroxypropionate,
- removing poly-3-hydroxypropionate from the product mixture A in a separation zone A, and
- thermolyzing poly-3-hydroxypropionate removed in separation zone A in a thermolysis zone A to form acrylic acid,
wherein the removing of poly-3-hydroxypropionate from the product mixture A in the separation zone A comprises at least one of the following process measures:
- adding water and/or an aqueous solution as an aqueous precipitation liquid to one or more portion(s) of product mixture A and/or to the total amount of product mixture A in order to precipitate poly-3-hydroxypropionate present in dissolved form in a portion of product mixture A or in the total amount of product mixture A;
- washing poly-3-hydroxypropionate removed from product mixture A in separation zone A with water and/or with an aqueous solution as an aqueous wash liquid.

2. The process according to claim 1, wherein the aprotic solvent comprises or is at least one solvent selected from the group consisting of saturated hydrocarbons, aromatic hydrocarbons, halogenated saturated hydrocarbons, halogenated aromatic hydrocarbons, esters of organic acids, ketones, nitriles, dialkylamides, carbonic esters, sulfoxides, sulfones, N-alkylpyrrolidones, cyclic ethers and acyclic ethers.

3. The process according to claim 1 or 2, wherein the poly-3-hydroxypropionate removed from product mixture A has a relative weight-average molecular weight of from 1000 to 20,000.

4. The process according to any of claims 1 to 3, wherein the catalyst system comprises the at least one cobalt source in such an amount that it comprises, based on the molar amount of ethylene oxide for carbonylating conversion, from 0.005 to 20 mol % of Co.

5. The process according to one of claims 1 to 4, wherein the at least one cobalt source is dicobalt octacarbonyl.

6. The process according to any of claims 1 to 5, wherein the catalyst system comprises at least one compound as at least one cocatalyst C which has both at least one nucleophilic Brønsted-basic functionality and at least one Brønsted-acidic functionality like a.

7. The process according to claim 6, wherein the at least one cocatalyst C is at least one compound selected from the group consisting of 2-hydroxypyridine, 3-hydroxypyridine, 4-hydroxypyridine, 3,4-dihydroxypyridine, 3-hydroxyquinoline, 4-hydroxy-2-methylpyridine, 3-hydroxy-4-methylpyridine, 2,6-dihydroxypyridine, 2-hydroxyquinoline, 1-hydroxyisoquinoline, 3-hydroxyquinoline, 2,3-dihydroxyquinoxaline, 8-hydroxyquinoline, 2-pyridylmethanol, 3-pyridylmethanol, 2-(2-pyridyl)ethanol and nicotinic acid.

8. The process according to claim 6 or 7, wherein the aprotic solvent comprises diglyme, the at least one cobalt source comprises dicobalt octacarbonyl, and the catalyst system additionally comprises 3-hydroxypyridine as at least one cocatalyst C.

9. The process according to any of claims 1 to 8, wherein the pH of the aqueous precipitation liquid, based on a temperature of 25° C and a pressure of 1.0133·10⁵ Pa, is ≤7.5.

10. The process according to any of claims 1 to 9, wherein the aqueous precipitation liquid is the aqueous solution of an inorganic acid and/or of an organic acid.

11. The process according to any of claims 1 to 10, wherein the adding of the aqueous precipitation liquid is undertaken in the presence of at least one oxidizing agent for Co in oxidation states <+2.

12. The process according to claim 11, wherein the adding of the aqueous precipitation liquid is undertaken in the presence of air or in the presence of a molecular oxygen-comprising gas other than air.

13. The process according to any of claims 1 to 12, wherein the pH of the aqueous wash liquid, based on a temperature of 25° C and a pressure of 1.0133·10⁵ Pa, is ≤7.5.

14. The process according to any of claims 1 to 13, wherein at least one splitting catalyst which catalyzes the thermolyzing is added to the poly-3-hydroxypropionate removed in a separation zone A.

15. The process according to claim 14, wherein the at least one splitting catalyst is a molecular organic active compound which, as well as carbon and hydrogen, as heteroatoms other than these, has at least one nitrogen atom and optionally at least one or more than one oxygen atom in covalently bonded form, with the proviso that
- the organic active compound does not have any heteroatom other than carbon and hydrogen over and above nitrogen and oxygen,
and
- at least one nitrogen atom is a tertiary nitrogen atom.

16. The process according to any of claims 1 to 15, wherein the process for preparing acrylic acid is followed by a process comprising free-radical polymerizing, in which the acrylic acid prepared is polymerized to a polymer with free-radical initiation as such and/or, in the form of the conjugate Brønsted base thereof and optionally in a mixture with other monounsaturated and/or polyunsaturated compounds.

## Revendications

1. Procédé de fabrication d'acide acrylique à partir d'oxyde d'éthylène et de monoxyde de carbone, qui comprend au moins les étapes de procédé suivantes :
- une réaction de carbonylation d'oxyde d'éthylène dissous dans un solvant non protique avec du monoxyde de carbone à pression élevée et température élevée en présence d'un système catalytique qui comprend au moins une source de cobalt, dans une zone de réaction A, pour obtenir un mélange de produits A contenant du poly-3-hydroxypropionate,
- une séparation du poly-3-hydroxypropionate du mélange de produits A dans une zone de séparation A, et
- une thermolyse du poly-3-hydroxypropionate séparé dans la zone de séparation A dans une zone de thermolyse A avec formation d'acide acrylique,
**caractérisé en ce que** la séparation du poly-3-hydroxypropionate du mélange de produits A dans la zone de séparation A comprend au moins une des mesures de procédé suivantes :
- un ajout d'eau et/ou d'une solution aqueuse en tant que liquide de précipitation aqueux à une ou plusieurs parties du mélange de produits A et/ou à la totalité du mélange de produits A, afin de précipiter le poly-3-hydroxypropionate contenu dissous dans une partie du mélange de produits A ou dans la totalité du mélange de produits A ;
- un lavage du poly-3-hydroxypropionate séparé du mélange de produits A dans la zone de séparation A avec de l'eau et/ou avec une solution aqueuse en tant que liquide de lavage aqueux.

2. Procédé selon la revendication 1, **caractérisé en ce que** le solvant non protique comprend ou est au moins un solvant du groupe constitué par les hydrocarbures saturés, les hydrocarbures aromatiques, les hydrocarbures saturés halogénés, les hydrocarbures aromatiques halogénés, les esters d'acides organiques, les cétones, les nitriles, les dialkylamides, les esters d'acide carbonique, les sulfoxydes, les sulfones, les N-alkylpyrrolidones, les éthers cycliques et les éthers non cycliques.

3. Procédé selon la revendication 1 ou 2, **caractérisé en ce que** le poly-3-hydroxypropionate séparé du mélange de produits A présente un poids moléculaire moyen en poids relatif de 1 000 à 20 000.

4. Procédé selon l'une quelconque des revendications 1 à 3, **caractérisé en ce que** le système catalytique comprend ladite au moins une source de cobalt en une quantité telle que celui-ci contienne, par rapport à la quantité molaire d'oxyde d'éthylène mis en réaction par carbonylation, de 0,005 à 20 % en moles de Co.

5. Procédé selon l'une quelconque des revendications 1 à 4, **caractérisé en ce qu'**au moins une source de cobalt est le dicobaltoctacarbonyle.

6. Procédé selon l'une quelconque des revendications 1 à 5, **caractérisé en ce que** le système catalytique comprend au moins un composé en tant qu'au moins un co-catalyseur C, qui présente aussi bien au moins une fonctionnalité nucléophile de base de Bronsted qu'au moins une fonctionnalité acide au sens de Bronsted comme un.

7. Procédé selon la revendication 6, **caractérisé en ce que** ledit au moins un co-catalyseur C est au moins un composé du groupe constitué par la 2-hydroxypyridine, la 3-hydroxypyridine, la 4-hydroxypyridine, la 3,4-dihydroxypyridine, la 3-hydroxyquinoline, la 4-hydroxy-2-méthylpyridine, la 3-hydroxy-4-méthylpyridine, la 2,6-dihydroxypyridine, la 2-hydroxyquinoline, la 1-hydroxyisoquinoline, la 3-hydroxyquinoline, la 2,3-dihydroxyquinoxaline, la 8-hydroxyquinoline, le 2-pyridylméthanol, le 3-pyridylméthanol, le 2-(2-pyridyl)éthanol et l'acide nicotinique.

8. Procédé selon la revendication 6 ou 7, **caractérisé en ce que** le solvant non protique comprend du diglyme, ladite au moins une source de cobalt comprend du dicobaltoctacarbonyle et le système catalytique comprend en outre de la 3-hydroxypyridine en tant qu'au moins un co-catalyseur C.

9. Procédé selon l'une quelconque des revendications 1 à 8, **caractérisé en ce que** le pH du liquide de précipitation aqueux, par rapport à une température de 25 °C et une pression de 1,0133·10⁵ Pa, est ≤ 7,5.

10. Procédé selon l'une quelconque des revendications 1 à 9, **caractérisé en ce que** le liquide de précipitation aqueux est la solution aqueuse d'un acide inorganique et/ou d'un acide organique.

11. Procédé selon l'une quelconque des revendications 1 à 10, **caractérisé en ce que** l'ajout du liquide de précipitation aqueux est réalisé en présence d'au moins un oxydant pour Co à des niveaux d'oxydation < +2.

12. Procédé selon la revendication 11, **caractérisé en ce que** l'ajout du liquide de précipitation aqueux est réalisé en présence d'air ou en présence d'un gaz contenant de l'oxygène moléculaire différent de l'air.

13. Procédé selon l'une quelconque des revendications 1 à 12, **caractérisé en ce que** le pH du liquide de lavage aqueux, par rapport à une température de 25 °C et une pression de 1,0133·10⁵ Pa, est ≤ 7,5.

14. Procédé selon l'une quelconque des revendications 1 à 13, **caractérisé en ce qu'**au moins un catalyseur de clivage catalysant la thermolyse est ajouté au poly-3-hydroxypropionate séparé dans une zone de séparation A.

15. Procédé selon la revendication 14, **caractérisé en ce que** ledit au moins un catalyseur de clivage est un composé actif organique moléculaire, qui comprend en plus du carbone et de l'hydrogène, en tant qu'hétéroatomes différents de ceux-ci, au moins un atome d'azote, ainsi qu'éventuellement au moins un ou plus d'un atome d'oxygène relié par une liaison covalente, à condition que
- le composé actif organique ne comprenne pas d'hétéroatomes différents du carbone et de l'hydrogène outre l'azote et l'oxygène,
et
- au moins un atome d'azote soit un atome d'azote tertiaire.

16. Procédé selon l'une quelconque des revendications 1 à 15, **caractérisé en ce que** le procédé de fabrication d'acide acrylique est suivi par un procédé de polymérisation radicalaire lors duquel l'acide acrylique fabriqué est polymérisé par voie radicalaire en un polymère en tant que tel et/ou sous la forme de sa base de Bronsted conjuguée, ainsi qu'éventuellement en mélange avec d'autres composés mono- et/ou polyinsaturés.
